# EUROPEAN PATENT APPLICATION

(11) **EP 2 224 244 A1**
(43) Date of publication of application: **01.09.2010**
(21) Application number: 09153774.6
(22) Date of filing: 26.02.2009
(51) Int. Cl.: G01N 33/68

(54) **A diagnostic in-vitro method or assay for diagnosing chronic obstructive pulmonary disease (COPD)**

(71) Applicant: Universität des Saarlandes, 66123 Saarbrücken (DE)
(72) Inventor: Meese, Eckart, 66882, Hütschenhausen (DE); Lenhof, Hans-Peter, 66822, Lebach (DE)
(74) Representative: Polypatent

(57) **Abstract**

The present invention refers to an in-vitro diagnostic assay or method for diagnosing chronic obstructive pulmonary disease (COPD), comprising screening for the presence of antibodies in a blood or serum sample, wherein the antibodies are directed to MCM3 (SEQ ID NO: 1).

SEQUENCE LISTING

<110>  Universität des Saarlandes

<120>  A diagnostic in-vitro method or assay for diagnosing
chronic
       obstructive pulmonary disease (COPD)

<130>  P22220EP

<160>  58

<170>  PatentIn version 3.3

<210>  1
<211>  808
<212>  PRT
<213>  Homo sapiens

<400>  1

Met Ala Gly Thr Val Val Leu Asp Asp Val Glu Leu Arg Glu Ala Gln
1               5                   10                  15

Arg Asp Tyr Leu Asp Phe Leu Asp Asp Glu Glu Asp Gln Gly Ile Tyr
            20                  25                  30

Gln Ser Lys Val Arg Glu Leu Ile Ser Asp Asn Gln Tyr Arg Leu Ile
        35                  40                  45

Val Asn Val Asn Asp Leu Arg Arg Lys Asn Glu Lys Arg Ala Asn Arg
    50                  55                  60

Leu Leu Asn Asn Ala Phe Glu Glu Leu Val Ala Phe Gln Arg Ala Leu
65                  70                  75                  80

Lys Asp Phe Val Ala Ser Ile Asp Ala Thr Tyr Ala Lys Gln Tyr Glu
                85                  90                  95

Glu Phe Tyr Val Gly Leu Glu Gly Ser Phe Gly Ser Lys His Val Ser
            100                 105                 110

Pro Arg Thr Leu Thr Ser Cys Phe Leu Ser Cys Val Val Cys Val Glu
        115                 120                 125

Gly Ile Val Thr Lys Cys Ser Leu Val Arg Pro Lys Val Val Arg Ser
    130                 135                 140

Val His Tyr Cys Pro Ala Thr Lys Lys Thr Ile Glu Arg Arg Tyr Ser
145                 150                 155                 160

Asp Leu Thr Thr Leu Val Ala Phe Pro Ser Ser Ser Val Tyr Pro Thr
                165                 170                 175

Lys Asp Glu Glu Asn Asn Pro Leu Glu Thr Glu Tyr Gly Leu Ser Val
            180                 185                 190

Tyr Lys Asp His Gln Thr Ile Thr Ile Gln Glu Met Pro Glu Lys Ala
        195                 200                 205

Pro Ala Gly Gln Leu Pro Arg Ser Val Asp Val Ile Leu Asp Asp Asp
    210                 215                 220

Leu Val Asp Lys Ala Lys Pro Gly Asp Arg Val Gln Val Val Gly Thr
225                 230                 235                 240

Tyr Arg Cys Leu Pro Gly Lys Lys Gly Gly Tyr Thr Ser Gly Thr Phe
                245                 250                 255

Arg Thr Val Leu Ile Ala Cys Asn Val Lys Gln Met Ser Lys Asp Ala
            260                 265                 270

Gln Pro Ser Phe Ser Ala Glu Asp Ile Ala Lys Ile Lys Lys Phe Ser
        275                 280                 285

Lys Thr Arg Ser Lys Asp Ile Phe Asp Gln Leu Ala Lys Ser Leu Ala
    290                 295                 300

Pro Ser Ile His Gly His Asp Tyr Val Lys Lys Ala Ile Leu Cys Leu
305                 310                 315                 320

Leu Leu Gly Gly Val Glu Arg Asp Leu Glu Asn Gly Ser His Ile Arg
                325                 330                 335

Gly Asp Ile Asn Ile Leu Leu Ile Gly Asp Pro Ser Val Ala Lys Ser

340                 345                 350

Gln Leu Leu Arg Tyr Val Leu Cys Thr Ala Pro Arg Ala Ile Pro Thr
        355                 360                 365

Thr Gly Arg Gly Ser Ser Gly Val Gly Leu Thr Ala Ala Val Thr Thr
    370                 375                 380

Asp Gln Glu Thr Gly Glu Arg Arg Leu Glu Ala Gly Ala Met Val Leu
385                 390                 395                 400

Ala Asp Arg Gly Val Val Cys Ile Asp Glu Phe Asp Lys Met Ser Asp
                405                 410                 415

Met Asp Arg Thr Ala Ile His Glu Val Met Glu Gln Gly Arg Val Thr
            420                 425                 430

Ile Ala Lys Ala Gly Ile His Ala Arg Leu Asn Ala Arg Cys Ser Val
        435                 440                 445

Leu Ala Ala Ala Asn Pro Val Tyr Gly Arg Tyr Asp Gln Tyr Lys Thr
    450                 455                 460

Pro Met Glu Asn Ile Gly Leu Gln Asp Ser Leu Leu Ser Arg Phe Asp
465                 470                 475                 480

Leu Leu Phe Ile Met Leu Asp Gln Met Asp Pro Glu Gln Asp Arg Glu
                485                 490                 495

Ile Ser Asp His Val Leu Arg Met His Arg Tyr Arg Ala Pro Gly Glu
            500                 505                 510

Gln Asp Gly Asp Ala Met Pro Leu Gly Ser Ala Val Asp Ile Leu Ala
        515                 520                 525

Thr Asp Asp Pro Asn Phe Ser Gln Glu Asp Gln Gln Asp Thr Gln Ile
    530                 535                 540

Tyr Glu Lys His Asp Asn Leu Leu His Gly Thr Lys Lys Lys Lys Glu
545                 550                 555                 560

Lys Met Val Ser Ala Ala Phe Met Lys Lys Tyr Ile His Val Ala Lys
                565                 570                 575

Ile Ile Lys Pro Val Leu Thr Gln Glu Ser Ala Thr Tyr Ile Ala Glu
            580                 585                 590

Glu Tyr Ser Arg Leu Arg Ser Gln Asp Ser Met Ser Ser Asp Thr Ala
        595                 600                 605

Arg Thr Ser Pro Val Thr Ala Arg Thr Leu Glu Thr Leu Ile Arg Leu
    610                 615                 620

Ala Thr Ala His Ala Lys Ala Arg Met Ser Lys Thr Val Asp Leu Gln
625                 630                 635                 640

Asp Ala Glu Glu Ala Val Glu Leu Val Gln Tyr Ala Tyr Phe Lys Lys
                645                 650                 655

Val Leu Glu Lys Glu Lys Lys Arg Lys Lys Arg Ser Glu Asp Glu Ser
            660                 665                 670

Glu Thr Glu Asp Glu Glu Glu Lys Ser Gln Glu Asp Gln Glu Gln Lys
        675                 680                 685

Arg Lys Arg Arg Lys Thr Arg Gln Pro Asp Ala Lys Asp Gly Asp Ser
    690                 695                 700

Tyr Asp Pro Tyr Asp Phe Ser Asp Thr Glu Glu Glu Met Pro Gln Val
705                 710                 715                 720

His Thr Pro Lys Thr Ala Asp Ser Gln Glu Thr Lys Glu Ser Gln Lys
                725                 730                 735

Val Glu Leu Ser Glu Ser Arg Leu Lys Ala Phe Lys Val Ala Leu Leu
            740                 745                 750

Asp Val Phe Arg Glu Ala His Ala Gln Ser Ile Gly Met Asn Arg Leu
        755                 760                 765

Thr Glu Ser Ile Asn Arg Asp Ser Glu Glu Pro Phe Ser Ser Val Glu
    770                 775                 780

Ile Gln Ala Ala Leu Ser Lys Met Gln Asp Asp Asn Gln Val Met Val
785                 790                 795                 800

Ser Glu Gly Ile Ile Phe Leu Ile
                805

<210>  2
<211>  1047
<212>  PRT
<213>  Homo sapiens

<400>  2
   Met Thr Asp Asn Ile Pro Leu Gln Pro Val Arg Gln Lys Lys Arg Met
   1               5                   10                  15
   Asp Ser Arg Pro Arg Ala Gly Cys Cys Glu Trp Leu Arg Cys Cys Gly
               20                  25                  30
   Gly Gly Glu Ala Arg Pro Arg Thr Val Trp Leu Gly His Pro Glu Lys
           35                  40                  45
   Arg Asp Gln Arg Tyr Pro Arg Asn Val Ile Asn Asn Gln Lys Tyr Asn
       50                  55                  60
   Phe Phe Thr Phe Leu Pro Gly Val Leu Phe Asn Gln Phe Lys Tyr Phe
   65                  70                  75                  80
   Phe Asn Leu Tyr Phe Leu Leu Leu Ala Cys Ser Gln Phe Val Pro Glu
                   85                  90                  95
   Met Arg Leu Gly Ala Leu Tyr Thr Tyr Trp Val Pro Leu Gly Phe Val
               100                 105                 110
   Leu Ala Val Thr Val Ile Arg Glu Ala Val Glu Glu Ile Arg Cys Tyr
           115                 120                 125
   Val Arg Asp Lys Glu Val Asn Ser Gln Val Tyr Ser Arg Leu Thr Ala
   130                 135                 140
   Arg Gly Thr Val Lys Val Lys Ser Ser Asn Ile Gln Val Gly Asp Leu
   145                 150                 155                 160
   Ile Ile Val Glu Lys Asn Gln Arg Val Pro Ala Asp Met Ile Phe Leu
                   165                 170                 175
   Arg Thr Ser Glu Lys Asn Gly Ser Cys Phe Leu Arg Thr Asp Gln Leu
               180                 185                 190
   Asp Gly Glu Thr Asp Trp Lys Leu Arg Leu Pro Val Ala Cys Thr Gln
           195                 200                 205
   Arg Leu Pro Thr Ala Ala Asp Leu Leu Gln Ile Arg Ser Tyr Val Tyr
       210                 215                 220
   Ala Glu Glu Pro Asn Ile Asp Ile His Asn Phe Val Gly Thr Phe Thr
   225                 230                 235                 240
   Arg Glu Asp Ser Asp Pro Pro Ile Ser Glu Ser Leu Ser Ile Glu Asn
                   245                 250                 255
   Thr Leu Trp Ala Gly Thr Val Val Ala Ser Gly Thr Val Val Gly Val
               260                 265                 270
   Val Leu Tyr Thr Gly Arg Glu Leu Arg Ser Val Met Asn Thr Ser Asn
           275                 280                 285
   Pro Arg Ser Lys Ile Gly Leu Phe Asp Leu Glu Val Asn Cys Leu Thr
       290                 295                 300
   Lys Ile Leu Phe Gly Ala Leu Val Val Val Ser Leu Val Met Val Ala
   305                 310                 315                 320
   Leu Gln His Phe Ala Gly Arg Trp Tyr Leu Gln Ile Ile Arg Phe Leu
                   325                 330                 335
   Leu Leu Phe Ser Asn Ile Ile Pro Ile Ser Leu Arg Val Asn Leu Asp
               340                 345                 350
   Met Gly Lys Ile Val Tyr Ser Trp Val Ile Arg Arg Asp Ser Lys Ile
           355                 360                 365
   Pro Gly Thr Val Val Arg Ser Ser Thr Ile Pro Glu Gln Leu Gly Arg
       370                 375                 380
   Ile Ser Tyr Leu Leu Thr Asp Lys Thr Gly Thr Leu Thr Gln Asn Glu
   385                 390                 395                 400
   Met Ile Phe Lys Arg Leu His Leu Gly Thr Val Ala Tyr Gly Leu Asp
                   405                 410                 415
   Ser Met Asp Glu Val Gln Ser His Ile Phe Ser Ile Tyr Thr Gln Gln
               420                 425                 430
   Ser Gln Asp Pro Pro Ala Gln Lys Gly Pro Thr Leu Thr Thr Lys Val
           435                 440                 445
   Arg Arg Thr Met Ser Ser Arg Val His Glu Ala Val Lys Ala Ile Ala
       450                 455                 460
   Leu Cys His Asn Val Thr Pro Val Tyr Glu Ser Asn Gly Val Thr Asp
   465                 470                 475                 480
   Gln Ala Glu Ala Glu Lys Gln Tyr Glu Asp Ser Cys Arg Val Tyr Gln
                   485                 490                 495
   Ala Ser Ser Pro Asp Glu Val Ala Leu Val Gln Trp Thr Glu Ser Val
               500                 505                 510
   Gly Leu Thr Leu Val Gly Arg Asp Gln Ser Ser Met Gln Leu Arg Thr
           515                 520                 525
   Pro Gly Asp Gln Ile Leu Asn Phe Thr Ile Leu Gln Ile Phe Pro Phe
       530                 535                 540
   Thr Tyr Glu Ser Lys Arg Met Gly Ile Ile Val Arg Asp Glu Ser Thr
   545                 550                 555                 560
   Gly Glu Ile Thr Phe Tyr Met Lys Gly Ala Asp Val Val Met Ala Gly
                   565                 570                 575
   Ile Val Gln Tyr Asn Asp Trp Leu Glu Glu Glu Cys Gly Asn Met Ala
               580                 585                 590
   Arg Glu Gly Leu Arg Val Leu Val Val Ala Lys Lys Ser Leu Ala Glu
           595                 600                 605
   Glu Gln Tyr Gln Asp Phe Glu Ala Arg Tyr Val Gln Ala Lys Leu Ser
       610                 615                 620
   Val His Asp Arg Ser Leu Lys Val Ala Thr Val Ile Glu Ser Leu Glu
   625                 630                 635                 640
   Met Glu Met Glu Leu Leu Cys Leu Thr Gly Val Glu Asp Gln Leu Gln
                   645                 650                 655
   Ala Asp Val Arg Pro Thr Leu Glu Thr Leu Arg Asn Ala Gly Ile Lys
               660                 665                 670
   Val Trp Met Leu Thr Gly Asp Lys Leu Glu Thr Ala Thr Cys Thr Ala
           675                 680                 685
   Lys Asn Ala His Leu Val Thr Arg Asn Gln Asp Ile His Val Phe Arg
       690                 695                 700
   Leu Val Thr Asn Arg Gly Glu Ala His Leu Glu Leu Asn Ala Phe Arg
   705                 710                 715                 720
   Arg Lys His Asp Cys Ala Leu Val Ile Ser Gly Asp Ser Leu Glu Val
                   725                 730                 735
   Cys Leu Lys Tyr Tyr Glu Tyr Glu Phe Met Glu Leu Ala Cys Gln Cys
               740                 745                 750
   Pro Ala Val Val Cys Cys Arg Cys Ala Pro Thr Gln Lys Ala Gln Ile
           755                 760                 765
   Val Arg Leu Leu Gln Glu Arg Thr Gly Lys Leu Thr Cys Ala Val Gly
       770                 775                 780
   Asp Gly Gly Asn Asp Val Ser Met Ile Gln Glu Ser Asp Cys Gly Val
   785                 790                 795                 800
   Gly Val Glu Gly Lys Glu Gly Lys Gln Ala Ser Leu Ala Ala Asp Phe
                   805                 810                 815
   Ser Ile Thr Gln Phe Lys His Leu Gly Arg Leu Leu Met Val His Gly
               820                 825                 830
   Arg Asn Ser Tyr Lys Arg Ser Ala Ala Leu Ser Gln Phe Val Ile His
           835                 840                 845
   Arg Ser Leu Cys Ile Ser Thr Met Gln Ala Val Phe Ser Ser Val Phe
       850                 855                 860
   Tyr Phe Ala Ser Val Pro Leu Tyr Gln Gly Phe Leu Ile Ile Gly Tyr
   865                 870                 875                 880
   Ser Thr Ile Tyr Thr Met Phe Pro Val Phe Ser Leu Val Leu Asp Lys
                   885                 890                 895
   Asp Val Lys Ser Glu Val Ala Met Leu Tyr Pro Glu Leu Tyr Lys Asp
               900                 905                 910
   Leu Leu Lys Gly Arg Pro Leu Ser Tyr Lys Thr Phe Leu Ile Trp Val
           915                 920                 925
   Leu Ile Ser Ile Tyr Gln Gly Ser Thr Ile Met Tyr Gly Ala Leu Leu
       930                 935                 940
   Leu Phe Glu Ser Glu Phe Val His Ile Val Ala Ile Ser Phe Thr Ser
   945                 950                 955                 960
   Leu Ile Leu Thr Glu Leu Leu Met Val Ala Leu Thr Ile Gln Thr Trp
                   965                 970                 975
   His Trp Leu Met Thr Val Ala Glu Leu Leu Ser Leu Ala Cys Tyr Ile
   980 985 990
   Ala Ser Leu Val Phe Leu His Glu Phe Ile Asp Val Tyr Phe Ile Ala
   995 1000 1005
   Thr Leu Ser Phe Leu Trp Lys Val Ser Val Ile Thr Leu Val Ser
   1010 1015 1020
   Cys Leu Pro Leu Tyr Val Leu Lys Tyr Leu Arg Arg Arg Phe Ser
   1025 1030 1035
   Pro Pro Ser Tyr Ser Lys Leu Thr Ser
   1040 1045

<210>  3
<211>  156
<212>  PRT
<213>  Homo sapiens

<400>  3

Met Gln Ile Phe Val Lys Thr Leu Met Gly Lys Thr Ile Thr Leu Glu
1               5                   10                  15

Val Glu Leu Ser Asp Thr Ile Asp Asn Val Lys Ala Lys Ile Gln Asp
            20                  25                  30

Lys Glu Gly Ile Pro Pro Asp Gln Gln Arg Leu Ile Phe Ala Gly Lys
        35                  40                  45

Gln Leu Glu Asp Gly Arg Thr Leu Ser Asp Tyr Asn Ile Gln Lys Glu
    50                  55                  60

Ser Thr Leu His Leu Val Leu Arg Leu Arg Gly Gly Ala Lys Lys Arg
65                  70                  75                  80

Lys Lys Lys Ser Tyr Thr Thr Pro Arg Lys Asn Lys His Lys Arg Lys
                85                  90                  95

Lys Val Lys Leu Ala Leu Leu Lys Tyr Tyr Lys Val Asp Glu Asn Gly
            100                 105                 110

Lys Ile Ser Cys Leu His Arg Glu Cys Pro Ser Asp Glu Cys Gly Ala
        115                 120                 125

Gly Val Phe Met Ala Ser His Phe Asp Arg His Tyr Cys Gly Lys Cys
    130                 135                 140

Cys Leu Thr Tyr Cys Phe Asn Lys Pro Glu Asp Lys
145                 150                 155

<210>  4
<211>  1711
<212>  PRT
<213>  Homo sapiens

<400>  4

<210> 5
<211> 126
<212> PRT
<213> Homo sapiens

<400> 5

Met Ala Ala Gly Pro Glu Pro Pro Glu Trp Glu Pro Arg Trp Arg Lys
1               5                   10                  15

Ala Leu Arg Gly Lys Glu Asn Lys Gly Ser Val Glu Ile Met Arg Lys
            20                  25                  30

Asp Leu Asn Asp Ala Arg Asp Leu His Gly Gln Ala Glu Ser Ala Ala
        35                  40                  45

Ala Val Trp Lys Gly His Val Met Asp Arg Arg Lys Lys Ala Leu Thr
    50                  55                  60

Asp Tyr Lys Lys Leu Arg Ala Phe Phe Val Glu Glu Glu Glu His Phe
65                  70                  75                  80

Leu Gln Glu Ala Glu Lys Glu Glu Gly Leu Pro Glu Asp Glu Leu Ala
                85                  90                  95

Asp Pro Thr Glu Arg Phe Arg Ser Leu Leu Gln Ala Val Ser Glu Leu
            100                 105                 110

Glu Lys Lys His Arg Asn Leu Gly Leu Ser Met Leu Leu Gln
        115                 120                 125

<210>  6
<211>  156
<212>  PRT
<213>  Homo sapiens

<400>  6

Met Gln Ile Phe Val Lys Thr Leu Thr Gly Lys Thr Ile Thr Leu Glu
1               5                   10                  15

Val Glu Pro Ser Asp Thr Ile Glu Asn Val Lys Ala Lys Ile Gln Asp
            20                  25                  30

Lys Glu Gly Ile Pro Pro Asp Gln Gln Arg Leu Ile Phe Ala Gly Lys
        35                  40                  45

Gln Leu Glu Asp Gly Arg Thr Leu Ser Asp Tyr Asn Ile Gln Lys Glu
    50                  55                  60

Ser Thr Leu His Leu Val Leu Arg Leu Arg Gly Gly Ala Lys Lys Arg
65                  70                  75                  80

Lys Lys Lys Ser Tyr Thr Thr Pro Lys Lys Asn Lys His Lys Arg Lys
                85                  90                  95

Lys Val Lys Leu Ala Val Leu Lys Tyr Tyr Lys Val Asp Glu Asn Gly
            100                 105                 110

Lys Ile Ser Arg Leu Arg Arg Glu Cys Pro Ser Asp Glu Cys Gly Ala
        115                 120                 125

Gly Val Phe Met Ala Ser His Phe Asp Arg His Tyr Cys Gly Lys Cys
    130                 135                 140

Cys Leu Thr Tyr Cys Phe Asn Lys Pro Glu Asp Lys
145                 150                 155

<210> 7
<211> 1100
<212> PRT
<213> Homo sapiens

<400> 7

Met Ser Tyr Tyr Gln Arg Pro Phe Ser Pro Ser Ala Tyr Ser Leu Pro
1               5                   10                  15

Ala Ser Leu Asn Ser Ser Ile Val Met Gln His Gly Thr Ser Leu Asp
            20                  25                  30

Ser Thr Asp Thr Tyr Pro Gln His Ala Gln Ser Leu Asp Gly Thr Thr
        35                  40                  45

Ser Ser Ser Ile Pro Leu Tyr Arg Ser Ser Glu Glu Glu Lys Arg Val
    50                  55                  60

Thr Val Ile Lys Ala Pro His Tyr Pro Gly Ile Gly Pro Val Asp Glu
65                  70                  75                  80

Ser Gly Ile Pro Thr Ala Ile Arg Thr Thr Val Asp Arg Pro Lys Asp
                85                  90                  95

Trp Tyr Lys Thr Met Phe Lys Gln Ile His Met Val His Lys Pro Asp
            100                 105                 110

Asp Asp Thr Asp Met Tyr Asn Thr Pro Tyr Thr Tyr Asn Ala Gly Leu
        115                 120                 125

Tyr Asn Pro Pro Tyr Ser Ala Gln Ser His Pro Ala Ala Lys Thr Gln
    130                 135                 140

Thr Tyr Arg Pro Leu Ser Lys Ser His Ser Asp Asn Ser Pro Asn Ala
145                 150                 155                 160

Phe Lys Asp Ala Ser Ser Pro Val Pro Pro Pro His Val Pro Pro Pro
                165                 170                 175

Val Pro Pro Leu Arg Pro Arg Asp Arg Ser Ser Thr Glu Lys His Asp
            180                 185                 190

Trp Asp Pro Pro Asp Arg Lys Val Asp Thr Arg Lys Phe Arg Ser Glu
        195                 200                 205

Pro Arg Ser Ile Phe Glu Tyr Glu Pro Gly Lys Ser Ser Ile Leu Gln
    210                 215                 220

His Glu Arg Pro Ala Ser Leu Tyr Gln Ser Ser Ile Asp Arg Ser Leu
225                 230                 235                 240

Glu Arg Pro Met Ser Ser Ala Ser Met Ala Ser Asp Phe Arg Lys Arg
                245                 250                 255

Arg Lys Ser Glu Pro Ala Val Gly Pro Pro Arg Gly Leu Gly Asp Gln
            260                 265                 270

Ser Ala Ser Arg Thr Ser Pro Gly Arg Val Asp Leu Pro Gly Ser Ser
        275                 280                 285

Thr Thr Leu Thr Lys Ser Phe Thr Ser Ser Ser Pro Ser Ser Pro Ser
    290                 295                 300

Arg Ala Lys Gly Gly Asp Asp Ser Lys Ile Cys Pro Ser Leu Cys Ser
305                 310                 315                 320

Tyr Ser Gly Leu Asn Gly Asn Pro Ser Ser Glu Leu Asp Tyr Cys Ser
                325                 330                 335

Thr Tyr Arg Gln His Leu Asp Val Pro Arg Asp Ser Pro Arg Ala Ile
            340                 345                 350

Ser Phe Lys Asn Gly Trp Gln Met Ala Arg Gln Asn Ala Glu Ile Trp
        355                 360                 365

Ser Ser Thr Glu Glu Thr Val Ser Pro Lys Ile Lys Ser Arg Ser Cys
    370                 375                 380

Asp Asp Leu Leu Asn Asp Asp Cys Asp Ser Phe Pro Asp Pro Lys Val
385                 390                 395                 400

Lys Ser Glu Ser Met Gly Ser Leu Leu Cys Glu Glu Asp Ser Lys Glu
                405                 410                 415

Ser Cys Pro Met Ala Trp Gly Ser Pro Tyr Val Pro Glu Val Arg Ser
            420                 425                 430

Asn Gly Arg Ser Arg Ile Arg His Arg Ser Ala Arg Asn Ala Pro Gly
        435                 440                 445

Phe Leu Lys Met Tyr Lys Lys Met His Arg Ile Asn Arg Lys Asp Leu
    450                 455                 460

Met Asn Ser Glu Val Ile Cys Ser Val Lys Ser Arg Ile Leu Gln Tyr
465                 470                 475                 480

Glu Ser Glu Gln Gln His Lys Asp Leu Leu Arg Ala Trp Ser Gln Cys
                485                 490                 495

Ser Thr Glu Glu Val Pro Arg Asp Met Val Pro Thr Arg Ile Ser Glu
            500                 505                 510

Phe Glu Lys Leu Ile Gln Lys Ser Lys Ser Met Pro Asn Leu Gly Asp
        515                 520                 525

Asp Met Leu Ser Pro Val Thr Leu Glu Pro Pro Gln Asn Gly Leu Cys
    530                 535                 540

Pro Lys Arg Arg Phe Ser Ile Glu Tyr Leu Leu Glu Glu Glu Asn Gln
545                 550                 555                 560

Ser Gly Pro Pro Ala Arg Gly Arg Arg Gly Cys Gln Ser Asn Ala Leu
                565                 570                 575

Val Pro Ile His Ile Glu Val Thr Ser Asp Glu Gln Pro Arg Ala His
            580                 585                 590

Val Glu Phe Ser Asp Ser Asp Gln Asp Gly Val Val Ser Asp His Ser
        595                 600                 605

Asp Tyr Ile His Leu Glu Gly Ser Ser Phe Cys Ser Glu Ser Asp Phe
    610                 615                 620

Asp His Phe Ser Phe Thr Ser Ser Glu Ser Phe Tyr Gly Ser Ser His
625                 630                 635                 640

His His His His His His His His His His Arg His Leu Ile Ser Ser
                645                 650                 655

Cys Lys Gly Arg Cys Pro Ala Ser Tyr Thr Arg Phe Thr Thr Met Leu
            660                 665                 670

Lys His Glu Arg Ala Arg His Glu Asn Thr Glu Glu Pro Arg Arg Gln
        675                 680                 685

Glu Met Asp Pro Gly Leu Ser Lys Leu Ala Phe Leu Val Ser Pro Val
    690                 695                 700

Pro Phe Arg Arg Lys Lys Asn Ser Ala Pro Lys Lys Gln Thr Glu Lys
705                 710                 715                 720

Ala Lys Cys Lys Ala Ser Val Phe Glu Ala Leu Asp Ser Ala Leu Lys
                725                 730                 735

Asp Ile Cys Asp Gln Ile Lys Ala Glu Lys Lys Arg Gly Ser Leu Pro
            740                 745                 750

Asp Asn Ser Ile Leu His Arg Leu Ile Ser Glu Leu Leu Pro Asp Val

755                 760                 765

Pro Glu Arg Asn Ser Ser Leu Arg Ala Leu Arg Arg Ser Pro Leu His
    770                 775                 780

Gln Pro Leu His Pro Leu Pro Pro Asp Gly Ala Ile His Cys Pro Pro
785                 790                 795                 800

Tyr Gln Asn Asp Cys Gly Arg Met Pro Arg Ser Ala Ser Phe Gln Asp
                805                 810                 815

Val Asp Thr Ala Asn Ser Ser Cys His His Gln Asp Arg Gly Gly Ala
            820                 825                 830

Leu Gln Asp Arg Glu Ser Pro Arg Ser Tyr Ser Ser Thr Leu Thr Asp
        835                 840                 845

Met Gly Arg Ser Ala Pro Arg Glu Arg Arg Gly Thr Pro Glu Lys Glu
    850                 855                 860

Lys Leu Pro Ala Lys Ala Val Tyr Asp Phe Lys Ala Gln Thr Ser Lys
865                 870                 875                 880

Glu Leu Ser Phe Lys Lys Gly Asp Thr Val Tyr Ile Leu Arg Lys Ile
                885                 890                 895

Asp Gln Asn Trp Tyr Glu Gly Glu His His Gly Arg Val Gly Ile Phe
            900                 905                 910

Pro Ile Ser Tyr Val Glu Lys Leu Thr Pro Pro Glu Lys Ala Gln Pro
        915                 920                 925

Ala Arg Pro Pro Pro Pro Ala Gln Pro Gly Glu Ile Gly Glu Ala Ile
    930                 935                 940

Ala Lys Tyr Asn Phe Asn Ala Asp Thr Asn Val Glu Leu Ser Leu Arg
945                 950                 955                 960

Lys Gly Asp Arg Val Ile Leu Leu Lys Arg Val Asp Gln Asn Trp Tyr
                965                 970                 975

Glu Gly Lys Ile Pro Gly Thr Asn Arg Gln Gly Ile Phe Pro Val Ser
            980                 985                 990

Tyr Val Glu Val Val Lys Lys Asn  Thr Lys Gly Ala Glu  Asp Tyr Pro
        995                 1000                 1005

Asp Pro  Pro Ile Pro His Ser  Tyr Ser Ser Asp Arg  Ile His Ser
    1010                 1015                 1020

Leu Ser  Ser Asn Lys Pro Gln  Arg Pro Val Phe Thr  His Glu Asn
    1025                 1030                 1035

Ile Gln  Gly Gly Gly Glu Pro  Phe Gln Ala Leu Tyr  Asn Tyr Thr
    1040                 1045                 1050

Pro Arg  Asn Glu Asp Glu Leu  Glu Leu Arg Glu Ser  Asp Val Ile
    1055                 1060                 1065

Asp Val  Met Glu Lys Cys Asp  Asp Gly Trp Phe Val  Gly Thr Ser
    1070                 1075                 1080

Arg Arg  Thr Lys Phe Phe Gly  Thr Phe Pro Gly Asn  Tyr Val Lys
    1085                 1090                 1095

Arg Leu
    1100

<210>  8
<211>  294
<212>  PRT
<213>  Homo sapiens

<400>  8

Met Glu Tyr Pro Ala Pro Ala Thr Val Gln Ala Ala Asp Gly Gly Ala
1               5                   10                  15

Ala Gly Pro Tyr Ser Ser Ser Glu Leu Leu Glu Gly Gln Glu Pro Asp
            20                  25                  30

Gly Val Arg Phe Asp Arg Glu Arg Ala Arg Arg Leu Trp Glu Ala Val
        35                  40                  45

Ser Gly Ala Gln Pro Val Gly Arg Glu Glu Val Glu His Met Ile Gln
    50                  55                  60

Lys Asn Gln Cys Leu Phe Thr Asn Thr Gln Cys Lys Val Cys Cys Ala
65                  70                  75                  80

Leu Leu Ile Ser Glu Ser Gln Lys Leu Ala His Tyr Gln Ser Lys Lys
                85                  90                  95

His Ala Asn Lys Val Lys Arg Tyr Leu Ala Ile His Gly Met Glu Thr
            100                 105                 110

Leu Lys Gly Glu Thr Lys Lys Leu Asp Ser Asp Gln Lys Ser Ser Arg
        115                 120                 125

Ser Lys Asp Lys Asn Gln Cys Cys Pro Ile Cys Asn Met Thr Phe Ser
    130                 135                 140

Ser Pro Val Val Ala Gln Ser His Tyr Leu Gly Lys Thr His Ala Lys
145                 150                 155                 160

Asn Leu Lys Leu Lys Gln Gln Ser Thr Lys Val Glu Ala Leu His Gln
                165                 170                 175

Asn Arg Glu Met Ile Asp Pro Asp Lys Phe Cys Ser Leu Cys His Ala
            180                 185                 190

Thr Phe Asn Asp Pro Val Met Ala Gln Gln His Tyr Val Gly Lys Lys
        195                 200                 205

His Arg Lys Gln Glu Thr Lys Leu Lys Leu Met Ala Arg Tyr Gly Arg
    210                 215                 220

Leu Ala Asp Pro Ala Val Thr Asp Phe Pro Ala Gly Lys Gly Tyr Pro
225                 230                 235                 240

Cys Lys Thr Cys Lys Ile Val Leu Asn Ser Ile Glu Gln Tyr Gln Ala

245                 250                 255

His Val Ser Gly Phe Lys His Lys Asn Gln Ser Pro Lys Thr Val Ala
            260                 265                 270

Ser Ser Leu Gly Gln Ile Pro Met Gln Arg Gln Pro Ile Gln Lys Asp
        275                 280                 285

Ser Thr Thr Leu Glu Asp
    290

<210>  9
<211>  310
<212>  PRT
<213>  Homo sapiens

<400>  9

Met Lys Gly Thr Asp Ser Gly Ser Cys Cys Arg Arg Arg Cys Asp Phe
1               5                   10                  15

Gly Cys Cys Cys Arg Ala Ser Arg Arg Ala His Tyr Thr Pro Tyr Arg
            20                  25                  30

Ser Gly Asp Ala Thr Arg Thr Pro Gln Ser Pro Arg Gln Thr Pro Ser
        35                  40                  45

Arg Glu Arg Arg Arg Pro Glu Pro Ala Gly Ser Trp Ala Ala Ala Ala
    50                  55                  60

Glu Glu Glu Glu Ala Ala Ala Ala Ala Thr Pro Trp Met Arg Asp Tyr
65                  70                  75                  80

Phe Ala Glu Asp Asp Gly Glu Met Val Pro Arg Thr Ser His Thr Ala
                85                  90                  95

Ala Phe Leu Ser Asp Thr Lys Asp Arg Gly Pro Pro Val Gln Ser Gln
            100                 105                 110

Ile Trp Arg Ser Gly Glu Lys Val Pro Phe Val Gln Thr Tyr Ser Leu
        115                 120                 125

Arg Ala Phe Glu Lys Pro Pro Gln Val Gln Thr Gln Ala Leu Arg Asp
    130                 135                 140

Phe Glu Lys His Leu Asn Asp Leu Lys Lys Glu Asn Phe Ser Leu Lys
145                 150                 155                 160

Leu Arg Ile Tyr Phe Leu Glu Glu Arg Met Gln Gln Lys Tyr Glu Ala
                165                 170                 175

Ser Arg Glu Asp Ile Tyr Lys Arg Asn Ile Glu Leu Lys Val Glu Val
            180                 185                 190

Glu Ser Leu Lys Arg Glu Leu Gln Asp Lys Lys Gln His Leu Asp Lys
        195                 200                 205

Thr Trp Ala Asp Val Glu Asn Leu Asn Ser Gln Asn Glu Ala Glu Leu
    210                 215                 220

Arg Arg Gln Phe Glu Glu Arg Gln Gln Glu Thr Glu His Val Tyr Glu
225                 230                 235                 240

Leu Leu Glu Asn Lys Ile Gln Leu Leu Gln Glu Glu Ser Arg Leu Ala
                245                 250                 255

Lys Asn Glu Ala Ala Arg Met Ala Ala Leu Val Glu Ala Glu Lys Glu
            260                 265                 270

Cys Asn Leu Glu Leu Ser Glu Lys Leu Lys Gly Val Thr Lys Asn Trp
        275                 280                 285

Glu Asp Val Pro Gly Asp Gln Val Lys Pro Asp Gln Tyr Thr Glu Ala
    290                 295                 300

Leu Ala Gln Arg Asp Lys
305                 310

<210>  10
<211>  282
<212>  PRT
<213>  Homo sapiens

<400>  10

Met Ser Gly Gly Trp Met Ala Gln Val Gly Ala Trp Arg Thr Gly Ala
1               5                   10                  15

Leu Gly Leu Ala Leu Leu Leu Leu Leu Gly Leu Gly Leu Gly Leu Glu
            20                  25                  30

Ala Ala Ala Ser Pro Leu Ser Thr Pro Thr Ser Ala Gln Ala Ala Gly
        35                  40                  45

Pro Ser Ser Gly Ser Cys Pro Pro Thr Lys Phe Gln Cys Arg Thr Ser
    50                  55                  60

Gly Leu Cys Val Pro Leu Thr Trp Arg Cys Asp Arg Asp Leu Asp Cys
65                  70                  75                  80

Ser Asp Gly Ser Asp Glu Glu Glu Cys Arg Ile Glu Pro Cys Thr Gln
                85                  90                  95

Lys Gly Gln Cys Pro Pro Pro Pro Gly Leu Pro Cys Pro Cys Thr Gly
            100                 105                 110

Val Ser Asp Cys Ser Gly Gly Thr Asp Lys Lys Leu Arg Asn Cys Ser
        115                 120                 125

Arg Leu Ala Cys Leu Ala Gly Glu Leu Arg Cys Thr Leu Ser Asp Asp
    130                 135                 140

Cys Ile Pro Leu Thr Trp Arg Cys Asp Gly His Pro Asp Cys Pro Asp
145                 150                 155                 160

Ser Ser Asp Glu Leu Gly Cys Gly Thr Asn Glu Ile Leu Pro Glu Gly
                165                 170                 175

Asp Ala Thr Thr Met Gly Pro Pro Val Thr Leu Glu Ser Val Thr Ser
            180                 185                 190

Leu Arg Asn Ala Thr Thr Met Gly Pro Pro Val Thr Leu Glu Ser Val
        195                 200                 205

Pro Ser Val Gly Asn Ala Thr Ser Ser Ser Ala Gly Asp Gln Ser Gly
    210                 215                 220

Ser Pro Thr Ala Tyr Gly Val Ile Ala Ala Ala Ala Val Leu Ser Ala
225                 230                 235                 240

Ser Leu Val Thr Ala Thr Leu Leu Leu Leu Ser Trp Leu Arg Ala Gln
                245                 250                 255

Glu Arg Leu Arg Pro Leu Gly Leu Leu Val Ala Met Lys Glu Ser Leu
            260                 265                 270

Leu Leu Ser Glu Gln Lys Thr Ser Leu Pro
        275                 280

<210>  11
<211>  249
<212>  PRT
<213>  Homo sapiens

<400>  11

Met Ala Ser Arg Arg Met Glu Thr Lys Pro Val Ile Thr Cys Leu Lys
1               5                   10                  15

Thr Leu Leu Ile Ile Tyr Ser Phe Val Phe Trp Ile Thr Gly Val Ile
            20                  25                  30

Leu Leu Ala Val Gly Val Trp Gly Lys Leu Thr Leu Gly Thr Tyr Ile
        35                  40                  45

Ser Leu Ile Ala Glu Asn Ser Thr Asn Ala Pro Tyr Val Leu Ile Gly
    50                  55                  60

Thr Gly Thr Thr Ile Val Val Phe Gly Leu Phe Gly Cys Phe Ala Thr
65                  70                  75                  80

Cys Arg Gly Ser Pro Trp Met Leu Lys Leu Tyr Ala Met Phe Leu Ser
                85                  90                  95

Leu Val Phe Leu Ala Glu Leu Val Ala Gly Ile Ser Gly Phe Val Phe
            100                 105                 110

Arg His Glu Ile Lys Asp Thr Phe Leu Arg Thr Tyr Thr Asp Ala Met
        115                 120                 125

Gln Thr Tyr Asn Gly Asn Asp Glu Arg Ser Arg Ala Val Asp His Val
    130                 135                 140

Gln Arg Ser Leu Ser Cys Cys Gly Val Gln Asn Tyr Thr Asn Trp Ser
145                 150                 155                 160

Thr Ser Pro Tyr Phe Leu Glu His Gly Ile Pro Pro Ser Cys Cys Met
                165                 170                 175

Asn Glu Thr Asp Cys Asn Pro Gln Asp Leu His Asn Leu Thr Val Ala
            180                 185                 190

Ala Thr Lys Val Asn Gln Lys Gly Cys Tyr Asp Leu Val Thr Ser Phe
        195                 200                 205

Met Glu Thr Asn Met Gly Ile Ile Ala Gly Val Ala Phe Gly Ile Ala
    210                 215                 220

Phe Ser Gln Leu Ile Gly Met Leu Leu Ala Cys Cys Leu Ser Arg Phe
225                 230                 235                 240

Ile Thr Ala Asn Gln Tyr Glu Met Val
                245

<210> 12
<211> 875
<212> PRT
<213> Homo sapiens

<400> 12

Met Asp Pro Ala Pro Ser Leu Gly Cys Ser Leu Lys Asp Val Lys Trp
1               5                   10                  15

Ser Ser Val Ala Val Pro Leu Asp Leu Leu Val Ser Thr Tyr Arg Leu
            20                  25                  30

Pro Gln Ile Ala Arg Leu Asp Asn Gly Glu Cys Val Glu Gly Leu Arg
        35                  40                  45

Glu Asn Asp Tyr Leu Leu Ile His Ser Cys Arg Gln Trp Thr Thr Ile
    50                  55                  60

Thr Ala His Ser Leu Glu Glu Gly His Tyr Val Ile Gly Pro Lys Ile
65                  70                  75                  80

Glu Ile Pro Val His Tyr Ala Gly Gln Phe Lys Leu Leu Glu Gln Asp
                85                  90                  95

Arg Asp Ile Lys Glu Pro Val Gln Tyr Phe Asn Ser Val Glu Glu Val
            100                 105                 110

Ala Lys Ala Phe Pro Glu Arg Val Tyr Val Met Glu Asp Ile Thr Phe
        115                 120                 125

Asn Val Lys Val Ala Ser Gly Glu Cys Asn Glu Asp Thr Glu Val Tyr
    130                 135                 140

Asn Ile Thr Leu Cys Thr Gly Asp Glu Leu Thr Leu Met Gly Gln Ala
145                 150                 155                 160

Glu Ile Leu Tyr Ala Lys Thr Phe Lys Glu Lys Ser Arg Leu Asn Thr
                165                 170                 175

Ile Phe Lys Lys Ile Gly Lys Leu Asn Ser Ile Ser Lys Leu Gly Lys
            180                 185                 190

Gly Lys Met Pro Cys Leu Ile Cys Met Asn His Arg Thr Asn Glu Ser
        195                 200                 205

Ile Ser Leu Pro Phe Gln Cys Lys Gly Arg Phe Ser Thr Arg Ser Pro
    210                 215                 220

Leu Glu Leu Gln Met Gln Glu Gly Glu His Thr Ile Arg Asn Ile Val
225                 230                 235                 240

Glu Lys Thr Arg Leu Pro Val Asn Val Thr Val Pro Ser Pro Pro Pro
                245                 250                 255

Arg Asn Pro Tyr Asp Leu His Phe Ile Arg Glu Gly His Arg Tyr Lys
            260                 265                 270

Phe Val Asn Ile Gln Thr Lys Thr Val Val Val Cys Cys Val Leu Arg
        275                 280                 285

Asn Asn Lys Ile Leu Pro Met His Phe Pro Leu His Leu Thr Val Pro
    290                 295                 300

Lys Phe Ser Leu Pro Glu His Leu Val Lys Gly Glu Ser Trp Pro Glu
305                 310                 315                 320

Thr Leu Val His His Trp Leu Gly Ile Cys Gln Glu Gln Phe Asp Ile
                325                 330                 335

Asp Glu Tyr Ser Arg Ala Val Arg Asp Val Lys Thr Asp Trp Asn Glu
            340                 345                 350

Glu Cys Lys Ser Pro Lys Lys Gly Arg Cys Ser Gly His Asn His Val
        355                 360                 365

Pro Asn Ser Leu Ser Tyr Ala Arg Asp Glu Leu Thr Gln Ser Phe His
    370                 375                 380

Arg Leu Ser Val Cys Val Tyr Gly Asn Asn Leu His Gly Asn Ser Glu
385                 390                 395                 400

Val Asn Leu His Gly Cys Arg Asp Leu Gly Gly Asp Trp Ala Pro Phe
                405                 410                 415

Pro His Asp Ile Leu Pro Tyr Gln Asp Ser Gly Asp Ser Gly Ser Asp
            420                 425                 430

Tyr Leu Phe Pro Glu Ala Ser Glu Glu Ser Ala Gly Ile Pro Gly Lys
        435                 440                 445

Ser Glu Leu Pro Tyr Glu Glu Leu Trp Leu Glu Glu Gly Lys Pro Ser
    450                 455                 460

His Gln Pro Leu Thr Arg Ser Leu Ser Glu Lys Asn Arg Cys Asp Gln
465                 470                 475                 480

Phe Arg Gly Ser Val Arg Ser Lys Cys Ala Thr Ser Pro Leu Pro Ile
                485                 490                 495

Pro Gly Thr Leu Gly Ala Ala Val Lys Ser Ser Asp Thr Ala Leu Pro
            500                 505                 510

Pro Pro Pro Val Pro Pro Lys Ser Glu Ala Val Arg Glu Glu Cys Arg
        515                 520                 525

Leu Leu Asn Ala Pro Pro Val Pro Pro Arg Ser Ala Lys Pro Leu Ser
    530                 535                 540

Thr Ser Pro Ser Ile Pro Pro Arg Thr Val Lys Pro Ala Arg Gln Gln
545                 550                 555                 560

Thr Arg Ser Pro Ser Pro Thr Leu Ser Tyr Tyr Ser Ser Gly Leu His
                565                 570                 575

Asn Ile Val Thr Lys Thr Asp Thr Asn Pro Ser Glu Ser Thr Pro Val
            580                 585                 590

Ser Cys Tyr Pro Cys Asn Arg Val Lys Thr Asp Ser Val Asp Leu Lys
        595                 600                 605

Ser Pro Phe Gly Ser Pro Ser Ala Glu Ala Val Ser Ser Arg Leu Ser
    610                 615                 620

Trp Pro Asn His Tyr Ser Gly Ala Ser Glu Ser Gln Thr Arg Ser Asp
625                 630                 635                 640

Phe Leu Leu Asp Pro Ser Arg Ser Tyr Ser Tyr Pro Arg Gln Lys Thr
                645                 650                 655

Pro Gly Thr Pro Lys Arg Asn Cys Pro Ala Pro Phe Asp Phe Asp Gly

            660                 665                 670

Cys Glu Leu Leu Ala Ser Pro Thr Ser Pro Val Thr Ala Glu Phe Ser
        675                 680                 685

Ser Ser Val Ser Gly Cys Pro Lys Ser Ala Ser Tyr Ser Leu Glu Ser
    690                 695                 700

Thr Asp Val Lys Ser Leu Ala Ala Gly Val Thr Lys Gln Ser Thr Ser
705                 710                 715                 720

Cys Pro Ala Leu Pro Pro Arg Ala Pro Lys Leu Val Glu Glu Lys Val
                725                 730                 735

Ala Ser Glu Thr Ser Pro Leu Pro Leu Lys Ile Asp Gly Ala Glu Glu
            740                 745                 750

Asp Pro Lys Ser Gly Ser Pro Asp Leu Ser Glu Asp Gln Tyr Phe Val
        755                 760                 765

Lys Lys Gly Met Gln Asp Ile Phe Ser Ala Ser Tyr Pro Phe Ser Ser
    770                 775                 780

Pro Leu His Leu Gln Leu Ala Pro Arg Ser Cys Gly Asp Gly Ser Pro
785                 790                 795                 800

Trp Gln Pro Pro Ala Asp Leu Ser Gly Leu Ser Ile Glu Glu Val Ser
                805                 810                 815

Lys Ser Leu Arg Phe Ile Gly Leu Ser Glu Asp Val Ile Ser Phe Phe
            820                 825                 830

Val Thr Glu Lys Ile Asp Gly Asn Leu Leu Val Gln Leu Thr Glu Glu
        835                 840                 845

Ile Leu Ser Glu Asp Phe Lys Leu Ser Lys Leu Gln Val Lys Lys Ile
    850                 855                 860

Met Gln Phe Ile Asn Gly Trp Arg Pro Lys Ile
865                 870                 875

<210>  13
<211>  466
<212>  PRT
<213>  Homo sapiens

<400>  13

Met Ser Thr Arg Ser Val Ser Ser Ser Ser Tyr Arg Arg Met Phe Gly
1               5                   10                  15

Gly Pro Gly Thr Ala Ser Arg Pro Ser Ser Ser Arg Ser Tyr Val Thr
            20                  25                  30

Thr Ser Thr Arg Thr Tyr Ser Leu Gly Ser Ala Leu Arg Pro Ser Thr
        35                  40                  45

Ser Arg Ser Leu Tyr Ala Ser Ser Pro Gly Gly Val Tyr Ala Thr Arg
    50                  55                  60

Ser Ser Ala Val Arg Leu Arg Ser Ser Val Pro Gly Val Arg Leu Leu
65                  70                  75                  80

Gln Asp Ser Val Asp Phe Ser Leu Ala Asp Ala Ile Asn Thr Glu Phe
                85                  90                  95

Lys Asn Thr Arg Thr Asn Glu Lys Val Glu Leu Gln Glu Leu Asn Asp
            100                 105                 110

Arg Phe Ala Asn Tyr Ile Asp Lys Val Arg Phe Leu Glu Gln Gln Asn
        115                 120                 125

Lys Ile Leu Leu Ala Glu Leu Glu Gln Leu Lys Gly Gln Gly Lys Ser
    130                 135                 140

Arg Leu Gly Asp Leu Tyr Glu Glu Glu Met Arg Glu Leu Arg Arg Gln
145                 150                 155                 160

Val Asp Gln Leu Thr Asn Asp Lys Ala Arg Val Glu Val Glu Arg Asp
                165                 170                 175

Asn Leu Ala Glu Asp Ile Met Arg Leu Arg Glu Lys Leu Gln Glu Glu
                180                 185                 190

Met Leu Gln Arg Glu Glu Ala Glu Asn Thr Leu Gln Ser Phe Arg Gln
            195                 200                 205

Asp Val Asp Asn Ala Ser Leu Ala Arg Leu Asp Leu Glu Arg Lys Val
    210                 215                 220

Glu Ser Leu Gln Glu Glu Ile Ala Phe Leu Lys Lys Leu His Glu Glu
225                 230                 235                 240

Glu Ile Gln Glu Leu Gln Ala Gln Ile Gln Glu Gln His Val Gln Ile
                245                 250                 255

Asp Val Asp Val Ser Lys Pro Asp Leu Thr Ala Ala Leu Arg Asp Val
                260                 265                 270

Arg Gln Gln Tyr Glu Ser Val Ala Ala Lys Asn Leu Gln Glu Ala Glu
            275                 280                 285

Glu Trp Tyr Lys Ser Lys Phe Ala Asp Leu Ser Glu Ala Ala Asn Arg
    290                 295                 300

Asn Asn Asp Ala Leu Arg Gln Ala Lys Gln Glu Ser Thr Glu Tyr Arg
305                 310                 315                 320

Arg Gln Val Gln Ser Leu Thr Cys Glu Val Asp Ala Leu Lys Gly Thr
                325                 330                 335

Asn Glu Ser Leu Glu Arg Gln Met Arg Glu Met Glu Glu Asn Phe Ala
                340                 345                 350

Val Glu Ala Ala Asn Tyr Gln Asp Thr Ile Gly Arg Leu Gln Asp Glu
            355                 360                 365

Ile Gln Asn Met Lys Glu Glu Met Ala Arg His Leu Arg Glu Tyr Gln
    370                 375                 380

Asp Leu Leu Asn Val Lys Met Ala Leu Asp Ile Glu Ile Ala Thr Tyr

385                 390                 395                 400

Arg Lys Leu Leu Glu Gly Glu Glu Ser Arg Ile Ser Leu Pro Leu Pro
                405                 410                 415

Asn Phe Ser Ser Leu Asn Leu Arg Glu Thr Asn Leu Asp Ser Leu Pro
            420                 425                 430

Leu Val Asp Thr His Ser Lys Arg Thr Leu Leu Ile Lys Thr Val Glu
        435                 440                 445

Thr Arg Asp Gly Gln Val Ile Asn Glu Thr Ser Gln His His Asp Asp
    450                 455                 460

Leu Glu
465

<210>  14
<211>  159
<212>  PRT
<213>  Homo sapiens

<400>  14

Met Pro Lys Phe Tyr Cys Asp Tyr Cys Asp Thr Tyr Leu Thr His Asp
1               5                   10                  15

Ser Pro Ser Val Arg Lys Thr His Cys Ser Gly Arg Lys His Lys Glu
            20                  25                  30

Asn Val Lys Asp Tyr Tyr Gln Lys Trp Met Glu Glu Gln Ala Gln Ser
        35                  40                  45

Leu Ile Asp Lys Thr Thr Ala Ala Phe Gln Gln Gly Lys Ile Pro Pro
    50                  55                  60

Thr Pro Phe Ser Ala Pro Pro Pro Ala Gly Ala Met Ile Pro Pro Pro
65                  70                  75                  80

Pro Ser Leu Pro Gly Pro Pro Arg Pro Gly Met Met Pro Ala Pro His
                85                  90                  95

Met Gly Gly Pro Pro Met Met Pro Met Met Gly Pro Pro Pro Pro Gly
            100                 105                 110

Met Met Pro Val Gly Pro Ala Pro Gly Met Arg Pro Pro Met Gly Gly
        115                 120                 125

His Met Pro Met Met Pro Gly Pro Pro Met Met Arg Pro Pro Ala Arg
    130                 135                 140

Pro Met Met Val Pro Thr Arg Pro Gly Met Thr Arg Pro Asp Arg
145                 150                 155

<210>  15
<211>  470
<212>  PRT
<213>  Homo sapiens

<400>  15

Met Pro Gly Phe Asp Tyr Lys Phe Leu Glu Lys Pro Lys Arg Arg Leu
1               5                   10                  15

Leu Cys Pro Leu Cys Gly Lys Pro Met Arg Glu Pro Val Gln Val Ser
            20                  25                  30

Thr Cys Gly His Arg Phe Cys Asp Thr Cys Leu Gln Glu Phe Leu Ser
        35                  40                  45

Glu Gly Val Phe Lys Cys Pro Glu Asp Gln Leu Pro Leu Asp Tyr Ala
    50                  55                  60

Lys Ile Tyr Pro Asp Pro Glu Leu Glu Val Gln Val Leu Gly Leu Pro
65                  70                  75                  80

Ile Arg Cys Ile His Ser Glu Glu Gly Cys Arg Trp Ser Gly Pro Leu
                85                  90                  95

Arg His Leu Gln Gly His Leu Asn Thr Cys Ser Phe Asn Val Ile Pro
            100                 105                 110

Cys Pro Asn Arg Cys Pro Met Lys Leu Ser Arg Arg Asp Leu Pro Ala

115                 120                 125

His Leu Gln His Asp Cys Pro Lys Arg Arg Leu Lys Cys Glu Phe Cys
    130                 135                 140

Gly Cys Asp Phe Ser Gly Glu Ala Tyr Glu Ser His Glu Gly Met Cys
145                 150                 155                 160

Pro Gln Glu Ser Val Tyr Cys Glu Asn Lys Cys Gly Ala Arg Met Met
                165                 170                 175

Arg Arg Leu Leu Ala Gln His Ala Thr Ser Glu Cys Pro Lys Arg Thr
            180                 185                 190

Gln Pro Cys Thr Tyr Cys Thr Lys Glu Phe Val Phe Asp Thr Ile Gln
        195                 200                 205

Ser His Gln Tyr Gln Cys Pro Arg Leu Pro Val Ala Cys Pro Asn Gln
    210                 215                 220

Cys Gly Val Gly Thr Val Ala Arg Glu Asp Leu Pro Gly His Leu Lys
225                 230                 235                 240

Asp Ser Cys Asn Thr Ala Leu Val Leu Cys Pro Phe Lys Asp Ser Gly
                245                 250                 255

Cys Lys His Arg Cys Pro Lys Leu Ala Met Ala Arg His Val Glu Glu
            260                 265                 270

Ser Val Lys Pro His Leu Ala Met Met Cys Ala Leu Val Ser Arg Gln
        275                 280                 285

Arg Gln Glu Leu Gln Glu Leu Arg Arg Glu Leu Glu Glu Leu Ser Val
    290                 295                 300

Gly Ser Asp Gly Val Leu Ile Trp Lys Ile Gly Ser Tyr Gly Arg Arg
305                 310                 315                 320

Leu Gln Glu Ala Lys Ala Lys Pro Asn Leu Glu Cys Phe Ser Pro Ala
                325                 330                 335

Phe Tyr Thr His Lys Tyr Gly Tyr Lys Leu Gln Val Ser Ala Phe Leu
            340                 345                 350

Asn Gly Asn Gly Ser Gly Glu Gly Thr His Leu Ser Leu Tyr Ile Arg
        355                 360                 365

Val Leu Pro Gly Ala Phe Asp Asn Leu Leu Glu Trp Pro Phe Ala Arg
    370                 375                 380

Arg Val Thr Phe Ser Leu Leu Asp Gln Ser Asp Pro Gly Leu Ala Lys
385                 390                 395                 400

Pro Gln His Val Thr Glu Thr Phe His Pro Asp Pro Asn Trp Lys Asn
                405                 410                 415

Phe Gln Lys Pro Gly Thr Trp Arg Gly Ser Leu Asp Glu Ser Ser Leu
            420                 425                 430

Gly Phe Gly Tyr Pro Lys Phe Ile Ser His Gln Asp Ile Arg Lys Arg
        435                 440                 445

Asn Tyr Val Arg Asp Asp Ala Val Phe Ile Arg Ala Ala Val Glu Leu
    450                 455                 460

Pro Arg Lys Ile Leu Ser
465                 470

<210>  16
<211>  559
<212>  PRT
<213>  Homo sapiens

<400>  16

Met Ser Phe Ser Gln Cys Asn Pro Glu Lys Arg Cys Glu Ile Lys Thr
1               5                   10                  15

Ala Ala Gln Gly Ser Arg Met Glu Gly Leu Leu Ser Val Ala Leu Gln
            20                  25                  30

Gly Ala Glu Leu Glu Gly Asn Trp Lys His Glu Gly Gln Val Glu Asp
        35                  40                  45

Leu Gln Glu Asn Gln Glu Ser Cys Pro Glu Pro Glu Ala Val Ala Cys
    50                  55                  60

Lys Gly Asp Pro Ala Gly Asp Ser Met Gln Glu Arg Asp Glu Phe Ser
65                  70                  75                  80

Arg Ile Pro Arg Thr Ile Ser Ser Pro Ala Ala Thr Gln Ala Ser Val
                85                  90                  95

Pro Asp Asp Ser Ser Ser Arg Arg Cys Ser Ala Pro Gly Glu Ser Pro
            100                 105                 110

Lys Glu Arg His Pro Asp Ser Arg Gln Arg Glu Arg Gly Gly Gly Pro
        115                 120                 125

Lys Lys Pro Trp Lys Cys Gly Asp Cys Gly Lys Ala Phe Ser Tyr Cys
    130                 135                 140

Ser Ala Phe Ile Leu His Gln Arg Ile His Thr Gly Glu Lys Pro Phe
145                 150                 155                 160

Ala Cys Pro Glu Cys Gly Lys Ala Phe Ser Gln Ser Val His Leu Thr
                165                 170                 175

Leu His Gln Arg Thr His Thr Gly Glu Lys Pro Tyr Ala Cys His Glu
            180                 185                 190

Cys Gly Lys Ala Phe Ser Gln Gly Ser Tyr Leu Ala Ser His Trp Arg
        195                 200                 205

Thr His Thr Gly Glu Lys Pro His Arg Cys Ala Asp Cys Gly Lys Ala
    210                 215                 220

Phe Thr Arg Val Thr His Leu Thr Gln His Arg Arg Val His Thr Gly
225                 230                 235                 240

Glu Arg Pro Tyr Ala Cys Ala Gln Cys Ala Lys Ala Phe Arg Asn Arg

                245                 250                 255

Ser Ser Leu Ile Glu His Gln Arg Ile His Thr Gly Glu Lys Pro Tyr
            260                 265                 270

Glu Cys Ser Ala Cys Ala Lys Ala Phe Arg Phe Ser Ser Ala Leu Ile
        275                 280                 285

Arg His Gln Arg Ile His Thr Glu Glu Lys Pro Tyr Arg Cys Gly Gln
    290                 295                 300

Cys Ala Lys Ala Phe Ala Gln Ile Ala His Leu Thr Gln His Arg Arg
305                 310                 315                 320

Val His Thr Gly Glu Lys Pro Tyr Thr Cys Gln Asp Cys Gly Ala Leu
                325                 330                 335

Phe Ser Gln Ser Ala Ser Leu Ala Glu His Arg Arg Ile His Thr Gly
            340                 345                 350

Glu Lys Pro Tyr Ala Cys Gly Gln Cys Ala Lys Ala Phe Thr Gln Val
        355                 360                 365

Ser His Leu Thr Gln His Gln Arg Thr His Thr Gly Glu Arg Pro Tyr
    370                 375                 380

Pro Cys His Asp Cys Gly Lys Arg Phe Ser Asn Arg Ser His Leu Leu
385                 390                 395                 400

Gln His Arg Leu Val His Thr Gly Glu Arg Pro Tyr Arg Cys Leu Gln
                405                 410                 415

Cys Gly Ala Ala Phe Ser His Val Ser Ser Leu Ile Glu His Gln Lys
            420                 425                 430

Ile His Thr Gly Glu Arg Pro Tyr Lys Cys Gly Glu Cys Gly Lys Ala
        435                 440                 445

Phe Ser Gln Gly Ser Ser Leu Ala Leu His Gln Arg Thr His Thr Gly
    450                 455                 460

Glu Arg Pro Tyr Thr Cys Pro Glu Cys Gly Lys Ala Phe Ser Asn Arg
465                 470                 475                 480

Ser Tyr Leu Ile Gln His His Ile Val His Thr Gly Glu Lys Pro Tyr
                485                 490                 495

Glu Cys Ser Gly Cys Gly Lys Ala Phe Ser Phe Ser Ser Ala Leu Ile
            500                 505                 510

Arg His Gln Arg Thr His Ala Asp Ser Ser Gly Arg Leu Cys Pro Ala
        515                 520                 525

Pro Thr Pro Asp Ser Thr Pro Gly Leu Ser Gln Gly Gly Glu Thr Cys
    530                 535                 540

Gln Gln Gly Cys Pro Gly Arg Asn Pro Arg Gly Pro Ala Glu Asp
545                 550                 555


<210>  17
<211>  261
<212>  PRT
<213>  Homo sapiens

<400>  17

Met Ala Ala Ala Val Pro Arg Ala Ala Phe Leu Ser Pro Leu Leu Pro
1               5                   10                  15

Leu Leu Leu Gly Phe Leu Leu Leu Ser Ala Pro His Gly Gly Ser Gly
            20                  25                  30

Leu His Thr Lys Gly Ala Leu Pro Leu Asp Thr Val Thr Phe Tyr Lys
        35                  40                  45

Val Ile Pro Lys Ser Lys Phe Val Leu Val Lys Phe Asp Thr Gln Tyr
    50                  55                  60

Pro Tyr Gly Glu Lys Gln Asp Glu Phe Lys Arg Leu Ala Glu Asn Ser
65                  70                  75                  80

Ala Ser Ser Asp Asp Leu Leu Val Ala Glu Val Gly Ile Ser Asp Tyr
                85                  90                  95

Gly Asp Lys Leu Asn Met Glu Leu Ser Glu Lys Tyr Lys Leu Asp Lys
            100                 105                 110

Glu Ser Tyr Pro Val Phe Tyr Leu Phe Arg Asp Gly Asp Phe Glu Asn
        115                 120                 125

Pro Val Pro Tyr Thr Gly Ala Val Lys Val Gly Ala Ile Gln Arg Trp
    130                 135                 140

Leu Lys Gly Gln Gly Val Tyr Leu Gly Met Pro Gly Cys Leu Pro Val
145                 150                 155                 160

Tyr Asp Ala Leu Ala Gly Glu Phe Ile Arg Ala Ser Gly Val Glu Ala
                165                 170                 175

Arg Gln Ala Leu Leu Lys Gln Gly Gln Asp Asn Leu Ser Ser Val Lys
            180                 185                 190

Glu Thr Gln Lys Lys Trp Ala Glu Gln Tyr Leu Lys Ile Met Gly Lys
        195                 200                 205

Ile Leu Asp Gln Gly Glu Asp Phe Pro Ala Ser Glu Met Thr Arg Ile
    210                 215                 220

Ala Arg Leu Ile Glu Lys Asn Lys Met Ser Asp Gly Lys Lys Glu Glu
225                 230                 235                 240

Leu Gln Lys Ser Leu Asn Ile Leu Thr Ala Phe Gln Lys Lys Gly Ala
                245                 250                 255

Glu Lys Glu Glu Leu
            260

<210>  18
<211>  599
<212>  PRT
<213>  Homo sapiens

<400>  18

Met Gly Pro Lys Arg Arg Gln Leu Thr Phe Arg Glu Lys Ser Arg Ile
1               5                   10                  15

Ile Gln Glu Val Glu Glu Asn Pro Asp Leu Arg Lys Gly Glu Ile Ala
            20                  25                  30

Arg Arg Phe Asn Ile Pro Pro Ser Thr Leu Ser Thr Ile Leu Lys Asn
        35                  40                  45

Lys Arg Ala Ile Leu Ala Ser Glu Arg Lys Tyr Gly Val Ala Ser Thr
    50                  55                  60

Cys Arg Lys Thr Asn Lys Leu Ser Pro Tyr Asp Lys Leu Glu Gly Leu
65                  70                  75                  80

Leu Ile Ala Trp Phe Gln Gln Ile Arg Ala Ala Gly Leu Pro Val Lys
                85                  90                  95

Gly Ile Ile Leu Lys Glu Lys Ala Leu Arg Ile Ala Glu Glu Leu Gly
            100                 105                 110

Met Asp Asp Phe Thr Ala Ser Asn Gly Trp Leu Asp Arg Phe Arg Arg
        115                 120                 125

Arg His Gly Val Val Ser Cys Ser Gly Val Ala Arg Ala Arg Ala Arg
    130                 135                 140

Asn Ala Ala Pro Arg Thr Pro Ala Ala Pro Ala Ser Pro Ala Ala Val
145                 150                 155                 160

Pro Ser Glu Gly Ser Gly Gly Ser Thr Thr Gly Trp Arg Ala Arg Glu
                165                 170                 175

Glu Gln Pro Pro Ser Val Ala Glu Gly Tyr Ala Ser Gln Asp Val Phe
            180                 185                 190

Ser Ala Thr Glu Thr Ser Leu Trp Tyr Asp Phe Leu Pro Asp Gln Ala
        195                 200                 205

Ala Gly Leu Cys Gly Gly Asp Gly Arg Pro Arg Gln Ala Thr Gln Arg
    210                 215                 220

Leu Ser Val Leu Leu Cys Ala Asn Ala Asp Gly Ser Glu Lys Leu Pro
225                 230                 235                 240

Pro Leu Val Ala Gly Lys Ser Ala Lys Pro Arg Ala Gly Gln Ala Gly
                245                 250                 255

Leu Pro Cys Asp Tyr Thr Ala Asn Ser Lys Gly Gly Val Thr Thr Gln
            260                 265                 270

Ala Leu Ala Lys Tyr Leu Lys Ala Leu Asp Thr Arg Met Ala Ala Glu
        275                 280                 285

Ser Arg Arg Val Leu Leu Leu Ala Gly Arg Leu Ala Ala Gln Ser Leu
    290                 295                 300

Asp Thr Ser Gly Leu Arg His Val Gln Leu Ala Phe Phe Pro Pro Gly
305                 310                 315                 320

Thr Val His Pro Leu Glu Arg Gly Val Val Gln Gln Val Lys Gly His
                325                 330                 335

Tyr Arg Gln Ala Met Leu Leu Lys Ala Met Ala Ala Leu Glu Gly Gln
            340                 345                 350

Asp Pro Ser Gly Leu Gln Leu Gly Leu Thr Glu Ala Leu His Phe Val
        355                 360                 365

Ala Ala Ala Trp Gln Ala Val Glu Pro Ser Asp Ile Ala Ala Cys Phe
    370                 375                 380

Arg Glu Ala Gly Phe Gly Gly Gly Pro Asn Ala Thr Ile Thr Thr Ser
385                 390                 395                 400

Leu Lys Ser Glu Gly Glu Glu Glu Glu Glu Glu Glu Glu Glu Glu Glu
                405                 410                 415

Glu Glu Glu Gly Glu Gly Glu Glu Glu Glu Glu Glu Gly Glu Glu Glu
            420                 425                 430

Glu Glu Glu Gly Gly Glu Gly Glu Glu Leu Gly Glu Glu Glu Glu Val
        435                 440                 445

Glu Glu Glu Gly Asp Val Asp Ser Asp Glu Glu Glu Glu Glu Asp Glu
    450                 455                 460

Glu Ser Ser Ser Glu Gly Leu Glu Ala Glu Asp Trp Ala Gln Gly Val
465                 470                 475                 480

Val Glu Ala Gly Gly Ser Phe Gly Ala Tyr Gly Ala Gln Glu Glu Ala
                485                 490                 495

Gln Cys Pro Thr Leu His Phe Leu Glu Gly Gly Glu Asp Ser Asp Ser
            500                 505                 510

Asp Ser Glu Glu Glu Asp Asp Glu Glu Glu Asp Asp Glu Asp Glu Asp
        515                 520                 525

Asp Asp Asp Asp Glu Glu Asp Gly Asp Glu Val Pro Val Pro Ser Phe
    530                 535                 540

Gly Glu Ala Met Ala Tyr Phe Ala Met Val Lys Arg Tyr Leu Thr Ser
545                 550                 555                 560

Phe Pro Ile Asp Asp Arg Val Gln Ser His Ile Leu His Leu Glu His
                565                 570                 575

Asp Leu Val His Val Thr Arg Lys Asn His Ala Arg Gln Ala Gly Val
            580                 585                 590

Arg Gly Leu Gly His Gln Ser
        595

<210>  19
<211>  323
<212>  PRT
<213>  Homo sapiens

<400>  19

Met Ala Pro Lys Gln Asp Pro Lys Pro Lys Phe Gln Glu Gly Glu Arg
1               5                   10                  15

Val Leu Cys Phe His Gly Pro Leu Leu Tyr Glu Ala Lys Cys Val Lys
            20                  25                  30

Val Ala Ile Lys Asp Lys Gln Val Lys Tyr Phe Ile His Tyr Ser Gly
        35                  40                  45

Trp Asn Lys Asn Trp Asp Glu Trp Val Pro Glu Ser Arg Val Leu Lys
    50                  55                  60

Tyr Val Asp Thr Asn Leu Gln Lys Gln Arg Glu Leu Gln Lys Ala Asn
65                  70                  75                  80

Gln Glu Gln Tyr Ala Glu Gly Lys Met Arg Gly Ala Ala Pro Gly Lys
                85                  90                  95

Lys Thr Ser Gly Leu Gln Gln Lys Asn Val Glu Val Lys Thr Lys Lys
            100                 105                 110

Asn Lys Gln Lys Thr Pro Gly Asn Gly Asp Gly Gly Ser Thr Ser Glu
        115                 120                 125

Thr Pro Gln Pro Pro Arg Lys Lys Arg Ala Arg Val Asp Pro Thr Val
    130                 135                 140

Glu Asn Glu Glu Thr Phe Met Asn Arg Val Glu Val Lys Val Lys Ile
145                 150                 155                 160

Pro Glu Glu Leu Lys Pro Trp Leu Val Asp Asp Trp Asp Leu Ile Thr
                165                 170                 175

Arg Gln Lys Gln Leu Phe Tyr Leu Pro Ala Lys Lys Asn Val Asp Ser
            180                 185                 190

Ile Leu Glu Asp Tyr Ala Asn Tyr Lys Lys Ser Arg Gly Asn Thr Asp
        195                 200                 205

Asn Lys Glu Tyr Ala Val Asn Glu Val Val Ala Gly Ile Lys Glu Tyr
    210                 215                 220

Phe Asn Val Met Leu Gly Thr Gln Leu Leu Tyr Lys Phe Glu Arg Pro
225                 230                 235                 240

Gln Tyr Ala Glu Ile Leu Ala Asp His Pro Asp Ala Pro Met Ser Gln
                245                 250                 255

Val Tyr Gly Ala Pro His Leu Leu Arg Leu Phe Val Arg Ile Gly Ala
            260                 265                 270

Met Leu Ala Tyr Thr Pro Leu Asp Glu Lys Ser Leu Ala Leu Leu Leu
        275                 280                 285

Asn Tyr Leu His Asp Phe Leu Lys Tyr Leu Ala Lys Asn Ser Ala Thr
    290                 295                 300

Leu Phe Ser Ala Ser Asp Tyr Glu Val Ala Pro Pro Glu Tyr His Arg
305                 310                 315                 320

Lys Ala Val

<210>  20
<211>  480
<212>  PRT
<213>  Homo sapiens

<400>  20

Met Glu Thr Asp Leu Asn Ser Gln Asp Arg Lys Asp Leu Asp Lys Phe
1               5                   10                  15

Ile Lys Phe Phe Ala Leu Lys Thr Val Gln Val Ile Val Gln Ala Arg
            20                  25                  30

Leu Gly Glu Lys Ile Cys Thr Arg Ser Ser Ser Ser Pro Thr Gly Ser
        35                  40                  45

Asp Trp Phe Asn Leu Ala Ile Lys Asp Ile Pro Glu Val Thr His Glu

    50                  55                  60

Ala Lys Lys Ala Leu Ala Gly Gln Leu Pro Ala Val Gly Arg Ser Met
65                  70                  75                  80

Cys Val Glu Ile Ser Leu Lys Thr Ser Glu Gly Asp Ser Met Glu Leu
                85                  90                  95

Glu Ile Trp Cys Leu Glu Met Asn Glu Lys Cys Asp Lys Glu Ile Lys
            100                 105                 110

Val Ser Tyr Thr Val Tyr Asn Arg Leu Ser Leu Leu Leu Lys Ser Leu
        115                 120                 125

Leu Ala Ile Thr Arg Val Thr Pro Ala Tyr Arg Leu Ser Arg Lys Gln
    130                 135                 140

Gly His Glu Tyr Val Ile Leu Tyr Arg Ile Tyr Phe Gly Glu Val Gln
145                 150                 155                 160

Leu Ser Gly Leu Gly Glu Gly Phe Gln Thr Val Arg Val Gly Thr Val
                165                 170                 175

Gly Thr Pro Val Gly Thr Ile Thr Leu Ser Cys Ala Tyr Arg Ile Asn
            180                 185                 190

Leu Ala Phe Met Ser Thr Arg Gln Phe Glu Arg Thr Pro Pro Ile Met
        195                 200                 205

Gly Ile Ile Ile Asp His Phe Val Asp Arg Pro Tyr Pro Ser Ser Ser
    210                 215                 220

Pro Met His Pro Cys Asn Tyr Arg Thr Ala Gly Glu Asp Thr Gly Val
225                 230                 235                 240

Ile Tyr Pro Ser Val Glu Asp Ser Gln Glu Val Cys Thr Thr Ser Phe
                245                 250                 255

Ser Thr Ser Pro Pro Ser Gln Leu Met Val Pro Gly Lys Glu Gly Gly
            260                 265                 270

Val Pro Leu Ala Pro Asn Gln Pro Val His Gly Thr Gln Ala Asp Gln
        275                 280                 285

Glu Arg Leu Ala Thr Cys Thr Pro Ser Asp Arg Thr His Cys Ala Ala
    290                 295                 300

Thr Pro Ser Ser Ser Glu Asp Thr Glu Thr Val Ser Asn Ser Ser Glu
305                 310                 315                 320

Gly Arg Ala Ser Pro His Asp Val Leu Glu Thr Ile Phe Val Arg Lys
                325                 330                 335

Val Gly Ala Phe Val Asn Lys Pro Ile Asn Gln Val Thr Leu Thr Ser
            340                 345                 350

Leu Asp Ile Pro Phe Ala Met Phe Ala Pro Lys Asn Leu Glu Leu Glu
        355                 360                 365

Asp Thr Asp Pro Met Val Asn Pro Pro Asp Ser Pro Glu Thr Glu Ser
    370                 375                 380

Pro Leu Gln Gly Ser Leu His Ser Asp Gly Ser Ser Gly Gly Ser Ser
385                 390                 395                 400

Gly Asn Thr His Asp Asp Phe Val Met Ile Asp Phe Lys Pro Ala Phe
                405                 410                 415

Ser Lys Asp Asp Ile Leu Pro Met Asp Leu Gly Thr Phe Tyr Arg Glu
            420                 425                 430

Phe Gln Asn Pro Pro Gln Leu Ser Ser Leu Ser Ile Asp Ile Gly Ala
        435                 440                 445

Gln Ser Met Ala Glu Asp Leu Asp Ser Leu Pro Glu Lys Leu Ala Val
    450                 455                 460

His Glu Lys Asn Val Arg Glu Phe Asp Ala Phe Val Glu Thr Leu Gln
465                 470                 475                 480

<210>  21
<211>  357
<212>  PRT
<213>  Homo sapiens

<400>  21

Met Ala Arg Glu Asn Gly Glu Ser Ser Ser Ser Trp Lys Lys Gln Ala
1               5                   10                  15

Glu Asp Ile Lys Lys Ile Phe Glu Phe Lys Glu Thr Leu Gly Thr Gly
            20                  25                  30

Ala Phe Ser Glu Val Val Leu Ala Glu Glu Lys Ala Thr Gly Lys Leu
        35                  40                  45

Phe Ala Val Lys Cys Ile Pro Lys Lys Ala Leu Lys Gly Lys Glu Ser
    50                  55                  60

Ser Ile Glu Asn Glu Ile Ala Val Leu Arg Lys Ile Lys His Glu Asn
65                  70                  75                  80

Ile Val Ala Leu Glu Asp Ile Tyr Glu Ser Pro Asn His Leu Tyr Leu
                85                  90                  95

Val Met Gln Leu Val Ser Gly Gly Glu Leu Phe Asp Arg Ile Val Glu
            100                 105                 110

Lys Gly Phe Tyr Thr Glu Lys Asp Ala Ser Thr Leu Ile Arg Gln Val
        115                 120                 125

Leu Asp Ala Val Tyr Tyr Leu His Arg Met Gly Ile Val His Arg Asp
    130                 135                 140

Leu Lys Pro Glu Asn Leu Leu Tyr Tyr Ser Gln Asp Glu Glu Ser Lys
145                 150                 155                 160

Ile Met Ile Ser Asp Phe Gly Leu Ser Lys Met Glu Gly Lys Gly Asp
                165                 170                 175

Val Met Ser Thr Ala Cys Gly Thr Pro Gly Tyr Val Ala Pro Glu Val

180                 185                 190

Leu Ala Gln Lys Pro Tyr Ser Lys Ala Val Asp Cys Trp Ser Ile Gly
        195                 200                 205

Val Ile Ala Tyr Ile Leu Leu Cys Gly Tyr Pro Pro Phe Tyr Asp Glu
    210                 215                 220

Asn Asp Ser Lys Leu Phe Glu Gln Ile Leu Lys Ala Glu Tyr Glu Phe
225                 230                 235                 240

Asp Ser Pro Tyr Trp Asp Asp Ile Ser Asp Ser Ala Lys Asp Phe Ile
                245                 250                 255

Arg Asn Leu Met Glu Lys Asp Pro Asn Lys Arg Tyr Thr Cys Glu Gln
            260                 265                 270

Ala Ala Arg His Pro Trp Ile Ala Gly Asp Thr Ala Leu Asn Lys Asn
        275                 280                 285

Ile His Glu Ser Val Ser Ala Gln Ile Arg Lys Asn Phe Ala Lys Ser
    290                 295                 300

Lys Trp Arg Gln Ala Phe Asn Ala Thr Ala Val Val Arg His Met Arg
305                 310                 315                 320

Lys Leu His Leu Gly Ser Ser Leu Asp Ser Ser Asn Ala Ser Val Ser
                325                 330                 335

Ser Ser Leu Ser Leu Ala Ser Gln Lys Asp Cys Ala Tyr Val Ala Lys
            340                 345                 350

Pro Glu Ser Leu Ser
        355

<210>  22
<211>  123
<212>  PRT
<213>  Homo sapiens

<400>  22

<210>  23
<211>  550
<212>  PRT
<213>  Homo sapiens

<400>  23

Met Asp Gly Ile Val Pro Asp Ile Ala Val Gly Thr Lys Arg Gly Ser
1               5                   10                  15

Asp Glu Leu Phe Ser Thr Cys Val Thr Asn Gly Pro Phe Ile Met Ser
            20                  25                  30

Ser Asn Ser Ala Ser Ala Ala Asn Gly Asn Asp Ser Lys Lys Phe Lys
        35                  40                  45

Gly Asp Ser Arg Ser Ala Gly Val Pro Ser Arg Val Ile His Ile Arg

50                  55                  60

Lys Leu Pro Ile Asp Val Thr Glu Gly Glu Val Ile Ser Leu Gly Leu
65                  70                  75                  80

Pro Phe Gly Lys Val Thr Asn Leu Leu Met Leu Lys Gly Lys Asn Gln
                85                  90                  95

Ala Phe Ile Glu Met Asn Thr Glu Glu Ala Ala Asn Thr Met Val Asn
            100                 105                 110

Tyr Tyr Thr Ser Val Thr Pro Val Leu Arg Gly Gln Pro Ile Tyr Ile
        115                 120                 125

Gln Phe Ser Asn His Lys Glu Leu Lys Thr Asp Ser Ser Pro Asn Gln
    130                 135                 140

Ala Arg Ala Gln Ala Ala Leu Gln Ala Val Asn Ser Val Gln Ser Gly
145                 150                 155                 160

Asn Leu Ala Leu Ala Ala Ser Ala Ala Ala Val Asp Ala Gly Met Ala
                165                 170                 175

Met Ala Gly Gln Ser Pro Val Leu Arg Ile Ile Val Glu Asn Leu Phe
            180                 185                 190

Tyr Pro Val Thr Leu Asp Val Leu His Gln Ile Phe Ser Lys Phe Gly
        195                 200                 205

Thr Val Leu Lys Ile Ile Thr Phe Thr Lys Asn Asn Gln Phe Gln Ala
    210                 215                 220

Leu Leu Gln Tyr Ala Asp Pro Val Ser Ala Gln His Ala Lys Leu Ser
225                 230                 235                 240

Leu Asp Gly Gln Asn Ile Tyr Asn Ala Cys Cys Thr Leu Arg Ile Asp
                245                 250                 255

Phe Ser Lys Leu Thr Ser Leu Asn Val Lys Tyr Asn Asn Asp Lys Ser
            260                 265                 270

Arg Asp Tyr Thr Arg Pro Asp Leu Pro Ser Gly Asp Ser Gln Pro Ser
        275                 280                 285

Leu Asp Gln Thr Met Ala Ala Ala Phe Ala Ser Pro Tyr Ala Gly Ala
    290                 295                 300

Gly Phe Pro Pro Thr Phe Ala Ile Pro Gln Ala Ala Gly Leu Ser Val
305                 310                 315                 320

Pro Asn Val His Gly Ala Leu Ala Pro Leu Ala Ile Pro Ser Ala Ala
                325                 330                 335

Ala Ala Ala Ala Ala Ala Gly Arg Ile Ala Ile Pro Gly Leu Ala Gly
            340                 345                 350

Ala Gly Asn Ser Val Leu Leu Val Ser Asn Leu Asn Pro Glu Arg Val
        355                 360                 365

Thr Pro Gln Ser Leu Phe Ile Leu Phe Gly Val Tyr Gly Asp Val Gln
    370                 375                 380

Arg Val Lys Ile Leu Phe Asn Lys Lys Glu Asn Ala Leu Val Gln Met
385                 390                 395                 400

Ala Asp Gly Asn Gln Ala Gln Leu Ala Met Ser His Leu Asn Gly His
                405                 410                 415

Lys Leu His Gly Lys Pro Ile Arg Ile Thr Leu Ser Lys His Gln Asn
            420                 425                 430

Val Gln Leu Pro Arg Glu Gly Gln Glu Asp Gln Gly Leu Thr Lys Asp
        435                 440                 445

Tyr Gly Asn Ser Pro Leu His Arg Phe Lys Lys Pro Gly Ser Lys Asn
    450                 455                 460

Phe Gln Asn Ile Phe Pro Pro Ser Ala Thr Leu His Leu Ser Asn Ile
465                 470                 475                 480

Pro Pro Ser Val Ser Glu Glu Asp Leu Lys Val Leu Phe Ser Ser Asn
                485                 490                 495

Gly Gly Val Val Lys Gly Phe Lys Phe Phe Gln Lys Asp Arg Lys Met
            500                 505                 510

Ala Leu Ile Gln Met Gly Ser Val Glu Glu Ala Val Gln Ala Leu Ile
        515                 520                 525

Asp Leu His Asn His Asp Leu Gly Glu Asn His His Leu Arg Val Ser
    530                 535                 540

Phe Ser Lys Ser Thr Ile
545                 550

<210> 24
<211> 621
<212> PRT
<213> Homo sapiens

<400> 24

Met Ala Asn Val Pro Trp Ala Glu Val Cys Glu Lys Phe Gln Ala Ala
1               5                   10                  15

Leu Ala Leu Ser Arg Val Glu Leu His Lys Asn Pro Glu Lys Glu Pro
            20                  25                  30

Tyr Lys Ser Lys Tyr Ser Ala Arg Ala Leu Leu Glu Glu Val Lys Ala
        35                  40                  45

Leu Leu Gly Pro Ala Pro Glu Asp Glu Asp Glu Arg Pro Glu Ala Glu
    50                  55                  60

Asp Gly Pro Gly Ala Gly Asp His Ala Leu Gly Leu Pro Ala Glu Val
65                  70                  75                  80

Val Glu Pro Glu Gly Pro Val Ala Gln Arg Ala Val Arg Leu Ala Val
                85                  90                  95

Ile Glu Phe His Leu Gly Val Asn His Ile Asp Thr Glu Glu Leu Ser

            100                 105                 110

Ala Gly Glu Glu His Leu Val Lys Cys Leu Arg Leu Leu Arg Arg Tyr
        115                 120                 125

Arg Leu Ser His Asp Cys Ile Ser Leu Cys Ile Gln Ala Gln Asn Asn
    130                 135                 140

Leu Gly Ile Leu Trp Ser Glu Arg Glu Glu Ile Glu Thr Ala Gln Ala
145                 150                 155                 160

Tyr Leu Glu Ser Ser Glu Ala Leu Tyr Asn Gln Tyr Met Lys Glu Val
                165                 170                 175

Gly Ser Pro Pro Leu Asp Pro Thr Glu Arg Phe Leu Pro Glu Glu Glu
            180                 185                 190

Lys Leu Thr Glu Gln Glu Arg Ser Lys Arg Phe Glu Lys Val Tyr Thr
        195                 200                 205

His Asn Leu Tyr Tyr Leu Ala Gln Val Tyr Gln His Leu Glu Met Phe
    210                 215                 220

Glu Lys Ala Ala His Tyr Cys His Ser Thr Leu Lys Arg Gln Leu Glu
225                 230                 235                 240

His Asn Ala Tyr His Pro Ile Glu Trp Ala Ile Asn Ala Ala Thr Leu
                245                 250                 255

Ser Gln Phe Tyr Ile Asn Lys Leu Cys Phe Met Glu Ala Arg His Cys
            260                 265                 270

Leu Ser Ala Ala Asn Val Ile Phe Gly Gln Thr Gly Lys Ile Ser Ala
        275                 280                 285

Thr Glu Asp Thr Pro Glu Ala Glu Gly Glu Val Pro Glu Leu Tyr His
    290                 295                 300

Gln Arg Lys Gly Glu Ile Ala Arg Cys Trp Ile Lys Tyr Cys Leu Thr
305                 310                 315                 320

Leu Met Gln Asn Ala Gln Leu Ser Met Gln Asp Asn Ile Gly Glu Leu
                325                 330                 335

Asp Leu Asp Lys Gln Ser Glu Leu Arg Ala Leu Arg Lys Lys Glu Leu
            340                 345                 350

Asp Glu Glu Glu Ser Ile Arg Lys Lys Ala Val Gln Phe Gly Thr Gly
        355                 360                 365

Glu Leu Cys Asp Ala Ile Ser Ala Val Glu Glu Lys Val Ser Tyr Leu
    370                 375                 380

Arg Pro Leu Asp Phe Glu Glu Ala Arg Glu Leu Phe Leu Leu Gly Gln
385                 390                 395                 400

His Tyr Val Phe Glu Ala Lys Glu Phe Phe Gln Ile Asp Gly Tyr Val
                405                 410                 415

Thr Asp His Ile Glu Val Val Gln Asp His Ser Ala Leu Phe Lys Val
            420                 425                 430

Leu Ala Phe Phe Glu Thr Asp Met Glu Arg Arg Cys Lys Met His Lys
        435                 440                 445

Arg Arg Ile Ala Met Leu Glu Pro Leu Thr Val Asp Leu Asn Pro Gln
    450                 455                 460

Tyr Tyr Leu Leu Val Asn Arg Gln Ile Gln Phe Glu Ile Ala His Ala
465                 470                 475                 480

Tyr Tyr Asp Met Met Asp Leu Lys Val Ala Ile Ala Asp Arg Leu Arg
                485                 490                 495

Asp Pro Asp Ser His Ile Val Lys Lys Ile Asn Asn Leu Asn Lys Ser
            500                 505                 510

Ala Leu Lys Tyr Tyr Gln Leu Phe Leu Asp Ser Leu Arg Asp Pro Asn
        515                 520                 525

Lys Val Phe Pro Glu His Ile Gly Glu Asp Val Leu Arg Pro Ala Met
    530                 535                 540

Leu Ala Lys Phe Arg Val Ala Arg Leu Tyr Gly Lys Ile Ile Thr Ala
545                 550                 555                 560

Asp Pro Lys Lys Glu Leu Glu Asn Leu Ala Thr Ser Leu Glu His Tyr
                565                 570                 575

Lys Phe Ile Val Asp Tyr Cys Glu Lys His Pro Glu Ala Ala Gln Glu
            580                 585                 590

Ile Glu Val Glu Leu Glu Leu Ser Lys Glu Met Val Ser Leu Leu Pro
        595                 600                 605

Thr Lys Met Glu Arg Phe Arg Thr Lys Met Ala Leu Thr
    610                 615                 620

<210>  25
<211>  249
<212>  PRT
<213>  Homo sapiens

<400>  25

Met Glu Met Gly Arg Arg Ile His Leu Glu Leu Arg Asn Arg Thr Pro
1               5                   10                  15

Ser Asp Val Lys Glu Leu Val Leu Asp Asn Ser Arg Ser Asn Glu Gly
            20                  25                  30

Lys Leu Glu Gly Leu Thr Asp Glu Phe Glu Glu Leu Glu Phe Leu Ser
        35                  40                  45

Thr Ile Asn Val Gly Leu Thr Ser Ile Ala Asn Leu Pro Lys Leu Asn
    50                  55                  60

Lys Leu Lys Lys Leu Glu Leu Ser Asp Asn Arg Val Ser Gly Gly Leu
65                  70                  75                  80

Glu Val Leu Ala Glu Lys Cys Pro Asn Leu Thr His Leu Asn Leu Ser

85                  90                  95

Gly Asn Lys Ile Lys Asp Leu Ser Thr Ile Glu Pro Leu Lys Lys Leu
            100                 105                 110

Glu Asn Leu Lys Ser Leu Asp Leu Phe Asn Cys Glu Val Thr Asn Leu
        115                 120                 125

Asn Asp Tyr Arg Glu Asn Val Phe Lys Leu Leu Pro Gln Leu Thr Tyr
    130                 135                 140

Leu Asp Gly Tyr Asp Arg Asp Asp Lys Glu Ala Pro Asp Ser Asp Ala
145                 150                 155                 160

Glu Gly Tyr Val Glu Gly Leu Asp Asp Glu Glu Glu Asp Glu Asp Glu
                165                 170                 175

Glu Glu Tyr Asp Glu Asp Ala Gln Val Val Glu Asp Glu Glu Asp Glu
            180                 185                 190

Asp Glu Glu Glu Glu Gly Glu Glu Glu Asp Val Ser Gly Glu Glu Glu
        195                 200                 205

Glu Asp Glu Glu Gly Tyr Asn Asp Gly Glu Val Asp Asp Glu Glu Asp
    210                 215                 220

Glu Glu Glu Leu Gly Glu Glu Glu Arg Gly Gln Lys Arg Lys Arg Glu
225                 230                 235                 240

Pro Glu Asp Glu Gly Glu Asp Asp Asp
                245

<210>  26
<211>  410
<212>  PRT
<213>  Homo sapiens

<400>  26

Met Gly Ala Asn Ala Ser Asn Tyr Pro His Ser Cys Ser Pro Arg Val
1               5                   10                  15

Gly Gly Asn Ser Gln Ala Gln Gln Thr Phe Ile Gly Thr Ser Ser Tyr
            20                  25                  30

Ser Gln Gln Gly Tyr Gly Cys Glu Ser Lys Leu Tyr Ser Leu Asp His
        35                  40                  45

Gly His Glu Lys Pro Gln Asp Lys Lys Lys Arg Thr Ser Gly Leu Ala
    50                  55                  60

Thr Leu Lys Lys Lys Phe Ile Lys Arg Arg Lys Ser Asn Arg Ser Ala
65                  70                  75                  80

Asp His Ala Lys Gln Met Arg Glu Leu Leu Ser Gly Trp Asp Val Arg
                85                  90                  95

Asp Val Asn Ala Leu Val Glu Glu Tyr Glu Gly Thr Ser Ala Leu Lys
            100                 105                 110

Glu Leu Ser Leu Gln Ala Ser Leu Ala Arg Pro Glu Ala Arg Thr Leu
        115                 120                 125

Gln Lys Asp Met Ala Asp Leu Tyr Glu Tyr Lys Tyr Cys Thr Asp Val
    130                 135                 140

Asp Leu Ile Phe Gln Glu Thr Cys Phe Pro Val His Arg Ala Ile Leu
145                 150                 155                 160

Ala Ala Arg Cys Pro Phe Phe Lys Thr Leu Leu Ser Ser Ser Pro Glu
                165                 170                 175

Tyr Gly Ala Glu Ile Ile Met Asp Ile Asn Thr Ala Gly Ile Asp Met
            180                 185                 190

Pro Met Phe Ser Ala Leu Leu His Tyr Leu Tyr Thr Gly Glu Phe Gly
        195                 200                 205

Met Glu Asp Ser Arg Phe Gln Asn Val Asp Ile Leu Val Gln Leu Ser
    210                 215                 220

Glu Glu Phe Gly Thr Pro Asn Ser Leu Asp Val Asp Met Arg Gly Leu
225                 230                 235                 240

Phe Asp Tyr Met Cys Tyr Tyr Asp Val Val Leu Ser Phe Ser Ser Asp
                245                 250                 255

Ser Glu Leu Val Glu Ala Phe Gly Gly Asn Gln Asn Cys Leu Asp Glu
            260                 265                 270

Glu Leu Lys Ala His Lys Ala Val Ile Ser Ala Arg Ser Pro Phe Phe
        275                 280                 285

Arg Asn Leu Leu Gln Arg Arg Ile Arg Thr Gly Glu Glu Ile Thr Asp
    290                 295                 300

Arg Thr Leu Arg Thr Pro Thr Arg Ile Ile Leu Asp Glu Ser Ile Ile
305                 310                 315                 320

Pro Lys Lys Tyr Ala Thr Val Ile Leu His Cys Met Tyr Thr Asp Val
                325                 330                 335

Val Asp Leu Ser Val Leu His Cys Ser Pro Ser Val Gly Ser Leu Ser
            340                 345                 350

Glu Val Gln Ala Leu Val Ala Gly Lys Pro Asn Met Thr Arg Ala Glu
        355                 360                 365

Glu Ala Met Glu Leu Tyr His Ile Ala Leu Phe Leu Glu Phe Asn Met
    370                 375                 380

Leu Ala Gln Glu Glu Thr Thr Val Ile Arg Pro Ala Cys Ala Ala Glu
385                 390                 395                 400

Leu Ser Asn Ser Cys Leu Leu Pro Gln Ser
                405                 410

<210>  27
<211>  520
<212>  PRT
<213>  Homo sapiens

<400>  27

Met Ala Ala Ala Ala Ala Ala Ala Gly Ala Ala Gly Ser Ala Ala Pro
1               5                   10                  15

Ala Ala Ala Ala Gly Ala Pro Gly Ser Gly Gly Ala Pro Ser Gly Ser
            20                  25                  30

Gln Gly Val Leu Ile Gly Asp Arg Leu Tyr Ser Gly Val Leu Ile Thr
        35                  40                  45

Leu Glu Asn Cys Leu Leu Pro Asp Asp Lys Leu Arg Phe Thr Pro Ser
    50                  55                  60

Met Ser Ser Gly Leu Asp Thr Asp Thr Glu Thr Asp Leu Arg Val Val
65                  70                  75                  80

Gly Cys Glu Leu Ile Gln Ala Ala Gly Ile Leu Leu Arg Leu Pro Gln
                85                  90                  95

Val Ala Met Ala Thr Gly Gln Val Leu Phe Gln Arg Phe Phe Tyr Thr
            100                 105                 110

Lys Ser Phe Val Lys His Ser Met Glu His Val Ser Met Ala Cys Val
        115                 120                 125

His Leu Ala Ser Lys Ile Glu Glu Ala Pro Arg Arg Ile Arg Asp Val
    130                 135                 140

Ile Asn Val Phe His Arg Leu Arg Gln Leu Arg Asp Lys Lys Lys Pro
145                 150                 155                 160

Val Pro Leu Leu Leu Asp Gln Asp Tyr Val Asn Leu Lys Asn Gln Ile
                165                 170                 175

Ile Lys Ala Glu Arg Arg Val Leu Lys Glu Leu Gly Phe Cys Val His
            180                 185                 190

Val Lys His Pro His Lys Ile Ile Val Met Tyr Leu Gln Val Leu Glu
        195                 200                 205

Cys Glu Arg Asn Gln His Leu Val Gln Thr Ser Trp Asn Tyr Met Asn
    210                 215                 220

Asp Ser Leu Arg Thr Asp Val Phe Val Arg Phe Gln Pro Glu Ser Ile
225                 230                 235                 240

Ala Cys Ala Cys Ile Tyr Leu Ala Ala Arg Thr Leu Glu Ile Pro Leu
                245                 250                 255

Pro Asn Arg Pro His Trp Phe Leu Leu Phe Gly Ala Thr Glu Glu Glu
            260                 265                 270

Ile Gln Glu Ile Cys Leu Lys Ile Leu Gln Leu Tyr Ala Arg Lys Lys
        275                 280                 285

Val Asp Leu Thr His Leu Glu Gly Glu Val Glu Lys Arg Lys His Ala
    290                 295                 300

Ile Glu Glu Ala Lys Ala Gln Ala Arg Gly Leu Leu Pro Gly Gly Thr
305                 310                 315                 320

Gln Val Leu Asp Gly Thr Ser Gly Phe Ser Pro Ala Pro Lys Leu Val
                325                 330                 335

Glu Ser Pro Lys Glu Gly Lys Gly Ser Lys Pro Ser Pro Leu Ser Val
            340                 345                 350

Lys Asn Thr Lys Arg Arg Leu Glu Gly Ala Lys Lys Ala Lys Ala Asp
        355                 360                 365

Ser Pro Val Asn Gly Leu Pro Lys Gly Arg Glu Ser Arg Ser Arg Ser
    370                 375                 380

Arg Ser Arg Glu Gln Ser Tyr Ser Arg Ser Pro Ser Arg Ser Ala Ser
385                 390                 395                 400

Pro Lys Arg Arg Lys Ser Asp Ser Gly Ser Thr Ser Gly Gly Ser Lys
                405                 410                 415

Ser Gln Ser Arg Ser Arg Ser Arg Ser Asp Ser Pro Pro Arg Gln Ala
            420                 425                 430

Pro Arg Ser Ala Pro Tyr Lys Gly Ser Glu Ile Arg Gly Ser Arg Lys
        435                 440                 445

Ser Lys Asp Cys Lys Tyr Pro Gln Lys Pro His Lys Ser Arg Ser Arg
    450                 455                 460

Ser Ser Ser Arg Ser Arg Ser Arg Ser Arg Glu Arg Ala Asp Asn Pro
465                 470                 475                 480

Gly Lys Tyr Lys Lys Lys Ser His Tyr Tyr Arg Asp Gln Arg Arg Glu
                485                 490                 495

Arg Ser Arg Ser Tyr Glu Arg Thr Gly Arg Arg Tyr Glu Arg Asp His
            500                 505                 510

Pro Gly His Ser Arg His Arg Arg
        515                 520

<210>  28
<211>  2426
<212>  PRT
<213>  Homo sapiens

<400>  28

Met Ala Thr Asn Ile Glu Gln Ile Phe Arg Ser Phe Val Val Ser Lys
1               5                   10                  15

Phe Arg Glu Ile Gln Gln Glu Leu Ser Ser Gly Arg Asn Glu Gly Gln
            20                  25                  30

Leu Asn Gly Glu Thr Asn Thr Pro Ile Glu Gly Asn Gln Ala Gly Asp
        35                  40                  45

Ala Ala Ala Ser Ala Arg Ser Leu Pro Asn Glu Glu Ile Val Gln Lys
    50                  55                  60

Ile Glu Glu Val Leu Ser Gly Val Leu Asp Thr Glu Leu Arg Tyr Lys
65                  70                  75                  80

Pro Asp Leu Lys Glu Gly Ser Arg Lys Ser Arg Cys Val Ser Val Gln
                85                  90                  95

Thr Asp Pro Thr Asp Glu Ile Pro Thr Lys Lys Ser Lys Lys His Lys
            100                 105                 110

Lys His Lys Asn Lys Lys Lys Lys Lys Lys Lys Glu Lys Glu Lys Lys
        115                 120                 125

Tyr Lys Arg Gln Pro Glu Glu Ser Glu Ser Lys Thr Lys Ser His Asp
    130                 135                 140

Asp Gly Asn Ile Asp Leu Glu Ser Asp Ser Phe Leu Lys Phe Asp Ser
145                 150                 155                 160

Glu Pro Ser Ala Val Ala Leu Glu Leu Pro Thr Arg Ala Phe Gly Pro
                165                 170                 175

Ser Glu Thr Asn Glu Ser Pro Ala Val Val Leu Glu Pro Pro Val Val
            180                 185                 190

Ser Met Glu Val Ser Glu Pro His Ile Leu Glu Thr Leu Lys Pro Ala
        195                 200                 205

Thr Lys Thr Ala Glu Leu Ser Val Val Ser Thr Ser Val Ile Ser Glu
    210                 215                 220

Gln Ser Glu Gln Ser Val Ala Val Met Pro Glu Pro Ser Met Thr Lys
225                 230                 235                 240

Ile Leu Asp Ser Phe Ala Ala Ala Pro Val Pro Thr Thr Thr Leu Val
                245                 250                 255

Leu Lys Ser Ser Glu Pro Val Val Thr Met Ser Val Glu Tyr Gln Met
            260                 265                 270

Lys Ser Val Leu Lys Ser Val Glu Ser Thr Ser Pro Glu Pro Ser Lys
        275                 280                 285

Ile Met Leu Val Glu Pro Pro Val Ala Lys Val Leu Glu Pro Ser Glu
    290                 295                 300

Thr Leu Val Val Ser Ser Glu Thr Pro Thr Glu Val Tyr Pro Glu Pro
305                 310                 315                 320

Ser Thr Ser Thr Thr Met Asp Phe Pro Glu Ser Ser Ala Ile Glu Ala
                325                 330                 335

Leu Arg Leu Pro Glu Gln Pro Val Asp Val Pro Ser Glu Ile Ala Asp
            340                 345                 350

Ser Ser Met Thr Arg Pro Gln Glu Leu Pro Glu Leu Pro Lys Thr Thr
        355                 360                 365

Ala Leu Glu Leu Gln Glu Ser Ser Val Ala Ser Ala Met Glu Leu Pro
    370                 375                 380

Gly Pro Pro Ala Thr Ser Met Pro Glu Leu Gln Gly Pro Pro Val Thr
385                 390                 395                 400

Pro Val Leu Glu Leu Pro Gly Pro Ser Ala Thr Pro Val Pro Glu Leu
                405                 410                 415

Pro Gly Pro Leu Ser Thr Pro Val Pro Glu Leu Pro Gly Pro Pro Ala
            420                 425                 430

Thr Ala Val Pro Glu Leu Pro Gly Pro Ser Val Thr Pro Val Pro Gln
        435                 440                 445

Leu Ser Gln Glu Leu Pro Gly Leu Pro Ala Pro Ser Met Gly Leu Glu
    450                 455                 460

Pro Pro Gln Glu Val Pro Glu Pro Pro Val Met Ala Gln Glu Leu Pro
465                 470                 475                 480

Gly Leu Pro Leu Val Thr Ala Ala Val Glu Leu Pro Glu Gln Pro Ala
                485                 490                 495

Val Thr Val Ala Met Glu Leu Thr Glu Gln Pro Val Thr Thr Thr Glu
            500                 505                 510

Leu Glu Gln Pro Val Gly Met Thr Thr Val Glu His Pro Gly His Pro
        515                 520                 525

Glu Val Thr Thr Ala Thr Gly Leu Leu Gly Gln Pro Glu Ala Thr Met
    530                 535                 540

Val Leu Glu Leu Pro Gly Gln Pro Val Ala Thr Thr Ala Leu Glu Leu
545                 550                 555                 560

Pro Gly Gln Pro Ser Val Thr Gly Val Pro Glu Leu Pro Gly Leu Pro
                565                 570                 575

Ser Ala Thr Arg Ala Leu Glu Leu Ser Gly Gln Pro Val Ala Thr Gly
            580                 585                 590

Ala Leu Glu Leu Pro Gly Pro Leu Met Ala Ala Gly Ala Leu Glu Phe
        595                 600                 605

Ser Gly Gln Ser Gly Ala Ala Gly Ala Leu Glu Leu Leu Gly Gln Pro
    610                 615                 620

Leu Ala Thr Gly Val Leu Glu Leu Pro Gly Gln Pro Gly Ala Pro Glu
625                 630                 635                 640

Leu Pro Gly Gln Pro Val Ala Thr Val Ala Leu Glu Ile Ser Val Gln
                645                 650                 655

Ser Val Val Thr Thr Ser Glu Leu Ser Thr Met Thr Val Ser Gln Ser
            660                 665                 670

Leu Glu Val Pro Ser Thr Thr Ala Leu Glu Ser Tyr Asn Thr Val Ala
        675                 680                 685

Gln Glu Leu Pro Thr Thr Leu Val Gly Glu Thr Ser Val Thr Val Gly
    690                 695                 700

Val Asp Pro Leu Met Ala Pro Glu Ser His Ile Leu Ala Ser Asn Thr

705                 710                 715                 720

Met Glu Thr His Ile Leu Ala Ser Asn Thr Met Asp Ser Gln Met Leu
                725                 730                 735

Ala Ser Asn Thr Met Asp Ser Gln Met Leu Ala Ser Asn Thr Met Asp
            740                 745                 750

Ser Gln Met Leu Ala Ser Ser Thr Met Asp Ser Gln Met Leu Ala Thr
        755                 760                 765

Ser Ser Met Asp Ser Gln Met Leu Ala Thr Ser Ser Met Asp Ser Gln
    770                 775                 780

Met Leu Ala Thr Ser Thr Met Asp Ser Gln Met Leu Ala Thr Ser Ser
785                 790                 795                 800

Met Asp Ser Gln Met Leu Ala Thr Ser Ser Met Asp Ser Gln Met Leu
                805                 810                 815

Ala Thr Ser Ser Met Asp Ser Gln Met Leu Ala Thr Ser Ser Met Asp
            820                 825                 830

Ser Gln Met Leu Ala Thr Ser Thr Met Asp Ser Gln Met Leu Ala Thr
        835                 840                 845

Ser Thr Met Asp Ser Gln Met Leu Ala Thr Ser Ser Met Asp Ser Gln
    850                 855                 860

Met Leu Ala Ser Gly Thr Met Asp Ser Gln Met Leu Ala Ser Gly Thr
865                 870                 875                 880

Met Asp Ala Gln Met Leu Ala Ser Gly Thr Met Asp Ala Gln Met Leu
                885                 890                 895

Ala Ser Ser Thr Gln Asp Ser Ala Met Leu Gly Ser Lys Ser Pro Asp
            900                 905                 910

Pro Tyr Arg Leu Ala Gln Asp Pro Tyr Arg Leu Ala Gln Asp Pro Tyr
        915                 920                 925

Arg Leu Gly His Asp Pro Tyr Arg Leu Gly His Asp Ala Tyr Arg Leu
    930                 935                 940

Gly Gln Asp Pro Tyr Arg Leu Gly His Asp Pro Tyr Arg Leu Thr Pro
945                 950                 955                 960

Asp Pro Tyr Arg Met Ser Pro Arg Pro Tyr Arg Ile Ala Pro Arg Ser
                965                 970                 975

Tyr Arg Ile Ala Pro Arg Pro Tyr Arg Leu Ala Pro Arg Pro Leu Met
            980                 985                 990

Leu Ala Ser Arg Arg Ser Met Met Met Ser Tyr Ala Ala Glu Arg Ser
        995                 1000                1005

Met Met Ser Ser Tyr Glu Arg Ser Met Met Ser Tyr Glu Arg Ser
    1010                1015                1020

Met Met Ser Pro Met Ala Glu Arg Ser Met Met Ser Ala Tyr Glu
    1025                1030                1035

Arg Ser Met Met Ser Ala Tyr Glu Arg Ser Met Met Ser Pro Met
    1040                1045                1050

Ala Glu Arg Ser Met Met Ser Ala Tyr Glu Arg Ser Met Met Ser
    1055                1060                1065

Ala Tyr Glu Arg Ser Met Met Ser Pro Met Ala Asp Arg Ser Met
    1070                1075                1080

Met Ser Met Gly Ala Asp Arg Ser Met Met Ser Ser Tyr Ser Ala
    1085                1090                1095

Ala Asp Arg Ser Met Met Ser Ser Tyr Ser Ala Ala Asp Arg Ser
    1100                1105                1110

Met Met Ser Ser Tyr Thr Ala Asp Arg Ser Met Met Ser Met Ala
    1115                1120                1125

Ala Asp Ser Tyr Thr Asp Ser Tyr Thr Asp Thr Tyr Thr Glu Ala
    1130                1135                1140

Tyr Met Val Pro Pro Leu Pro Pro Glu Glu Pro Pro Thr Met Pro
    1145                1150                1155

Pro Leu Pro Pro Glu Glu Pro Pro Met Thr Pro Pro Leu Pro Pro
    1160                1165                1170

Glu Glu Pro Pro Glu Gly Pro Ala Leu Pro Thr Glu Gln Ser Ala
    1175                1180                1185

Leu Thr Ala Glu Asn Thr Trp Pro Thr Glu Val Pro Ser Ser Pro
    1190                1195                1200

Ser Glu Glu Ser Val Ser Gln Pro Glu Pro Pro Val Ser Gln Ser
    1205                1210                1215

Glu Ile Ser Glu Pro Ser Ala Val Pro Thr Asp Tyr Ser Val Ser
    1220                1225                1230

Ala Ser Asp Pro Ser Val Leu Val Ser Glu Ala Ala Val Thr Val
    1235                1240                1245

Pro Glu Pro Pro Pro Glu Pro Glu Ser Ser Ile Thr Leu Thr Pro
    1250                1255                1260

Val Glu Ser Ala Val Val Ala Glu Glu His Glu Val Val Pro Glu
    1265                1270                1275

Arg Pro Val Thr Cys Met Val Ser Glu Thr Pro Ala Met Ser Ala
    1280                1285                1290

Glu Pro Thr Val Leu Ala Ser Glu Pro Pro Val Met Ser Glu Thr
    1295                1300                1305

Ala Glu Thr Phe Asp Ser Met Arg Ala Ser Gly His Val Ala Ser
    1310                1315                1320

Glu Val Ser Thr Ser Leu Leu Val Pro Ala Val Thr Thr Pro Val
    1325                1330                1335

Leu Ala Glu Ser Ile Leu Glu Pro Pro Ala Met Ala Ala Pro Glu
    1340                1345                1350

Ser Ser Ala Met Ala Val Leu Glu Ser Ser Ala Val Thr Val Leu
    1355                1360                1365

Glu Ser Ser Thr Val Thr Val Leu Glu Ser Ser Thr Val Thr Val
    1370                1375                1380

Leu Glu Pro Ser Val Val Thr Val Pro Glu Pro Pro Val Val Ala
    1385                1390                1395

Glu Pro Asp Tyr Val Thr Ile Pro Val Pro Val Val Ser Ala Leu
    1400                1405                1410

Glu Pro Ser Val Pro Val Leu Glu Pro Ala Val Ser Val Leu Gln
    1415                1420                1425

Pro Ser Met Ile Val Ser Glu Pro Ser Val Ser Val Gln Glu Ser
    1430                1435                1440

Thr Val Thr Val Ser Glu Pro Ala Val Thr Val Ser Glu Gln Thr
    1445                1450                1455

Gln Val Ile Pro Thr Glu Val Ala Ile Glu Ser Thr Pro Met Ile
    1460                1465                1470

Leu Glu Ser Ser Ile Met Ser Ser His Val Met Lys Gly Ile Asn
    1475                1480                1485

Leu Ser Ser Gly Asp Gln Asn Leu Ala Pro Glu Ile Gly Met Gln
    1490                1495                1500

Glu Ile Ala Leu His Ser Gly Glu Glu Pro His Ala Glu Glu His
    1505                1510                1515

Leu Lys Gly Asp Phe Tyr Glu Ser Glu His Gly Ile Asn Ile Asp

1520                1525                1530

Leu Asn  Ile Asn Asn His Leu  Ile Ala Lys Glu Met  Glu His Asn
    1535                1540                1545

Thr Val  Cys Ala Ala Gly Thr  Ser Pro Val Gly Glu  Ile Gly Glu
    1550                1555                1560

Glu Lys  Ile Leu Pro Thr Ser  Glu Thr Lys Gln Arg  Thr Val Leu
    1565                1570                1575

Asp Thr  Tyr Pro Gly Val Ser  Glu Ala Asp Ala Gly  Glu Thr Leu
    1580                1585                1590

Ser Ser  Thr Gly Pro Phe Ala  Leu Glu Pro Asp Ala  Thr Gly Thr
    1595                1600                1605

Ser Lys  Gly Ile Glu Phe Thr  Thr Ala Ser Thr Leu  Ser Leu Val
    1610                1615                1620

Asn Lys  Tyr Asp Val Asp Leu  Ser Leu Thr Thr Gln  Asp Thr Glu
    1625                1630                1635

His Asp  Met Val Ile Ser Thr  Ser Pro Ser Gly Gly  Ser Glu Ala
    1640                1645                1650

Asp Ile  Glu Gly Pro Leu Pro  Ala Lys Asp Ile His  Leu Asp Leu
    1655                1660                1665

Pro Ser  Asn Asn Asn Leu Val  Ser Lys Asp Thr Glu  Glu Pro Leu
    1670                1675                1680

Pro Val  Lys Glu Ser Asp Gln  Thr Leu Ala Ala Leu  Leu Ser Pro
    1685                1690                1695

Lys Glu  Ser Ser Gly Gly Glu  Lys Glu Val Pro Pro  Pro Pro Lys
    1700                1705                1710

Glu Thr  Leu Pro Asp Ser Gly  Phe Ser Ala Asn Ile  Glu Asp Ile
    1715                1720                1725

Asn Glu Ala Asp Leu Val Arg Pro Leu Leu Pro Lys Asp Met Glu
    1730                1735                1740

Arg Leu Thr Ser Leu Arg Ala Gly Ile Glu Gly Pro Leu Leu Ala
    1745                1750                1755

Ser Asp Val Gly Arg Asp Arg Ser Ala Ala Ser Pro Val Val Ser
    1760                1765                1770

Ser Met Pro Glu Arg Ala Ser Glu Ser Ser Ser Glu Glu Lys Asp
    1775                1780                1785

Asp Tyr Glu Ile Phe Val Lys Val Lys Asp Thr His Glu Lys Ser
    1790                1795                1800

Lys Lys Asn Lys Asn Arg Asp Lys Gly Glu Lys Glu Lys Lys Arg
    1805                1810                1815

Asp Ser Ser Leu Arg Ser Arg Ser Lys Arg Ser Lys Ser Ser Glu
    1820                1825                1830

His Lys Ser Arg Lys Arg Thr Ser Glu Ser Arg Ser Arg Ala Arg
    1835                1840                1845

Lys Arg Ser Ser Lys Ser Lys Ser His Arg Ser Gln Thr Arg Ser
    1850                1855                1860

Arg Ser Arg Ser Arg Arg Arg Arg Arg Ser Ser Arg Ser Arg Ser
    1865                1870                1875

Lys Ser Arg Gly Arg Arg Ser Val Ser Lys Glu Lys Arg Lys Arg
    1880                1885                1890

Ser Pro Lys His Arg Ser Lys Ser Arg Glu Arg Lys Arg Lys Arg
    1895                1900                1905

Ser Ser Ser Arg Asp Asn Arg Lys Thr Val Arg Ala Arg Ser Arg
    1910                1915                1920

Thr Pro Ser Arg Arg Ser Arg Ser His Thr Pro Ser Arg Arg Arg
    1925                1930                1935

Arg Ser Arg Ser Val Gly Arg Arg Arg Ser Phe Ser Ile Ser Pro
    1940                1945                1950

Ser Arg Arg Ser Arg Thr Pro Ser Arg Arg Ser Arg Thr Pro Ser
    1955                1960                1965

Arg Arg Ser Arg Thr Pro Ser Arg Arg Ser Arg Thr Pro Ser Arg
    1970                1975                1980

Arg Ser Arg Thr Pro Ser Arg Arg Ser Arg Thr Pro Ser Arg Arg
    1985                1990                1995

Arg Arg Ser Arg Ser Val Val Arg Arg Arg Ser Phe Ser Ile Ser
    2000                2005                2010

Pro Val Arg Leu Arg Arg Ser Arg Thr Pro Leu Arg Arg Arg Phe
    2015                2020                2025

Ser Arg Ser Pro Ile Arg Arg Lys Arg Ser Arg Ser Ser Glu Arg
    2030                2035                2040

Gly Arg Ser Pro Lys Arg Leu Thr Asp Leu Asp Lys Ala Gln Leu
    2045                2050                2055

Leu Glu Ile Ala Lys Ala Asn Ala Ala Ala Met Cys Ala Lys Ala
    2060                2065                2070

Gly Val Pro Leu Pro Pro Asn Leu Lys Pro Ala Pro Pro Pro Thr
    2075                2080                2085

Ile Glu Glu Lys Val Ala Lys Lys Ser Gly Gly Ala Thr Ile Glu
    2090                2095                2100

Glu Leu Thr Glu Lys Cys Lys Gln Ile Ala Gln Ser Lys Glu Asp
    2105                2110                2115

Asp Asp Val Ile Val Asn Lys Pro His Val Ser Asp Glu Glu Glu
    2120                2125                2130

Glu Glu Pro Pro Phe Tyr His His Pro Phe Lys Leu Ser Glu Pro
    2135                2140                2145

Lys Pro Ile Phe Phe Asn Leu Asn Ile Ala Ala Ala Lys Pro Thr
    2150                2155                2160

Pro Pro Lys Ser Gln Val Thr Leu Thr Lys Glu Phe Pro Val Ser
    2165                2170                2175

Ser Gly Ser Gln His Arg Lys Lys Glu Ala Asp Ser Val Tyr Gly
    2180                2185                2190

Glu Trp Val Pro Val Glu Lys Asn Gly Glu Glu Asn Lys Asp Asp
    2195                2200                2205

Asp Asn Val Phe Ser Ser Asn Leu Pro Ser Glu Pro Val Asp Ile
    2210                2215                2220

Ser Thr Ala Met Ser Glu Arg Ala Leu Ala Gln Lys Arg Leu Ser
    2225                2230                2235

Glu Asn Ala Phe Asp Leu Glu Ala Met Ser Met Leu Asn Arg Ala
    2240                2245                2250

Gln Glu Arg Ile Asp Ala Trp Ala Gln Leu Asn Ser Ile Pro Gly
    2255                2260                2265

Gln Phe Thr Gly Ser Thr Gly Val Gln Val Leu Thr Gln Glu Gln
    2270                2275                2280

Leu Ala Asn Thr Gly Ala Gln Ala Trp Ile Lys Lys Asp Gln Phe
    2285                2290                2295

Leu Arg Ala Ala Pro Val Thr Gly Gly Met Gly Ala Val Leu Met
    2300                2305                2310

Arg Lys Met Gly Trp Arg Glu Gly Glu Gly Leu Gly Lys Asn Lys

2315                2320                2325

Glu Gly  Asn Lys Glu Pro Ile  Leu Val Asp Phe Lys  Thr Asp Arg
    2330                2335                2340

Lys Gly  Leu Val Ala Val Gly  Glu Arg Ala Gln Lys  Arg Ser Gly
    2345                2350                2355

Asn Phe  Ser Ala Ala Met Lys  Asp Leu Ser Gly Lys  His Pro Val
    2360                2365                2370

Ser Ala  Leu Met Glu Ile Cys  Asn Lys Arg Arg Trp  Gln Pro Pro
    2375                2380                2385

Glu Phe  Leu Leu Val His Asp  Ser Gly Pro Asp His  Arg Lys His
    2390                2395                2400

Phe Leu  Phe Arg Val Leu Arg  Asn Gly Ala Leu Thr  Arg Pro Asn
    2405                2410                2415

Cys Met  Phe Phe Leu Asn Arg  Tyr
    2420                2425

<210>  29
<211>  312
<212>  PRT
<213>  Homo sapiens

<400>  29

Met Ala Asp Phe Gly Ile Ser Ala Gly Gln Phe Val Ala Val Val Trp
1               5                   10                  15

Asp Lys Ser Ser Pro Val Glu Ala Leu Lys Gly Leu Val Asp Lys Leu
            20                  25                  30

Gln Ala Leu Thr Gly Asn Glu Gly Arg Val Ser Val Glu Asn Ile Lys
        35                  40                  45

Gln Leu Leu Gln Ser Ala His Lys Glu Ser Ser Phe Asp Ile Ile Leu
    50                  55                  60

Ser Gly Leu Val Pro Gly Ser Thr Thr Leu His Ser Ala Glu Ile Leu
65                  70                  75                  80

Ala Glu Ile Ala Arg Ile Leu Arg Pro Gly Gly Cys Leu Phe Leu Lys
                85                  90                  95

Glu Pro Val Glu Thr Ala Val Asp Asn Asn Ser Lys Val Lys Thr Ala
            100                 105                 110

Ser Lys Leu Cys Ser Ala Leu Thr Leu Ser Gly Leu Val Glu Val Lys
        115                 120                 125

Glu Leu Gln Arg Glu Pro Leu Thr Pro Glu Glu Val Gln Ser Val Arg
    130                 135                 140

Glu His Leu Gly His Glu Ser Asp Asn Leu Leu Phe Val Gln Ile Thr
145                 150                 155                 160

Gly Lys Lys Pro Asn Phe Glu Val Gly Ser Ser Arg Gln Leu Lys Leu
                165                 170                 175

Ser Ile Thr Lys Lys Ser Ser Pro Ser Val Lys Pro Ala Val Asp Pro
            180                 185                 190

Ala Ala Ala Lys Leu Trp Thr Leu Ser Ala Asn Asp Met Glu Asp Asp
        195                 200                 205

Ser Met Asp Leu Ile Asp Ser Asp Glu Leu Leu Asp Pro Glu Asp Leu
    210                 215                 220

Lys Lys Pro Asp Pro Ala Ser Leu Arg Ala Ala Ser Cys Gly Glu Gly
225                 230                 235                 240

Lys Lys Arg Lys Ala Cys Lys Asn Cys Thr Cys Gly Leu Ala Glu Glu
                245                 250                 255

Leu Glu Lys Glu Lys Ser Arg Glu Gln Met Ser Ser Gln Pro Lys Ser
            260                 265                 270

Ala Cys Gly Asn Cys Tyr Leu Gly Asp Ala Phe Arg Cys Ala Ser Cys
        275                 280                 285

Pro Tyr Leu Gly Met Pro Ala Phe Lys Pro Gly Glu Lys Val Leu Leu
    290                 295                 300

Ser Asp Ser Asn Leu His Asp Ala
305                 310

<210>  30
<211>  316
<212>  PRT
<213>  Homo sapiens

<400>  30

Met Ala Ser Thr Ser Arg Leu Asp Ala Leu Pro Arg Val Thr Cys Pro
1               5                   10                  15

Asn His Pro Asp Ala Ile Leu Val Glu Asp Tyr Arg Ala Gly Asp Met
            20                  25                  30

Ile Cys Pro Glu Cys Gly Leu Val Val Gly Asp Arg Val Ile Asp Val
        35                  40                  45

Gly Ser Glu Trp Arg Thr Phe Ser Asn Asp Lys Ala Thr Lys Asp Pro
    50                  55                  60

Ser Arg Val Gly Asp Ser Gln Asn Pro Leu Leu Ser Asp Gly Asp Leu
65                  70                  75                  80

Ser Thr Met Ile Gly Lys Gly Thr Gly Ala Ala Ser Phe Asp Glu Phe
                85                  90                  95

Gly Asn Ser Lys Tyr Gln Asn Arg Arg Thr Met Ser Ser Ser Asp Arg
            100                 105                 110

Ala Met Met Asn Ala Phe Lys Glu Ile Thr Thr Met Ala Asp Arg Ile
        115                 120                 125

Asn Leu Pro Arg Asn Ile Val Asp Arg Thr Asn Asn Leu Phe Lys Gln
    130                 135                 140

Val Tyr Glu Gln Lys Ser Leu Lys Gly Arg Ala Asn Asp Ala Ile Ala
145                 150                 155                 160

Ser Ala Cys Leu Tyr Ile Ala Cys Arg Gln Glu Gly Val Pro Arg Thr
                165                 170                 175

Phe Lys Glu Ile Cys Ala Val Ser Arg Ile Ser Lys Lys Glu Ile Gly
            180                 185                 190

Arg Cys Phe Lys Leu Ile Leu Lys Ala Leu Glu Thr Ser Val Asp Leu
        195                 200                 205

Ile Thr Thr Gly Asp Phe Met Ser Arg Phe Cys Ser Asn Leu Cys Leu
    210                 215                 220

Pro Lys Gln Val Gln Met Ala Ala Thr His Ile Ala Arg Lys Ala Val
225                 230                 235                 240

Glu Leu Asp Leu Val Pro Gly Arg Ser Pro Ile Ser Val Ala Ala Ala
                245                 250                 255

Ala Ile Tyr Met Ala Ser Gln Ala Ser Ala Glu Lys Arg Thr Gln Lys
            260                 265                 270

Glu Ile Gly Asp Ile Ala Gly Val Ala Asp Val Thr Ile Arg Gln Ser
        275                 280                 285

Tyr Arg Leu Ile Tyr Pro Arg Ala Pro Asp Leu Phe Pro Thr Asp Phe
    290                 295                 300

Lys Phe Asp Thr Pro Val Asp Lys Leu Pro Gln Leu
305                 310                 315

<210>  31
<211>  1469
<212>  PRT
<213>  Homo sapiens

<400>  31

Met Ala Arg Leu Ala Ala Val Leu Trp Asn Leu Cys Val Thr Ala Val
1               5                   10                  15

Leu Val Thr Ser Ala Thr Gln Gly Leu Ser Arg Ala Gly Leu Pro Phe
            20                  25                  30

Gly Leu Met Arg Arg Glu Leu Ala Cys Glu Gly Tyr Pro Ile Glu Leu
        35                  40                  45

Arg Cys Pro Gly Ser Asp Val Ile Met Val Glu Asn Ala Asn Tyr Gly
    50                  55                  60

Arg Thr Asp Asp Lys Ile Cys Asp Ala Asp Pro Phe Gln Met Glu Asn
65                  70                  75                  80

Val Gln Cys Tyr Leu Pro Asp Ala Phe Lys Ile Met Ser Gln Arg Cys
                85                  90                  95

Asn Asn Arg Thr Gln Cys Val Val Val Ala Gly Ser Asp Ala Phe Pro
            100                 105                 110

Asp Pro Cys Pro Gly Thr Tyr Lys Tyr Leu Glu Val Gln Tyr Asp Cys
        115                 120                 125

Val Pro Tyr Ile Phe Val Cys Pro Gly Thr Leu Gln Lys Val Leu Glu
    130                 135                 140

Pro Thr Ser Thr His Glu Ser Glu His Gln Ser Gly Ala Trp Cys Lys
145                 150                 155                 160

Asp Pro Leu Gln Ala Gly Asp Arg Ile Tyr Val Met Pro Trp Ile Pro
                165                 170                 175

Tyr Arg Thr Asp Thr Leu Thr Glu Tyr Ala Ser Trp Glu Asp Tyr Val
            180                 185                 190

Ala Ala Arg His Thr Thr Thr Tyr Arg Leu Pro Asn Arg Val Asp Gly
        195                 200                 205

Thr Gly Phe Val Val Tyr Asp Gly Ala Val Phe Tyr Asn Lys Glu Arg

210                 215                 220

Thr Arg Asn Ile Val Lys Tyr Asp Leu Arg Thr Arg Ile Lys Ser Gly
225                 230                 235                 240

Glu Thr Val Ile Asn Thr Ala Asn Tyr His Asp Thr Ser Pro Tyr Arg
                245                 250                 255

Trp Gly Gly Lys Thr Asp Ile Asp Leu Ala Val Asp Glu Asn Gly Leu
            260                 265                 270

Trp Val Ile Tyr Ala Thr Glu Gly Asn Asn Gly Arg Leu Val Val Ser
        275                 280                 285

Gln Leu Asn Pro Tyr Thr Leu Arg Phe Glu Gly Thr Trp Glu Thr Gly
    290                 295                 300

Tyr Asp Lys Arg Ser Ala Ser Asn Ala Phe Met Val Cys Gly Val Leu
305                 310                 315                 320

Tyr Val Leu Arg Ser Val Tyr Val Asp Asp Asp Ser Glu Ala Ala Gly
                325                 330                 335

Asn Arg Val Asp Tyr Ala Phe Asn Thr Asn Ala Asn Arg Glu Glu Pro
            340                 345                 350

Val Ser Leu Thr Phe Pro Asn Pro Tyr Gln Phe Ile Ser Ser Val Asp
        355                 360                 365

Tyr Asn Pro Arg Asp Asn Gln Leu Tyr Val Trp Asn Asn Tyr Phe Val
    370                 375                 380

Val Arg Tyr Ser Leu Glu Phe Gly Pro Pro Asp Pro Ser Ala Gly Pro
385                 390                 395                 400

Ala Thr Ser Pro Pro Leu Ser Thr Thr Thr Thr Ala Arg Pro Thr Pro
                405                 410                 415

Leu Thr Ser Thr Ala Ser Pro Ala Ala Thr Thr Pro Leu Arg Arg Ala
            420                 425                 430

Pro Leu Thr Thr His Pro Val Gly Ala Ile Asn Gln Leu Gly Pro Asp
        435                 440                 445

Leu Pro Pro Ala Thr Ala Pro Val Pro Ser Thr Arg Arg Pro Pro Ala
    450                 455                 460

Pro Asn Leu His Val Ser Pro Glu Leu Phe Cys Glu Pro Arg Glu Val
465                 470                 475                 480

Arg Arg Val Gln Trp Pro Ala Thr Gln Gln Gly Met Leu Val Glu Arg
                485                 490                 495

Pro Cys Pro Lys Gly Thr Arg Gly Ile Ala Ser Phe Gln Cys Leu Pro
            500                 505                 510

Ala Leu Gly Leu Trp Asn Pro Arg Gly Pro Asp Leu Ser Asn Cys Thr
        515                 520                 525

Ser Pro Trp Val Asn Gln Val Ala Gln Lys Ile Lys Ser Gly Glu Asn
    530                 535                 540

Ala Ala Asn Ile Ala Ser Glu Leu Ala Arg His Thr Arg Gly Ser Ile
545                 550                 555                 560

Tyr Ala Gly Asp Val Ser Ser Ser Val Lys Leu Met Glu Gln Leu Leu
                565                 570                 575

Asp Ile Leu Asp Ala Gln Leu Gln Ala Leu Arg Pro Ile Glu Arg Glu
            580                 585                 590

Ser Ala Gly Lys Asn Tyr Asn Lys Met His Lys Arg Glu Arg Thr Cys
        595                 600                 605

Lys Asp Tyr Ile Lys Ala Val Val Glu Thr Val Asp Asn Leu Leu Arg
    610                 615                 620

Pro Glu Ala Leu Glu Ser Trp Lys Asp Met Asn Ala Thr Glu Gln Val
625                 630                 635                 640

His Thr Ala Thr Met Leu Leu Asp Val Leu Glu Glu Gly Ala Phe Leu
                645                 650                 655

Leu Ala Asp Asn Val Arg Glu Pro Ala Arg Phe Leu Ala Ala Lys Glu
            660                 665                 670

Asn Val Val Leu Glu Val Thr Val Leu Asn Thr Glu Gly Gln Val Gln
        675                 680                 685

Glu Leu Val Phe Pro Gln Glu Glu Tyr Pro Arg Lys Asn Ser Ile Gln
    690                 695                 700

Leu Ser Ala Lys Thr Ile Lys Gln Asn Ser Arg Asn Gly Val Val Lys
705                 710                 715                 720

Val Val Phe Ile Leu Tyr Asn Asn Leu Gly Leu Phe Leu Ser Thr Glu
                725                 730                 735

Asn Ala Thr Val Lys Leu Ala Gly Glu Ala Gly Pro Gly Gly Pro Gly
            740                 745                 750

Gly Ala Ser Leu Val Val Asn Ser Gln Val Ile Ala Ala Ser Ile Asn
        755                 760                 765

Lys Glu Ser Ser Arg Val Phe Leu Met Asp Pro Val Ile Phe Thr Val
    770                 775                 780

Ala His Leu Glu Asp Lys Asn His Phe Asn Ala Asn Cys Ser Phe Trp
785                 790                 795                 800

Asn Tyr Ser Glu Arg Ser Met Leu Gly Tyr Trp Ser Thr Gln Gly Cys
                805                 810                 815

Arg Leu Val Glu Ser Asn Lys Thr His Thr Thr Cys Ala Cys Ser His
            820                 825                 830

Leu Thr Asn Phe Ala Val Leu Met Ala His Arg Glu Ile Tyr Gln Gly
        835                 840                 845

Arg Ile Asn Glu Leu Leu Leu Ser Val Ile Thr Trp Val Gly Ile Val
    850                 855                 860

Ile Ser Leu Val Cys Leu Ala Ile Cys Ile Ser Thr Phe Cys Phe Leu
865                 870                 875                 880

Arg Gly Leu Gln Thr Asp Arg Asn Thr Ile His Lys Asn Leu Cys Ile
                885                 890                 895

Asn Leu Phe Leu Ala Glu Leu Leu Phe Leu Val Gly Ile Asp Lys Thr
            900                 905                 910

Gln Tyr Glu Ile Ala Cys Pro Ile Phe Ala Gly Leu Leu His Tyr Phe
        915                 920                 925

Phe Leu Ala Ala Phe Ser Trp Leu Cys Leu Glu Gly Val His Leu Tyr
    930                 935                 940

Leu Leu Leu Val Glu Val Phe Glu Ser Glu Tyr Ser Arg Thr Lys Tyr
945                 950                 955                 960

Tyr Tyr Leu Gly Gly Tyr Cys Phe Pro Ala Leu Val Val Gly Ile Ala
                965                 970                 975

Ala Ala Ile Asp Tyr Arg Ser Tyr Gly Thr Glu Lys Ala Cys Trp Leu
            980                 985                 990

Arg Val Asp Asn Tyr Phe Ile Trp  Ser Phe Ile Gly Pro  Val Ser Phe
        995                 1000                 1005

Val Ile  Val Val Asn Leu Val  Phe Leu Met Val Thr  Leu His Lys
    1010                 1015                 1020

Met Ile  Arg Ser Ser Ser Val  Leu Lys Pro Asp Ser  Ser Arg Leu
    1025                 1030                 1035

Asp Asn  Ile Lys Ser Trp Ala  Leu Gly Ala Ile Ala  Leu Leu Phe
    1040                 1045                 1050

Leu Leu  Gly Leu Thr Trp Ala  Phe Gly Leu Leu Phe  Ile Asn Lys

1055                 1060                 1065

Glu Ser  Val Val Met Ala Tyr  Leu Phe Thr Thr Phe  Asn Ala Phe
    1070                 1075                 1080

Gln Gly  Val Phe Ile Phe Val  Phe His Cys Ala Leu  Gln Lys Lys
    1085                 1090                 1095

Val His  Lys Glu Tyr Ser Lys  Cys Leu Arg His Ser  Tyr Cys Cys
    1100                 1105                 1110

Ile Arg  Ser Pro Pro Gly Gly  Thr His Gly Ser Leu  Lys Thr Ser
    1115                 1120                 1125

Ala Met  Arg Ser Asn Thr Arg  Tyr Tyr Thr Gly Thr  Gln Ser Arg
    1130                 1135                 1140

Ile Arg  Arg Met Trp Asn Asp  Thr Val Arg Lys Gln  Thr Glu Ser
    1145                 1150                 1155

Ser Phe  Met Ala Gly Asp Ile  Asn Ser Thr Pro Thr  Leu Asn Arg
    1160                 1165                 1170

Gly Thr  Met Gly Asn His Leu  Leu Thr Asn Pro Val  Leu Gln Pro
    1175                 1180                 1185

Arg Gly  Gly Thr Ser Pro Tyr  Asn Thr Leu Ile Ala  Glu Ser Val
    1190                 1195                 1200

Gly Phe  Asn Pro Ser Ser Pro  Pro Val Phe Asn Ser  Pro Gly Ser
    1205                 1210                 1215

Tyr Arg  Glu Pro Lys His Pro  Leu Gly Gly Arg Glu  Ala Cys Gly
    1220                 1225                 1230

Met Asp  Thr Leu Pro Leu Asn  Gly Asn Phe Asn Asn  Ser Tyr Ser
    1235                 1240                 1245

Leu Arg  Ser Gly Asp Phe Pro  Pro Gly Asp Gly Gly  Pro Glu Pro
    1250                 1255                 1260

Pro Arg Gly Arg Asn Leu Ala Asp Ala Ala Ala Phe Glu Lys Met
    1265                1270                1275

Ile Ile Ser Glu Leu Val His Asn Asn Leu Arg Gly Ser Ser Ser
    1280                1285                1290

Ala Ala Lys Gly Pro Pro Pro Pro Glu Pro Pro Val Pro Pro Val
    1295                1300                1305

Pro Gly Gly Gly Gly Glu Glu Glu Ala Gly Gly Pro Gly Gly Ala
    1310                1315                1320

Asp Arg Ala Glu Ile Glu Leu Leu Tyr Lys Ala Leu Glu Glu Pro
    1325                1330                1335

Leu Leu Leu Pro Arg Ala Gln Ser Val Leu Tyr Gln Ser Asp Leu
    1340                1345                1350

Asp Glu Ser Glu Ser Cys Thr Ala Glu Asp Gly Ala Thr Ser Arg
    1355                1360                1365

Pro Leu Ser Ser Pro Pro Gly Arg Asp Ser Leu Tyr Ala Ser Gly
    1370                1375                1380

Ala Asn Leu Arg Asp Ser Pro Ser Tyr Pro Asp Ser Ser Pro Glu
    1385                1390                1395

Gly Pro Ser Glu Ala Leu Pro Pro Pro Pro Pro Ala Pro Pro Gly
    1400                1405                1410

Pro Pro Glu Ile Tyr Tyr Thr Ser Arg Pro Pro Ala Leu Val Ala
    1415                1420                1425

Arg Asn Pro Leu Gln Gly Tyr Tyr Gln Val Arg Arg Pro Ser His
    1430                1435                1440

Glu Gly Tyr Leu Ala Ala Pro Gly Leu Glu Gly Pro Gly Pro Asp
    1445                1450                1455

Gly Asp  Gly Gln Met Gln Leu  Val Thr Ser Leu
    1460                1465

<210>  32
<211>  573
<212>  PRT
<213>  Homo sapiens

<400>  32

<210>  33
<211>  121
<212>  PRT

<213>  Homo sapiens

<400>  33

Met Val Asp Met Met Asp Leu Pro Arg Ser Arg Ile Asn Ala Gly Met
1               5                   10                  15

Leu Ala Gln Phe Ile Asp Lys Pro Val Cys Phe Val Gly Arg Leu Glu
            20                  25                  30

Lys Ile His Pro Thr Gly Lys Met Phe Ile Leu Ser Asp Gly Glu Gly
        35                  40                  45

Lys Asn Gly Thr Ile Glu Leu Met Glu Pro Leu Asp Glu Glu Ile Ser
    50                  55                  60

Gly Ile Val Glu Val Val Gly Arg Val Thr Ala Lys Ala Thr Ile Leu
65                  70                  75                  80

Cys Thr Ser Tyr Val Gln Phe Lys Glu Asp Ser His Pro Phe Asp Leu
                85                  90                  95

Gly Leu Tyr Asn Glu Ala Val Lys Ile Ile His Asp Phe Pro Gln Phe
            100                 105                 110

Tyr Pro Leu Gly Ile Val Gln His Asp
        115                 120


<210>  34
<211>  560
<212>  PRT
<213>  Homo sapiens

<400>  34

Met Tyr Asp Asn Met Ser Thr Met Val Tyr Ile Lys Glu Asp Lys Leu
1               5                   10                  15

Glu Lys Leu Thr Gln Asp Glu Ile Ile Ser Lys Thr Lys Gln Val Ile
            20                  25                  30

Gln Gly Leu Glu Ala Leu Lys Asn Glu His Asn Ser Ile Leu Gln Ser
        35                  40                  45

Leu Leu Glu Thr Leu Lys Cys Leu Lys Lys Asp Asp Glu Ser Asn Leu
    50                  55                  60

Val Glu Glu Lys Ser Asn Met Ile Arg Lys Ser Leu Glu Met Leu Glu
65                  70                  75                  80

Leu Gly Leu Ser Glu Ala Gln Val Met Met Ala Leu Ser Asn His Leu
                85                  90                  95

Asn Ala Val Glu Ser Glu Lys Gln Lys Leu Arg Ala Gln Val Arg Arg
            100                 105                 110

Leu Cys Gln Glu Asn Gln Trp Leu Arg Asp Glu Leu Ala Asn Thr Gln
        115                 120                 125

Gln Lys Leu Gln Lys Ser Glu Gln Ser Val Ala Gln Leu Glu Glu Glu
    130                 135                 140

Lys Lys His Leu Glu Phe Met Asn Gln Leu Lys Lys Tyr Asp Asp Asp
145                 150                 155                 160

Ile Ser Pro Ser Glu Asp Lys Asp Thr Asp Ser Thr Lys Glu Pro Leu
                165                 170                 175

Asp Asp Leu Phe Pro Asn Asp Glu Asp Asp Pro Gly Gln Gly Ile Gln
            180                 185                 190

Gln Gln His Ser Ser Ala Ala Ala Ala Ala Gln Gln Gly Gly Tyr Glu
        195                 200                 205

Ile Pro Ala Arg Leu Arg Thr Leu His Asn Leu Val Ile Gln Tyr Ala
    210                 215                 220

Ser Gln Gly Arg Tyr Glu Val Ala Val Pro Leu Cys Lys Gln Ala Leu
225                 230                 235                 240

Glu Asp Leu Glu Lys Thr Ser Gly His Asp His Pro Asp Val Ala Thr
                245                 250                 255

Met Leu Asn Ile Leu Ala Leu Val Tyr Arg Asp Gln Asn Lys Tyr Lys
            260                 265                 270

Asp Ala Ala Asn Leu Leu Asn Asp Ala Leu Ala Ile Arg Glu Lys Thr
        275                 280                 285

Leu Gly Lys Asp His Pro Ala Val Ala Ala Thr Leu Asn Asn Leu Ala
    290                 295                 300

Val Leu Tyr Gly Lys Arg Gly Lys Tyr Lys Glu Ala Glu Pro Leu Cys
305                 310                 315                 320

Lys Arg Ala Leu Glu Ile Arg Glu Lys Val Leu Gly Lys Asp His Pro
                325                 330                 335

Asp Val Ala Lys Gln Leu Asn Asn Leu Ala Leu Leu Cys Gln Asn Gln
            340                 345                 350

Gly Lys Tyr Glu Glu Val Glu Tyr Tyr Tyr Gln Arg Ala Leu Glu Ile
        355                 360                 365

Tyr Gln Thr Lys Leu Gly Pro Asp Asp Pro Asn Val Ala Lys Thr Lys
    370                 375                 380

Asn Asn Leu Ala Ser Cys Tyr Leu Lys Gln Gly Lys Phe Lys Gln Ala
385                 390                 395                 400

Glu Thr Leu Tyr Lys Glu Ile Leu Thr Arg Ala His Glu Arg Glu Phe
                405                 410                 415

Gly Ser Val Asp Asp Glu Asn Lys Pro Ile Trp Met His Ala Glu Glu
            420                 425                 430

Arg Glu Glu Cys Lys Gly Lys Gln Lys Asp Gly Thr Ser Phe Gly Glu
        435                 440                 445

Tyr Gly Gly Trp Tyr Lys Ala Cys Lys Val Asp Ser Pro Thr Val Thr
    450                 455                 460

Thr Thr Leu Lys Asn Leu Gly Ala Leu Tyr Arg Arg Gln Gly Lys Phe
465                 470                 475                 480

Glu Ala Ala Glu Thr Leu Glu Glu Ala Ala Met Arg Ser Arg Lys Gln
                485                 490                 495

Gly Leu Asp Asn Val His Lys Gln Arg Val Ala Glu Val Leu Asn Asp
            500                 505                 510

Pro Glu Asn Met Glu Lys Arg Arg Ser Arg Glu Ser Leu Asn Val Asp
        515                 520                 525

Val Val Lys Tyr Glu Ser Gly Pro Asp Gly Gly Glu Glu Val Ser Met
    530                 535                 540

Ser Val Glu Trp Asn Gly Met Arg Lys Met Lys Leu Gly Leu Val Asn
545                 550                 555                 560

<210>  35
<211>  987
<212>  PRT
<213>  Homo sapiens

<400>  35

<210>  36
<211>  631
<212>  PRT
<213>  Homo sapiens

<400>  36

Met Ala Ala His Leu Val Lys Arg Cys Thr Cys Leu Leu Arg Glu Ala
1               5                   10                  15

Ala Arg Gln Ala Pro Ala Met Ala Pro Val Gly Arg Leu Arg Leu Ala
            20                  25                  30

Trp Val Ala His Lys Thr Leu Thr Ser Ser Ala Thr Ser Pro Ile Ser
        35                  40                  45

His Leu Pro Gly Ser Leu Met Glu Pro Val Glu Lys Glu Arg Ala Ser
    50                  55                  60

Thr Pro Tyr Ile Glu Lys Gln Val Asp His Leu Ile Lys Lys Ala Thr
65                  70                  75                  80

Arg Pro Glu Glu Leu Leu Glu Leu Leu Gly Gly Ser His Asp Leu Asp
                85                  90                  95

Ser Asn Gln Ala Ala Met Val Leu Ile Arg Leu Ser His Leu Leu Ser
            100                 105                 110

Glu Lys Pro Glu Asp Lys Gly Leu Leu Ile Gln Asp Ala His Phe His
        115                 120                 125

Gln Leu Leu Cys Leu Leu Asn Ser Gln Ile Ala Ser Val Trp His Gly
    130                 135                 140

Thr Leu Ser Lys Leu Leu Gly Ser Leu Tyr Ala Leu Gly Ile Pro Lys
145                 150                 155                 160

Ala Ser Lys Glu Leu Gln Ser Val Glu Gln Glu Val Arg Trp Arg Met
                165                 170                 175

Arg Lys Leu Lys Tyr Lys His Leu Ala Phe Leu Ala Glu Ser Cys Ala
            180                 185                 190

Thr Leu Ser Gln Glu Gln His Ser Gln Glu Leu Leu Ala Glu Leu Leu
        195                 200                 205

Thr His Leu Glu Arg Arg Trp Thr Glu Ile Glu Asp Ser His Thr Leu
    210                 215                 220

Val Thr Val Met Met Lys Val Gly His Leu Ser Glu Pro Leu Met Asn
225                 230                 235                 240

Arg Leu Glu Asp Lys Cys Leu Glu Leu Val Glu His Phe Gly Pro Asn
                245                 250                 255

Glu Leu Arg Lys Val Leu Val Met Leu Ala Ala Gln Ser Arg Arg Ser
            260                 265                 270

Val Pro Leu Leu Arg Ala Ile Ser Tyr His Leu Val Gln Lys Pro Phe
        275                 280                 285

Ser Leu Thr Lys Asp Val Leu Leu Asp Val Ala Tyr Ala Tyr Gly Lys
    290                 295                 300

Leu Ser Phe His Gln Thr Gln Val Ser Gln Arg Leu Ala Thr Asp Leu
305                 310                 315                 320

Leu Ser Leu Met Pro Ser Leu Thr Ser Gly Glu Val Ala His Cys Ala
                325                 330                 335

Lys Ser Phe Ala Leu Leu Lys Trp Leu Ser Leu Pro Leu Phe Glu Ala

            340                 345                 350

Phe Ala Gln His Val Leu Asn Arg Ala Gln Asp Ile Thr Leu Pro His
        355                 360                 365

Leu Cys Ser Val Leu Leu Ala Phe Ala Arg Leu Asn Phe His Pro Asp
    370                 375                 380

Gln Glu Asp Gln Phe Phe Ser Leu Val His Glu Lys Leu Gly Ser Glu
385                 390                 395                 400

Leu Pro Gly Leu Glu Pro Ala Leu Gln Val Asp Leu Val Trp Ala Leu
                405                 410                 415

Cys Val Leu Gln Gln Ala Arg Glu Ala Glu Leu Gln Ala Val Leu His
            420                 425                 430

Pro Glu Phe His Ile Gln Phe Leu Gly Gly Lys Ser Gln Lys Asp Gln
        435                 440                 445

Asn Thr Phe Gln Lys Leu Leu His Ile Asn Ala Thr Ala Leu Leu Glu
    450                 455                 460

Tyr Pro Glu Tyr Ser Gly Pro Leu Leu Pro Ala Ser Ala Val Ala Pro
465                 470                 475                 480

Gly Pro Ser Ala Leu Asp Arg Lys Val Thr Pro Leu Gln Lys Glu Leu
                485                 490                 495

Gln Glu Thr Leu Lys Gly Leu Leu Gly Ser Ala Asp Lys Gly Ser Leu
            500                 505                 510

Glu Val Ala Thr Gln Tyr Gly Trp Val Leu Asp Ala Glu Val Leu Leu
        515                 520                 525

Asp Ser Asp Gly Glu Phe Leu Pro Val Arg Asp Phe Val Ala Pro His
    530                 535                 540

Leu Ala Gln Pro Thr Gly Ser Gln Ser Pro Pro Pro Gly Ser Lys Arg
545                 550                 555                 560

Leu Ala Phe Leu Arg Trp Glu Phe Pro Asn Phe Asn Ser Arg Ser Lys
                565                 570                 575

Asp Leu Leu Gly Arg Phe Val Leu Ala Arg Arg His Ile Val Ala Ala
            580                 585                 590

Gly Phe Leu Ile Val Asp Val Pro Phe Tyr Glu Trp Leu Glu Leu Lys
        595                 600                 605

Ser Glu Trp Gln Lys Gly Ala Tyr Leu Lys Asp Lys Met Arg Lys Ala
    610                 615                 620

Val Ala Glu Glu Leu Ala Lys
625                 630

<210>  37
<211>  100
<212>  PRT
<213>  Homo sapiens

<400>  37

Met Pro Lys Arg Lys Val Ser Ser Ala Glu Gly Ala Ala Lys Glu Glu
1               5                   10                  15

Pro Lys Arg Arg Ser Ala Arg Leu Ser Ala Lys Pro Pro Ala Lys Val
            20                  25                  30

Glu Ala Lys Pro Lys Lys Ala Ala Ala Lys Asp Lys Ser Ser Asp Lys
        35                  40                  45

Lys Val Gln Thr Lys Gly Lys Arg Gly Ala Lys Gly Lys Gln Ala Glu
    50                  55                  60

Val Ala Asn Gln Glu Thr Lys Glu Asp Leu Pro Ala Glu Asn Gly Glu
65                  70                  75                  80

Thr Lys Thr Glu Glu Ser Pro Ala Ser Asp Glu Ala Gly Glu Lys Glu
                85                  90                  95

Ala Lys Ser Asp
            100

<210>  38
<211>  303
<212>  PRT
<213>  Homo sapiens

<400>  38

<210>  39
<211>  493
<212>  PRT
<213>  Homo sapiens

<400>  39

Met Phe Pro Val Phe Pro Cys Thr Leu Leu Ala Pro Pro Phe Pro Val
1               5                   10                  15

Leu Gly Leu Asp Ser Arg Gly Val Gly Gly Leu Met Asn Ser Phe Pro
            20                  25                  30

Pro Pro Gln Gly His Ala Gln Asn Pro Leu Gln Val Gly Ala Glu Leu
        35                  40                  45

Gln Ser Arg Phe Phe Ala Ser Gln Gly Cys Ala Gln Ser Pro Phe Gln
    50                  55                  60

Ala Ala Pro Ala Pro Pro Pro Thr Pro Gln Ala Pro Ala Ala Glu Pro
65                  70                  75                  80

Leu Gln Val Asp Leu Leu Pro Val Leu Ala Ala Ala Gln Glu Ser Ala
                85                  90                  95

Ala Ala Ala Ala Ala Ala Ala Ala Ala Ala Ala Ala Val Ala Ala Ala
            100                 105                 110

Pro Pro Ala Pro Ala Ala Ala Ser Thr Val Asp Thr Ala Ala Leu Lys
        115                 120                 125

Gln Pro Pro Ala Pro Pro Pro Pro Pro Pro Pro Val Ser Ala Pro Ala
    130                 135                 140

Ala Glu Ala Ala Pro Pro Ala Ser Ala Ala Thr Ile Ala Ala Ala Ala
145                 150                 155                 160

Ala Thr Ala Val Val Ala Pro Thr Ser Thr Val Ala Val Ala Pro Val
                165                 170                 175

Ala Ser Ala Leu Glu Lys Lys Thr Lys Ser Lys Gly Pro Tyr Ile Cys
            180                 185                 190

Ala Leu Cys Ala Lys Glu Phe Lys Asn Gly Tyr Asn Leu Arg Arg His
        195                 200                 205

Glu Ala Ile His Thr Gly Ala Lys Ala Gly Arg Val Pro Ser Gly Ala
    210                 215                 220

Met Lys Met Pro Thr Met Val Pro Leu Ser Leu Leu Ser Val Pro Gln
225                 230                 235                 240

Leu Ser Gly Ala Gly Gly Gly Gly Gly Glu Ala Gly Ala Gly Gly Gly
                245                 250                 255

Ala Ala Ala Val Ala Ala Gly Gly Val Val Thr Thr Thr Ala Ser Gly

260                 265                 270

Lys Arg Ile Arg Lys Asn His Ala Cys Glu Met Cys Gly Lys Ala Phe
        275                 280                 285

Arg Asp Val Tyr His Leu Asn Arg His Lys Leu Ser His Ser Asp Glu
    290                 295                 300

Lys Pro Tyr Gln Cys Pro Val Cys Gln Gln Arg Phe Lys Arg Lys Asp
305                 310                 315                 320

Arg Met Ser Tyr His Val Arg Ser His Asp Gly Ala Val His Lys Pro
                325                 330                 335

Tyr Asn Cys Ser His Cys Gly Lys Ser Phe Ser Arg Pro Asp His Leu
            340                 345                 350

Asn Ser His Val Arg Gln Val His Ser Thr Glu Arg Pro Phe Lys Cys
        355                 360                 365

Glu Lys Cys Glu Ala Ala Phe Ala Thr Lys Asp Arg Leu Arg Ala His
    370                 375                 380

Thr Val Arg His Glu Glu Lys Val Pro Cys His Val Cys Gly Lys Met
385                 390                 395                 400

Leu Ser Ser Ala Tyr Ile Ser Asp His Met Lys Val His Ser Gln Gly
                405                 410                 415

Pro His His Val Cys Glu Leu Cys Asn Lys Gly Phe Thr Thr Ala Ala
            420                 425                 430

Tyr Leu Arg Ile His Ala Val Lys Asp His Gly Leu Gln Ala Pro Arg
        435                 440                 445

Ala Asp Arg Ile Leu Cys Lys Leu Cys Ser Val His Cys Lys Thr Pro
    450                 455                 460

Ala Gln Leu Ala Gly His Met Gln Thr His Leu Gly Gly Ala Ala Pro
465                 470                 475                 480

Pro Val Pro Gly Asp Ala Pro Gln Pro Gln Pro Thr Cys
                485                 490

<210>  40
<211>  746
<212>  PRT
<213>  Homo sapiens

<400>  40

Met Ile Ser Thr Lys Glu Lys Asn Lys Ile Pro Lys Asp Ser Met Thr
1               5                   10                  15

Leu Leu Pro Cys Phe Tyr Phe Val Glu Leu Pro Ile Val Ala Ser Ser
            20                  25                  30

Ile Val Ser Leu Tyr Phe Leu Glu Leu Thr Asp Leu Phe Lys Pro Ala
        35                  40                  45

Lys Val Gly Phe Gln Cys Tyr Asp Arg Thr Leu Ser Met Pro Tyr Val
    50                  55                  60

Glu Thr Asn Glu Glu Leu Ile Pro Leu Leu Met Leu Leu Ser Leu Ala
65                  70                  75                  80

Phe Ala Ala Pro Ala Ala Ser Ile Met Val Ala Glu Gly Met Leu Tyr
                85                  90                  95

Cys Leu Gln Ser Arg Leu Trp Gly Arg Ala Gly Gly Pro Ala Gly Ala
            100                 105                 110

Glu Gly Ser Ile Asn Ala Gly Gly Cys Asn Phe Asn Ser Phe Leu Arg
        115                 120                 125

Arg Thr Val Arg Phe Val Gly Val His Val Phe Gly Leu Cys Ala Thr
    130                 135                 140

Ala Leu Val Thr Asp Val Ile Gln Leu Ala Thr Gly Tyr His Thr Pro
145                 150                 155                 160

Phe Phe Leu Thr Val Cys Lys Pro Asn Tyr Thr Leu Leu Gly Thr Ser
                165                 170                 175

Cys Glu Val Asn Pro Tyr Ile Thr Gln Asp Ile Cys Ser Gly His Asp
            180                 185                 190

Ile His Ala Ile Leu Ser Ala Arg Lys Thr Phe Pro Ser Gln His Ala
        195                 200                 205

Thr Leu Ser Ala Phe Ala Ala Val Tyr Val Ser Val Ser Pro Ala Pro
    210                 215                 220

His Cys Pro Ser Gln Ala Leu Leu Leu Thr Arg Gly Glu Pro Ser Leu
225                 230                 235                 240

Thr Pro Thr Pro Met Pro Gln Met Tyr Phe Asn Ser Val Ile Ser Asp
                245                 250                 255

Thr Thr Lys Leu Leu Lys Pro Ile Leu Val Phe Ala Phe Ala Ile Ala
            260                 265                 270

Ala Gly Val Cys Gly Leu Thr Gln Ile Thr Gln Tyr Arg Ser His Pro
        275                 280                 285

Val Asp Val Tyr Ala Gly Phe Leu Ile Gly Ala Gly Ile Ala Ala Tyr
    290                 295                 300

Leu Ala Cys His Ala Val Gly Asn Phe Gln Ala Pro Pro Ala Glu Lys
305                 310                 315                 320

Pro Ala Ala Pro Ala Pro Ala Lys Asp Ala Leu Arg Ala Leu Thr Gln
                325                 330                 335

Arg Gly His Asp Ser Val Tyr Gln Gln Asn Lys Ser Val Ser Thr Asp
            340                 345                 350

Glu Leu Gly Pro Pro Gly Arg Leu Glu Gly Ala Pro Arg Pro Val Ala
        355                 360                 365

Arg Glu Lys Thr Ser Leu Gly Ser Leu Lys Arg Ala Ser Val Asp Val

370                 375                 380

Asp Leu Leu Ala Pro Arg Ser Pro Met Ala Lys Glu Asn Met Val Thr
385                 390                 395                 400

Phe Ser His Thr Leu Pro Arg Ala Ser Ala Pro Ser Leu Asp Asp Pro
                405                 410                 415

Ala Arg Arg His Met Thr Ile His Val Pro Leu Asp Ala Ser Arg Ser
            420                 425                 430

Lys Gln Leu Ile Ser Glu Trp Lys Gln Lys Ser Leu Glu Gly Arg Gly
        435                 440                 445

Leu Gly Leu Pro Asp Asp Ala Ser Pro Gly His Leu Arg Ala Pro Ala
    450                 455                 460

Glu Pro Met Ala Glu Glu Glu Glu Glu Glu Glu Asp Glu Glu Glu Glu
465                 470                 475                 480

Glu Glu Glu Glu Glu Glu Glu Asp Glu Gly Pro Ala Pro Pro Ser Leu
                485                 490                 495

Tyr Pro Thr Val Gln Ala Arg Pro Gly Leu Gly Pro Arg Val Ile Leu
            500                 505                 510

Pro Pro Arg Ala Gly Pro Pro Pro Leu Val His Ile Pro Glu Glu Gly
        515                 520                 525

Ala Gln Thr Gly Ala Gly Leu Ser Pro Lys Ser Gly Ala Gly Val Arg
    530                 535                 540

Ala Lys Trp Leu Met Met Ala Glu Lys Ser Gly Ala Ala Val Ala Asn
545                 550                 555                 560

Pro Pro Arg Leu Leu Gln Val Ile Ala Met Ser Lys Ala Pro Gly Ala
                565                 570                 575

Pro Gly Pro Lys Ala Ala Glu Thr Ala Ser Ser Ser Ser Ala Ser Ser
            580                 585                 590

Asp Ser Ser Gln Tyr Arg Ser Pro Ser Asp Arg Asp Ser Ala Ser Ile
        595                 600                 605

Val Thr Ile Asp Ala His Ala Pro His His Pro Val Val His Leu Ser
    610                 615                 620

Ala Gly Gly Ala Pro Trp Glu Trp Lys Ala Ala Gly Gly Gly Ala Lys
625                 630                 635                 640

Ala Glu Ala Asp Gly Gly Tyr Glu Leu Gly Asp Leu Ala Arg Gly Phe
                645                 650                 655

Arg Gly Gly Ala Lys Pro Pro Gly Val Ser Pro Gly Ser Ser Val Ser
            660                 665                 670

Asp Val Asp Gln Glu Glu Pro Arg Phe Gly Ala Val Ala Thr Val Asn
        675                 680                 685

Leu Ala Thr Gly Glu Gly Leu Pro Pro Leu Gly Ala Ala Asp Gly Ala
    690                 695                 700

Leu Gly Pro Gly Ser Arg Glu Ser Thr Leu Arg Arg His Ala Gly Gly
705                 710                 715                 720

Leu Gly Leu Ala Glu Arg Glu Ala Glu Ala Glu Ala Glu Gly Tyr Phe
                725                 730                 735

Arg Lys Met Gln Ala Arg Arg Phe Pro Asp
            740                 745

<210>  41
<211>  204
<212>  PRT
<213>  Homo sapiens

<400>  41

Met Gly Glu Ala Glu Val Gly Gly Gly Gly Ala Ala Gly Asp Lys Gly
1               5                   10                  15

Pro Gly Glu Ala Ala Thr Ser Pro Ala Glu Glu Thr Val Val Trp Ser
            20                  25                  30

Pro Glu Val Glu Val Cys Leu Phe His Ala Met Leu Gly His Lys Pro
        35                  40                  45

Val Gly Val Asn Arg His Phe His Met Ile Cys Ile Arg Asp Lys Phe
    50                  55                  60

Ser Gln Asn Ile Gly Arg Gln Val Pro Ser Lys Val Ile Trp Asp His
65                  70                  75                  80

Leu Ser Thr Met Tyr Asp Met Gln Ala Leu His Glu Ser Glu Ile Leu
                85                  90                  95

Pro Phe Pro Asn Pro Glu Arg Asn Phe Val Leu Pro Glu Glu Ile Ile
            100                 105                 110

Gln Glu Val Arg Glu Gly Lys Val Met Ile Glu Glu Glu Met Lys Glu
        115                 120                 125

Glu Met Lys Glu Asp Val Asp Pro His Asn Gly Ala Asp Asp Val Phe
    130                 135                 140

Ser Ser Ser Gly Ser Leu Gly Lys Ala Ser Glu Lys Ser Ser Lys Asp
145                 150                 155                 160

Lys Glu Lys Asn Ser Ser Asp Leu Gly Cys Lys Glu Gly Ala Asp Lys
                165                 170                 175

Arg Lys Arg Ser Arg Val Thr Asp Lys Val Leu Thr Ala Asn Ser Asn
            180                 185                 190

Pro Ser Ser Pro Ser Ala Ala Lys Arg Arg Arg Thr
        195                 200

<210>  42
<211>  461
<212>  PRT
<213>  Homo sapiens

<400>  42

Met Val Ser Arg Val Gln Leu Pro Pro Glu Ile Gln Leu Ala Gln Arg
1               5                   10                  15

Leu Ala Gly Asn Glu Gln Val Thr Arg Asp Arg Ala Val Arg Lys Leu
            20                  25                  30

Arg Lys Tyr Ile Val Ala Arg Thr Gln Arg Ala Ala Gly Gly Phe Thr
        35                  40                  45

His Asp Glu Leu Leu Lys Val Trp Lys Gly Leu Phe Tyr Cys Met Trp
    50                  55                  60

Met Gln Asp Lys Pro Leu Leu Gln Glu Glu Leu Gly Arg Thr Ile Ser
65                  70                  75                  80

Gln Leu Val His Ala Phe Gln Thr Thr Glu Ala Gln His Leu Phe Leu
                85                  90                  95

Gln Ala Phe Trp Gln Thr Met Asn Arg Glu Trp Thr Gly Ile Asp Arg
            100                 105                 110

Leu Arg Leu Asp Lys Phe Tyr Met Leu Met Arg Met Val Leu Asn Glu
        115                 120                 125

Ser Leu Lys Val Leu Lys Met Gln Gly Trp Glu Glu Arg Gln Ile Glu
    130                 135                 140

Glu Leu Leu Glu Leu Leu Met Thr Glu Ile Leu His Pro Ser Ser Gln
145                 150                 155                 160

Ala Pro Asn Gly Val Lys Ser His Phe Ile Glu Ile Phe Leu Glu Glu
                165                 170                 175

Leu Thr Lys Val Gly Ala Glu Glu Leu Thr Ala Asp Gln Asn Leu Lys
            180                 185                 190

Phe Ile Asp Pro Phe Cys Arg Ile Ala Ala Arg Thr Lys Asp Ser Leu
        195                 200                 205

Val Leu Asn Asn Ile Thr Arg Gly Ile Phe Glu Thr Ile Val Glu Gln
    210                 215                 220

Ala Pro Leu Ala Ile Glu Asp Leu Leu Asn Glu Leu Asp Thr Gln Asp
225                 230                 235                 240

Glu Glu Val Ala Ser Asp Ser Asp Glu Ser Ser Glu Gly Gly Glu Arg
                245                 250                 255

Gly Asp Ala Leu Ser Gln Lys Arg Ser Glu Lys Pro Pro Ala Gly Ser
            260                 265                 270

Ile Cys Arg Ala Glu Pro Glu Ala Gly Glu Glu Gln Ala Gly Asp Asp
        275                 280                 285

Arg Asp Ser Gly Gly Pro Val Leu Gln Phe Asp Tyr Glu Ala Val Ala
    290                 295                 300

Asn Arg Leu Phe Glu Met Ala Ser Arg Gln Ser Thr Pro Ser Gln Asn
305                 310                 315                 320

Arg Lys Arg Leu Tyr Lys Val Ile Arg Lys Leu Gln Asp Leu Ala Gly
                325                 330                 335

Gly Ile Phe Pro Glu Asp Glu Ile Pro Glu Lys Ala Cys Arg Arg Leu
            340                 345                 350

Leu Glu Gly Arg Arg Gln Lys Lys Thr Lys Lys Gln Lys Arg Leu Leu
        355                 360                 365

Arg Leu Gln Gln Glu Arg Gly Lys Gly Glu Lys Glu Pro Pro Ser Pro
    370                 375                 380

Gly Met Glu Arg Lys Arg Ser Arg Arg Arg Gly Val Gly Ala Asp Pro
385                 390                 395                 400

Glu Ala Arg Ala Glu Ala Gly Glu Gln Pro Gly Thr Ala Glu Arg Ala
                405                 410                 415

Leu Leu Arg Asp Gln Pro Arg Gly Arg Gly Gln Arg Gly Ala Arg Gln
            420                 425                 430

Arg Arg Arg Thr Pro Arg Pro Leu Thr Ser Ala Arg Ala Lys Ala Ala
        435                 440                 445

Asn Val Gln Glu Pro Glu Lys Lys Lys Lys Arg Arg Glu
    450                 455                 460

<210>  43
<211>  97
<212>  PRT
<213>  Homo sapiens

<400>  43

<210>  44
<211>  216
<212>  PRT
<213>  Homo sapiens

<400>  44

Met Thr Leu Ala Ala Tyr Lys Glu Lys Met Lys Glu Leu Pro Leu Val
1               5                   10                  15

Ser Leu Phe Cys Ser Cys Phe Leu Ala Asp Pro Leu Asn Lys Ser Ser
            20                  25                  30

Tyr Lys Tyr Glu Gly Trp Cys Gly Arg Gln Cys Arg Arg Lys Asp Glu
        35                  40                  45

Ser Gln Arg Lys Asp Ser Ala Asp Trp Arg Glu Arg Arg Ala Gln Ala
    50                  55                  60

Asp Thr Val Asp Leu Asn Trp Cys Val Ile Ser Asp Met Glu Val Ile
65                  70                  75                  80

Glu Leu Asn Lys Cys Thr Ser Gly Gln Ser Phe Glu Val Ile Leu Lys
                85                  90                  95

Pro Pro Ser Phe Asp Gly Val Pro Glu Phe Asn Ala Ser Leu Pro Arg
            100                 105                 110

Arg Arg Asp Pro Ser Leu Glu Glu Ile Gln Lys Lys Leu Glu Ala Ala
        115                 120                 125

Glu Glu Arg Arg Lys Tyr Gln Glu Ala Glu Leu Leu Lys His Leu Ala
    130                 135                 140

Glu Lys Arg Glu His Glu Arg Glu Val Ile Gln Lys Ala Ile Glu Glu
145                 150                 155                 160

Asn Asn Asn Phe Ile Lys Met Ala Lys Glu Lys Leu Ala Gln Lys Met
                165                 170                 175

Glu Ser Asn Lys Glu Asn Arg Glu Ala His Leu Ala Ala Met Leu Glu
            180                 185                 190

Arg Leu Gln Glu Lys Asp Lys His Ala Glu Glu Val Arg Lys Asn Lys
        195                 200                 205

Glu Leu Lys Glu Glu Ala Ser Arg
    210                 215

<210>  45
<211>  156
<212>  PRT
<213>  Homo sapiens

<400>  45

<210>  46
<211>  249

<212>  PRT
<213>  Homo sapiens

<400>  46

Met Lys Leu Asn Ile Ser Phe Pro Ala Thr Gly Cys Gln Lys Leu Ile
1               5                   10                  15

Glu Val Asp Asp Glu Arg Lys Leu Arg Thr Phe Tyr Glu Lys Arg Met
            20                  25                  30

Ala Thr Glu Val Ala Ala Asp Ala Leu Gly Glu Glu Trp Lys Gly Tyr
        35                  40                  45

Val Val Arg Ile Ser Gly Gly Asn Asp Lys Gln Gly Phe Pro Met Lys
    50                  55                  60

Gln Gly Val Leu Thr His Gly Arg Val Arg Leu Leu Leu Ser Lys Gly
65                  70                  75                  80

His Ser Cys Tyr Arg Pro Arg Arg Thr Gly Glu Arg Lys Arg Lys Ser
                85                  90                  95

Val Arg Gly Cys Ile Val Asp Ala Asn Leu Ser Val Leu Asn Leu Val
            100                 105                 110

Ile Val Lys Lys Gly Glu Lys Asp Ile Pro Gly Leu Thr Asp Thr Thr
        115                 120                 125

Val Pro Arg Arg Leu Gly Pro Lys Arg Ala Ser Arg Ile Arg Lys Leu
    130                 135                 140

Phe Asn Leu Ser Lys Glu Asp Asp Val Arg Gln Tyr Val Val Arg Lys
145                 150                 155                 160

Pro Leu Asn Lys Glu Gly Lys Lys Pro Arg Thr Lys Ala Pro Lys Ile
                165                 170                 175

Gln Arg Leu Val Thr Pro Arg Val Leu Gln His Lys Arg Arg Arg Ile
            180                 185                 190

Ala Leu Lys Lys Gln Arg Thr Lys Lys Asn Lys Glu Glu Ala Ala Glu
        195                 200                 205

Tyr Ala Lys Leu Leu Ala Lys Arg Met Lys Glu Ala Lys Glu Lys Arg
    210                 215                 220

Gln Glu Gln Ile Ala Lys Arg Arg Arg Leu Ser Ser Leu Arg Ala Ser
225                 230                 235                 240

Thr Ser Lys Ser Glu Ser Ser Gln Lys
                245

<210>  47
<211>  293
<212>  PRT
<213>  Homo sapiens

<400>  47

<210>  48
<211>  113
<212>  PRT
<213>  Homo sapiens

<400>  48

Met Ser Ala Ile Gln Asn Leu His Ser Phe Asp Pro Phe Ala Asp Ala
1               5                   10                  15

Ser Lys Gly Asp Asp Leu Leu Pro Ala Gly Thr Glu Asp Tyr Ile His
            20                  25                  30

Ile Arg Ile Gln Gln Arg Asn Gly Arg Lys Thr Leu Thr Thr Val Gln
        35                  40                  45

Gly Ile Ala Asp Asp Tyr Asp Lys Lys Lys Leu Val Lys Ala Phe Lys
    50                  55                  60

Lys Lys Phe Ala Cys Asn Gly Thr Val Ile Glu His Pro Glu Tyr Gly
65                  70                  75                  80

Glu Val Ile Gln Leu Gln Gly Asp Gln Arg Lys Asn Ile Cys Gln Phe
                85                  90                  95

Leu Val Glu Ile Gly Leu Ala Lys Asp Asp Gln Leu Lys Val His Gly
            100                 105                 110

Phe

<210>  49
<211>  420
<212>  PRT
<213>  Homo sapiens

<400>  49

<210>  50
<211>  417
<212>  PRT
<213>  Homo sapiens

<400>  50

Met Thr Thr Gln Leu Gly Pro Ala Leu Val Leu Gly Val Ala Leu Cys
1               5                   10                  15

Leu Gly Cys Gly Gln Pro Leu Pro Gln Val Pro Glu Arg Pro Phe Ser

20                  25                  30

Val Leu Trp Asn Val Pro Ser Ala His Cys Glu Ala Arg Phe Gly Val
        35                  40                  45

His Leu Pro Leu Asn Ala Leu Gly Ile Ile Ala Asn Arg Gly Gln His
    50                  55                  60

Phe His Gly Gln Asn Met Thr Ile Phe Tyr Lys Asn Gln Leu Gly Leu
65                  70                  75                  80

Tyr Pro Tyr Phe Gly Pro Arg Gly Thr Ala His Asn Gly Gly Ile Pro
                85                  90                  95

Gln Ala Leu Pro Leu Asp Arg His Leu Ala Leu Ala Ala Tyr Gln Ile
            100                 105                 110

His His Ser Leu Arg Pro Gly Phe Ala Gly Pro Ala Val Leu Asp Trp
        115                 120                 125

Glu Glu Trp Cys Pro Leu Trp Ala Gly Asn Trp Gly Arg Arg Arg Ala
    130                 135                 140

Tyr Gln Ala Ala Ser Trp Ala Trp Ala Gln Gln Val Phe Pro Asp Leu
145                 150                 155                 160

Asp Pro Gln Glu Gln Leu Tyr Lys Ala Tyr Thr Gly Phe Glu Gln Ala
                165                 170                 175

Ala Arg Ala Leu Met Glu Asp Thr Leu Arg Val Ala Gln Ala Leu Arg
            180                 185                 190

Pro His Gly Leu Trp Gly Phe Tyr His Tyr Pro Ala Cys Gly Asn Gly
        195                 200                 205

Trp His Ser Met Ala Ser Asn Tyr Thr Gly Arg Cys His Ala Ala Thr
    210                 215                 220

Leu Ala Arg Asn Thr Gln Leu His Trp Leu Trp Ala Ala Ser Ser Ala
225                 230                 235                 240

Leu Phe Pro Ser Ile Tyr Leu Pro Pro Arg Leu Pro Pro Ala His His
                245                 250                 255

Gln Ala Phe Val Arg His Arg Leu Glu Glu Ala Phe Arg Val Ala Leu
            260                 265                 270

Val Gly His Arg His Pro Leu Pro Val Leu Ala Tyr Val Arg Leu Thr
        275                 280                 285

His Arg Arg Ser Gly Arg Phe Leu Ser Gln Asp Asp Leu Val Gln Ser
    290                 295                 300

Ile Gly Val Ser Ala Ala Leu Gly Ala Ala Gly Val Val Leu Trp Gly
305                 310                 315                 320

Asp Leu Ser Leu Ser Ser Ser Glu Glu Glu Cys Trp His Leu His Asp
                325                 330                 335

Tyr Leu Val Asp Thr Leu Gly Pro Tyr Val Ile Asn Val Thr Arg Ala
            340                 345                 350

Ala Met Ala Cys Ser His Gln Arg Cys His Gly His Gly Arg Cys Ala
        355                 360                 365

Arg Arg Asp Pro Gly Gln Met Glu Ala Phe Leu His Leu Trp Pro Asp
    370                 375                 380

Gly Ser Leu Gly Asp Trp Lys Ser Phe Ser Cys His Cys Tyr Trp Gly
385                 390                 395                 400

Trp Ala Gly Pro Thr Cys Gln Glu Pro Arg Pro Gly Pro Lys Glu Ala
                405                 410                 415

Val

<210>  51
<211>  152
<212>  PRT

<213>  Homo sapiens

<400>  51

Met Ala Asn Leu Glu Arg Thr Phe Ile Ala Ile Lys Pro Asp Gly Val
1               5                   10                  15

Gln Arg Gly Leu Val Gly Glu Ile Ile Lys Arg Phe Glu Gln Lys Gly
            20                  25                  30

Phe Arg Leu Val Ala Met Lys Phe Leu Arg Ala Ser Glu Glu His Leu
        35                  40                  45

Lys Gln His Tyr Ile Asp Leu Lys Asp Arg Pro Phe Phe Pro Gly Leu
    50                  55                  60

Val Lys Tyr Met Asn Ser Gly Pro Val Val Ala Met Val Trp Glu Gly
65                  70                  75                  80

Leu Asn Val Val Lys Thr Gly Arg Val Met Leu Gly Glu Thr Asn Pro
                85                  90                  95

Ala Asp Ser Lys Pro Gly Thr Ile Arg Gly Asp Phe Cys Ile Gln Val
            100                 105                 110

Gly Arg Asn Ile Ile His Gly Ser Asp Ser Val Lys Ser Ala Glu Lys
        115                 120                 125

Glu Ile Ser Leu Trp Phe Lys Pro Glu Glu Leu Val Asp Tyr Lys Ser
    130                 135                 140

Cys Ala His Asp Trp Val Tyr Glu
145                 150


<210>  52
<211>  586
<212>  PRT
<213>  Homo sapiens

<400>  52

Met Ser Ala Thr Val Val Asp Ala Val Asn Ala Ala Pro Leu Ser Gly
1               5                   10                  15

Ser Lys Glu Met Ser Leu Glu Glu Pro Lys Lys Met Thr Arg Glu Asp
            20                  25                  30

Trp Arg Lys Lys Lys Glu Leu Glu Glu Gln Arg Lys Leu Gly Asn Ala
        35                  40                  45

Pro Ala Glu Val Asp Glu Glu Gly Lys Asp Ile Asn Pro His Ile Pro
    50                  55                  60

Gln Tyr Ile Ser Ser Val Pro Trp Tyr Ile Asp Pro Ser Lys Arg Pro
65                  70                  75                  80

Thr Leu Lys His Gln Arg Pro Gln Pro Glu Lys Gln Lys Gln Phe Ser
                85                  90                  95

Ser Ser Gly Glu Trp Tyr Lys Arg Gly Val Lys Glu Asn Ser Ile Ile
            100                 105                 110

Thr Lys Tyr Arg Lys Gly Ala Cys Glu Asn Cys Gly Ala Met Thr His
        115                 120                 125

Lys Lys Lys Asp Cys Phe Glu Arg Pro Arg Arg Val Gly Ala Lys Phe
    130                 135                 140

Thr Gly Thr Asn Ile Ala Pro Asp Glu His Val Gln Pro Gln Leu Met
145                 150                 155                 160

Phe Asp Tyr Asp Gly Lys Arg Asp Arg Trp Asn Gly Tyr Asn Pro Glu
                165                 170                 175

Glu His Met Lys Ile Val Glu Glu Tyr Ala Lys Val Asp Leu Ala Lys
            180                 185                 190

Arg Thr Leu Lys Ala Gln Lys Leu Gln Glu Glu Leu Ala Ser Gly Lys
        195                 200                 205

Leu Val Glu Gln Ala Asn Ser Pro Lys His Gln Trp Gly Glu Glu Glu
    210                 215                 220

Pro Asn Ser Gln Met Glu Lys Asp His Asn Ser Glu Asp Glu Asp Glu
225                 230                 235                 240

Asp Lys Tyr Ala Asp Asp Ile Asp Met Pro Gly Gln Asn Phe Asp Ser
                245                 250                 255

Lys Arg Arg Ile Thr Val Arg Asn Leu Arg Ile Arg Glu Asp Ile Ala
            260                 265                 270

Lys Tyr Leu Arg Asn Leu Asp Pro Asn Ser Ala Tyr Tyr Asp Pro Lys
        275                 280                 285

Thr Arg Ala Met Arg Glu Asn Pro Tyr Ala Asn Ala Gly Lys Asn Pro
    290                 295                 300

Asp Glu Val Ser Tyr Ala Gly Asp Asn Phe Val Arg Tyr Thr Gly Asp
305                 310                 315                 320

Thr Ile Ser Met Ala Gln Thr Gln Leu Phe Ala Trp Glu Ala Tyr Asp
                325                 330                 335

Lys Gly Ser Glu Val His Leu Gln Ala Asp Pro Thr Lys Leu Glu Leu
            340                 345                 350

Leu Tyr Lys Ser Phe Lys Val Lys Lys Glu Asp Phe Lys Glu Gln Gln
        355                 360                 365

Lys Glu Ser Ile Leu Glu Lys Tyr Gly Gly Gln Glu His Leu Asp Ala
    370                 375                 380

Pro Pro Ala Glu Leu Leu Leu Ala Gln Thr Glu Asp Tyr Val Glu Tyr
385                 390                 395                 400

Ser Arg His Gly Thr Val Ile Lys Gly Gln Glu Arg Ala Val Ala Cys
                405                 410                 415

Ser Lys Tyr Glu Glu Asp Val Lys Ile His Asn His Thr His Ile Trp
            420                 425                 430

Gly Ser Tyr Trp Lys Glu Gly Arg Trp Gly Tyr Lys Cys Cys His Ser
        435                 440                 445

Phe Phe Lys Tyr Ser Tyr Cys Thr Gly Glu Ala Gly Lys Glu Ile Val
    450                 455                 460

Asn Ser Glu Glu Cys Ile Ile Asn Glu Ile Thr Gly Glu Glu Ser Val
465                 470                 475                 480

Lys Lys Pro Gln Thr Leu Met Glu Leu His Gln Glu Lys Leu Lys Glu
                485                 490                 495

Glu Lys Lys Lys Lys Lys Lys Lys Lys Lys Lys His Arg Lys Ser Ser
            500                 505                 510

Ser Asp Ser Asp Asp Glu Glu Lys Lys His Glu Lys Leu Lys Lys Ala
        515                 520                 525

Leu Asn Ala Glu Glu Ala Arg Leu Leu His Val Lys Glu Thr Met Gln
    530                 535                 540

Ile Asp Glu Arg Lys Arg Pro Tyr Asn Ser Met Tyr Glu Thr Arg Glu
545                 550                 555                 560

Pro Thr Glu Glu Glu Met Glu Ala Tyr Arg Met Lys Arg Gln Arg Pro
                565                 570                 575

Asp Asp Pro Met Ala Ser Phe Leu Gly Gln
            580                 585

<210>  53
<211>  408
<212>  PRT
<213>  Homo sapiens

<400>  53

Met Ile Pro Pro Glu Gln Pro Gln Gln Gln Leu Gln Pro Pro Ser Pro
1               5                   10                  15

Ala Pro Pro Asn His Val Val Thr Thr Ile Glu Asn Leu Pro Ala Glu
            20                  25                  30

Gly Ser Gly Gly Gly Gly Ser Leu Ser Ala Ser Ser Arg Ala Gly Val
        35                  40                  45

Arg Gln Arg Ile Arg Lys Val Leu Asn Arg Glu Met Leu Ile Ser Val
    50                  55                  60

Ala Leu Gly Gln Val Leu Ser Leu Leu Ile Cys Gly Ile Gly Leu Thr
65                  70                  75                  80

Ser Lys Tyr Leu Ser Glu Asp Phe His Ala Asn Thr Pro Val Phe Gln
                85                  90                  95

Ser Phe Leu Asn Tyr Ile Leu Leu Phe Leu Val Tyr Thr Thr Thr Leu
            100                 105                 110

Ala Val Arg Gln Gly Glu Glu Asn Leu Leu Ala Ile Leu Arg Arg Arg
        115                 120                 125

Trp Trp Lys Tyr Met Ile Leu Gly Leu Ile Asp Leu Glu Ala Asn Tyr
    130                 135                 140

Leu Val Val Lys Ala Tyr Gln Tyr Thr Thr Leu Thr Ser Ile Gln Leu
145                 150                 155                 160

Leu Asp Cys Phe Val Ile Pro Val Val Ile Leu Leu Ser Trp Phe Phe
                165                 170                 175

Leu Leu Ile Arg Tyr Lys Ala Val His Phe Ile Gly Ile Val Val Cys
            180                 185                 190

Ile Leu Gly Met Gly Cys Met Val Gly Ala Asp Val Leu Val Gly Arg
        195                 200                 205

His Gln Gly Ala Gly Glu Asn Lys Leu Val Gly Asp Leu Leu Val Leu
    210                 215                 220

Gly Gly Ala Thr Leu Tyr Gly Ile Ser Asn Val Trp Glu Glu Tyr Ile
225                 230                 235                 240

Ile Arg Thr Leu Ser Arg Val Glu Phe Leu Gly Met Ile Gly Leu Phe
                245                 250                 255

Gly Ala Phe Phe Ser Gly Ile Gln Leu Ala Ile Met Glu His Lys Glu
            260                 265                 270

Leu Leu Lys Val Pro Trp Asp Trp Gln Ile Gly Leu Leu Tyr Val Gly
        275                 280                 285

Phe Ser Ala Cys Met Phe Gly Leu Tyr Ser Phe Met Pro Val Val Ile
    290                 295                 300

Lys Lys Thr Ser Ala Thr Ser Val Asn Leu Ser Leu Leu Thr Ala Asp
305                 310                 315                 320

Leu Tyr Ser Leu Phe Cys Gly Leu Phe Leu Phe His Tyr Lys Phe Ser
                325                 330                 335

Gly Leu Tyr Leu Leu Ser Phe Phe Thr Ile Leu Ile Gly Leu Val Leu
            340                 345                 350

Tyr Ser Ser Thr Ser Thr Tyr Ile Ala Gln Asp Pro Arg Val Tyr Lys
        355                 360                 365

Gln Phe Arg Asn Pro Ser Gly Pro Val Val Asp Leu Pro Thr Thr Ala
    370                 375                 380

Gln Val Glu Pro Ser Val Thr Tyr Thr Ser Leu Gly Gln Glu Thr Glu
385                 390                 395                 400

Glu Glu Pro His Val Arg Val Ala
                405

<210>  54
<211>  324
<212>  PRT
<213>  Homo sapiens

<400>  54

Met Ser Ser Glu Ala Glu Thr Gln Gln Pro Pro Ala Ala Pro Pro Ala

1               5                   10                  15

Ala Pro Ala Leu Ser Ala Ala Asp Thr Lys Pro Gly Thr Thr Gly Ser
            20                  25                  30

Gly Ala Gly Ser Gly Gly Pro Gly Gly Leu Thr Ser Ala Ala Pro Ala
        35                  40                  45

Gly Gly Asp Lys Lys Val Ile Ala Thr Lys Val Leu Gly Thr Val Lys
    50                  55                  60

Trp Phe Asn Val Arg Asn Gly Tyr Gly Phe Ile Asn Arg Asn Asp Thr
65                  70                  75                  80

Lys Glu Asp Val Phe Val His Gln Thr Ala Ile Lys Lys Asn Asn Pro
                85                  90                  95

Arg Lys Tyr Leu Arg Ser Val Gly Asp Gly Glu Thr Val Glu Phe Asp
            100                 105                 110

Val Val Glu Gly Glu Lys Gly Ala Glu Ala Ala Asn Val Thr Gly Pro
        115                 120                 125

Gly Gly Val Pro Val Gln Gly Ser Lys Tyr Ala Ala Asp Arg Asn His
    130                 135                 140

Tyr Arg Arg Tyr Pro Arg Arg Arg Gly Pro Pro Arg Asn Tyr Gln Gln
145                 150                 155                 160

Asn Tyr Gln Asn Ser Glu Ser Gly Glu Lys Asn Glu Gly Ser Glu Ser
                165                 170                 175

Ala Pro Glu Gly Gln Ala Gln Gln Arg Arg Pro Tyr Arg Arg Arg Arg
            180                 185                 190

Phe Pro Pro Tyr Tyr Met Arg Arg Pro Tyr Gly Arg Arg Pro Gln Tyr
        195                 200                 205

Ser Asn Pro Pro Val Gln Gly Glu Val Met Glu Gly Ala Asp Asn Gln
    210                 215                 220

Gly Ala Gly Glu Gln Gly Arg Pro Val Arg Gln Asn Met Tyr Arg Gly
225                 230                 235                 240

Tyr Arg Pro Arg Phe Arg Arg Gly Pro Pro Arg Gln Arg Gln Pro Arg
                245                 250                 255

Glu Asp Gly Asn Glu Glu Asp Lys Glu Asn Gln Gly Asp Glu Thr Gln
            260                 265                 270

Gly Gln Gln Pro Pro Gln Arg Arg Tyr Arg Arg Asn Phe Asn Tyr Arg
        275                 280                 285

Arg Arg Arg Pro Glu Asn Pro Lys Pro Gln Asp Gly Lys Glu Thr Lys
    290                 295                 300

Ala Ala Asp Pro Pro Ala Glu Asn Ser Ser Ala Pro Glu Ala Glu Gln
305                 310                 315                 320

Gly Gly Ala Glu

<210>  55
<211>  445
<212>  PRT
<213>  Homo sapiens

<400>  55

<210>  56
<211>  467
<212>  PRT
<213>  Homo sapiens

<400>  56

Met Asp Arg Arg Lys Lys Pro Leu Asp Val Thr Ala Ser Ser Leu Val
1               5                   10                  15

Asp Leu Lys Ala Glu Leu Phe Arg Lys Gln Glu Glu Phe Lys Gln Glu
            20                  25                  30

Lys Leu Leu Lys Asp Ser Gly Val Phe Gly Lys Pro Lys Thr Thr Asn
        35                  40                  45

Lys Lys Pro Ser Ile Trp Ser Lys Gln Asn Val Gly Val Ser Asn Arg
    50                  55                  60

Ala Glu Lys Asp Ala Glu Gln Lys Ile Glu Glu Gln Lys Thr Leu Asp
65                  70                  75                  80

Lys Ala Arg Glu Lys Leu Glu Glu Lys Ala Lys Leu Tyr Glu Lys Met
                85                  90                  95

Thr Lys Gly Asp Phe Ile Asp Glu Glu Val Glu Asp Met Tyr Leu Val
            100                 105                 110

Asp Phe Thr Gln Lys Ile Ile Asp Lys Arg Lys Glu Met Glu Ala Ser
        115                 120                 125

Gly Ala His Arg Asp Ser Gln Lys Ala Gly Glu Arg Asp Asp Asp Glu
    130                 135                 140

Glu Asn Leu Pro Glu Gly Glu Ile Pro Pro Pro Gln Asp Pro Ser Glu
145                 150                 155                 160

Glu Trp Val Asp Tyr Val Asp Ser Leu Gly Arg Ser Arg Arg Cys Met
                165                 170                 175

Arg Lys Asp Leu Pro Asp Leu Leu Glu Met Asp Lys Asn Leu Gln Gly
            180                 185                 190

Arg Leu Phe Ile Ser Pro Ala Asn Glu Lys Thr Leu Leu Ser Glu Asp
        195                 200                 205

Met Arg Lys Glu Leu Gln Arg Gln Gln Trp Glu Glu Glu Glu Arg Glu
    210                 215                 220

Ala Leu Lys Arg Pro Met Gly Pro Val His Tyr Glu Asp Ile Arg Glu
225                 230                 235                 240

Asn Glu Ala Arg Gln Leu Gly Val Gly Tyr Phe Ala Phe Ala Arg Asp
                245                 250                 255

Lys Glu Leu Arg Asn Lys Gln Met Lys Thr Leu Glu Met Leu Arg Glu
            260                 265                 270

Gln Thr Thr Asp Gln Arg Thr Lys Arg Glu Asn Ile Lys Glu Lys Arg
        275                 280                 285

Lys Ala Ile Leu Glu Ala Arg Leu Ala Lys Leu Arg Gln Lys Lys Met
    290                 295                 300

Lys Lys Ser Lys Glu Gly Gly Thr Glu Glu Glu Asn Arg Asp Gly Asp
305                 310                 315                 320

Val Ile Gly Pro Leu Pro Pro Glu Pro Glu Ala Val Pro Thr Pro Arg
                325                 330                 335

Pro Ala Ala Gln Ser Ser Lys Val Glu Val Ile Val Gln Glu Arg Lys
            340                 345                 350

Asp Thr Lys Pro Gly Val Pro His Ile Arg Glu Trp Asp Arg Gly Lys
        355                 360                 365

Glu Phe Ser Phe Gly Tyr Trp Ser Lys Arg Gln Ser Asp Leu Arg Ala
    370                 375                 380

Glu Arg Asp Pro Glu Phe Ala Pro Pro Ser Asp Tyr Phe Val Gly Gln
385                 390                 395                 400

Lys Arg Thr Gly Phe Ser Ser Ser Gln Ala Trp Ser Arg Pro Gly Pro
                405                 410                 415

Ala Gln Ser Asp Pro Gly Gln Cys Pro Asp Gln Ser His Gly Pro Ser
            420                 425                 430

Pro Glu His Thr Ser Pro Thr Pro Ala Pro Asp Asn Pro Pro Gln Ala

435                 440                 445

Pro Thr Val Thr Phe Lys Thr Leu Asp Asp Met Ile Ser Tyr Tyr Lys
    450                 455                 460

Gln Val Thr
465

<210>  57
<211>  531
<212>  PRT
<213>  Homo sapiens

<400>  57

<210>  58
<211>  288
<212>  PRT
<213>  Homo sapiens

<400>  58

## Description

The present invention refers to a diagnostic in-vitro method or assay for diagnosing chronic obstructive pulmonary disease (COPD), which comprises screening for the presence of several antibodies in a blood or serum sample.

Chronic obstructive pulmonary disease (COPD) is a common pulmonary affection, which is characterized by a range of pathophysiological changes including airflow limitation based on an obstructive bronchiolitis and persistent inflammation with neutrophils, macrophages, B and T lymphocytes and dendritic cells, a mucociliary dysfunction, apoptosis, and on structural changes in the airways causing emphysema, and by extrapulmonary systemic effects. The overall global prevalence of COPD in adults that are 40 years or older is approximately 10%. While COPD is currently the fourth leading cause of death worldwide it is expected to be the third leading cause of death in 2020. Besides, COPD is a major factor for disease-related loss of quality of life, health expenditure and loss of productivity.

Various host and environmental risk factors are supposed to contribute to the development of COPD. Host factors include airway hyper-responsiveness and aberrant lung growth. Several candidate genes are found to contribute to the individual risk. Environmental risk factors include smoking as the first cause followed by air pollution and occupational dust or chemicals.

COPD shares many clinical and pathophysiological features with autoimmune diseases. There is strong evidence for an active adaptive T-cell response in COPD patients (Agusti A et al., Thorax 2003, 58(10):832-834). Antielastin antibody and T-helper type 1 responses characterize emphysema as an autoimmune disease (Lee SH et al., Nat Med 2007, 13(5):567-569). The most recent evidence for a prevalence of IgG autoantibodies in COPD patients further supports the idea of a strong autoimmune component in COPD (Feghali-Bostwick CA et al., Am J Respir Crit Care Med 2008, 177(2):156-163). However, there is rather limited information on the nature of antigens reacting with autoantibodies in COPD patients.

However, due to the very small number of markers known in the prior art which may indicate COPD, so far it was not possible to develop a sure and reliable method for the diagnosis of chronic obstructive pulmonary disease.

### Summary of the invention

The object of the present invention was to provide a reliable and improved method for the diagnosis of chronic obstructive pulmonary disease (COPD).

In their efforts to solve the problem the present inventors identified a large number of different antigens immunogenic in COPD patients and determined how many antigens reacted with each COPD serum. Likewise, it was determined how many COPD sera reacted with each antigen. Additionally, the reactivity of each antigen was compared between COPD sera and control sera of healthy individuals. In particular, the present inventors identified a series of antigens or markers with which blood or serum samples of (human) individuals having COPD are reactive and thereby indicating the presence of chronic obstructive pulmonary disease (COPD). These markers or antigens have the amino acid sequences SEQ ID NOs: 1 to 36. Further, the present inventors identified a series of antigens or markers with which blood or serum samples of (human) individuals having COPD are reactive and thereby indicating the absence of chronic obstructive pulmonary disease (COPD). These markers or antigens have the amino acid sequences SEQ ID NOs: 37 to 58.

Therefore, the present invention is solved by an in-vitro diagnostic assay or method for diagnosing chronic obstructive pulmonary disease (COPD), comprising screening for the presence of antibodies in a blood or serum sample, wherein the antibodies are directed to MCM3 (SEQ ID NO: 1).

In a preferred method, in addition for the presence of antibodies directed to ATP9A (SEQ ID NO: 2) is screened. In a further preferred method in addition for the presence of antibodies directed to LOC388720 (SEQ ID NO: 3) is screened. In a still further preferred method in addition for the presence of antibodies directed to CDC42BPB (SEQ ID NO: 4) is screened. In another preferred method in addition for the presence of antibodies directed to RNF187 (SEQ ID NO: 5) is screened. In yet another preferred method in addition for the presence of antibodies directed to RPS27A (SEQ ID NO: 6) is screened.

In further embodiments of the present invention in addition for the presence of antibodies directed to antigens selected from the group SEQ ID NOs: 7 to 36 is screened.

The present inventors found that the reactivity of any one or more of the antigens or markers having the amino acid sequences SEQ ID NOs: 1 to 36 with a blood or serum sample of the (human) individual to be tested indicate the presence of chronic obstructive pulmonary disease (COPD). From the antigens or markers belonging to this group having the amino acid sequences selected from SEQ ID NOs: 1 to 36 those which have higher AUC-values are more preferred (see table 3). The person skilled in the art understands that the more of the markers (selected from SEQ ID NOs: 1-36) are tested the higher is the likelihood of a correct diagnosis.

The present inventors also found that the reactivity of further antigens or markers with a blood or serum sample of the (human) individual to be tested indicate the absence of chronic obstructive pulmonary disease. These markers or antigens are having the amino acid sequences SEQ ID NOs: 58 to 37. From the antigens or markers belonging to this group having the amino acid sequences selected from SEQ ID NOs: 58 to 37 those which have lower AUC-values are more preferred (see table 3). The person skilled in the art understands that the more of the markers (selected from SEQ ID NOs: 58-37) are tested the higher is the likelihood of a correct diagnosis.

Therefore, in a preferred method of the present invention in addition for the presence of antibodies directed to RPL6 (SEQ ID NO: 58) is screened. In a further preferred method in addition for the presence of antibodies directed to ZNF238 (SEQ ID NO: 57) is screened. In a still further preferred method in addition for the presence of antibodies directed to C3orf19 (SEQ ID NO: 56) is screened. In yet another preferred method in addition for the presence of antibodies directed to TUBB2A (SEQ ID NO: 55) is screened.

In further embodiments of the present invention in addition for the presence of antibodies directed to antigens selected from the group SEQ ID NOs: 54 to 37 is screened.

The present invention was achieved by the finding that the presence of antibodies in a blood or serum sample derived from the individual to be tested, which show reactivity with markers or antigens selected from the group SEQ ID NOs 1 to 36 indicate presence of COPD, whereas the presence of antibodies in a blood or serum sample derived from the individual to be tested, which show reactivity with markers or antigens selected from the group SEQ ID NOs 58 to 37 indicate absence of COPD. The person skilled in the art understands that the higher the difference of the AUC-value from 0.5 of the marker used in the method the higher is the likelihood of a correct diagnosis. Further, the person skilled in the art also understands that the more of the markers selected from SEQ ID NOs: 1-58 are tested the higher is the likelihood of a correct diagnosis.

Therefore, the present invention also provides a diagnostic assay or method for diagnosing chronic obstructive pulmonary disease (COPD), comprising screening for the presence of antibodies in a blood or serum sample, wherein the antibodies are directed to one or more sequences selected from the group consisting of SEQ ID NOs 1 to 58.

The present invention also provides a kit for the diagnosis of chronic obstructive pulmonary disease (COPD), comprising one or more antigens selected from the group consisting of SEQ ID NOs 1 to 58. In a preferred embodiment of the kit as antigen the polypeptide MCM3 (SEQ ID NO: 1) is comprised; further preferred in addition as antigens one or more polypeptides selected from the group SEQ ID NOs: 2 to 36; still further preferred in addition as antigens one or more polypeptides selected from the group SEQ ID NOs: 58 to 37 are comprised in the kit.

### Description of the Figures

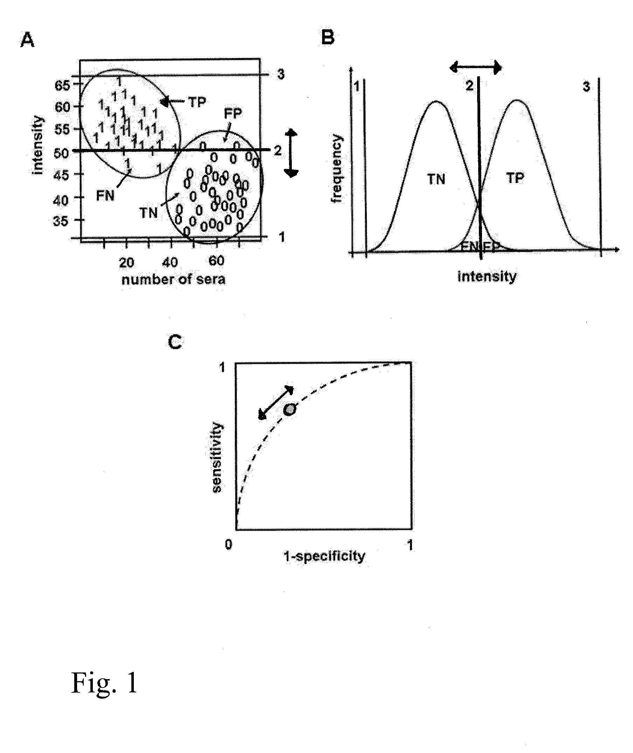

**Figure 1** shows the separation of intensity values of COPD and control sera for an arbitrary antigen A. A: Intensity values of each single COPD (1) and control (0) serum for antigen A are exemplarily shown. The position of the threshold (vertical bar (2)) determines the number of true positives (TP), true negatives (TN), false positives (FP) and false negatives (FN). The vertical bars 1 and 3 indicate the minimal and maximal thresholds. B: The two curves represent density estimations of intensity values of COPD patients (curve on the right) and controls (curve on the left) for antigen A corresponding to Figure 1A. C: The specificity (TN/(TN+FP)) and sensitivity (TP/(TP+FN)) of a test are visualized by the receiver operator characteristics (ROC) curve. The performance of the test can be represented by the area under the ROC curve (AUC). Here, the threshold is represented by the circle. The values for sensitivity and specificity can be modified by moving the threshold.

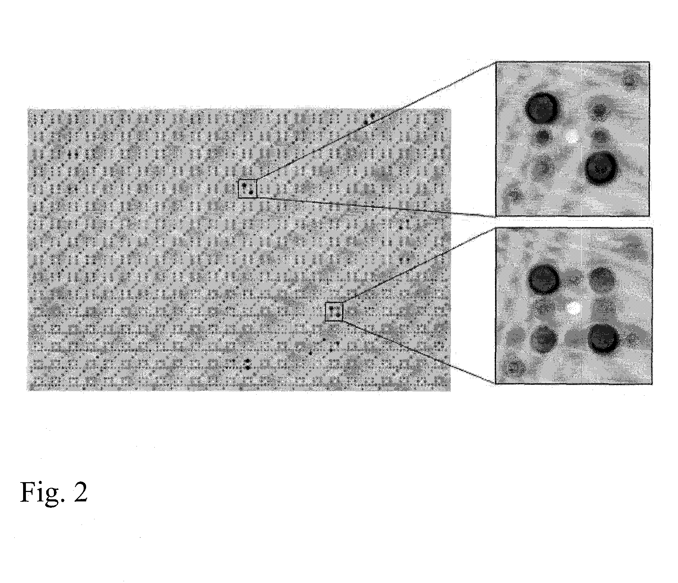

**Figure 2** shows an example for a protein macroarray analyzed with serum. Since all clones are spotted in duplicate, any antigen - antibody reactivity is indicated by two signals.

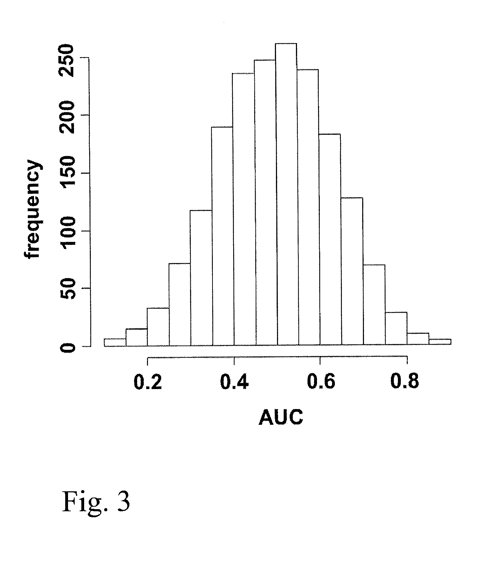

**Figure 3** shows the frequency of antigens according to their AUC value. AUC values of all antigens were calculated for the classification COPD sera versus control sera. The distribution demonstrates a large number of antigens with AUCs < 0.3 and > 0.7.

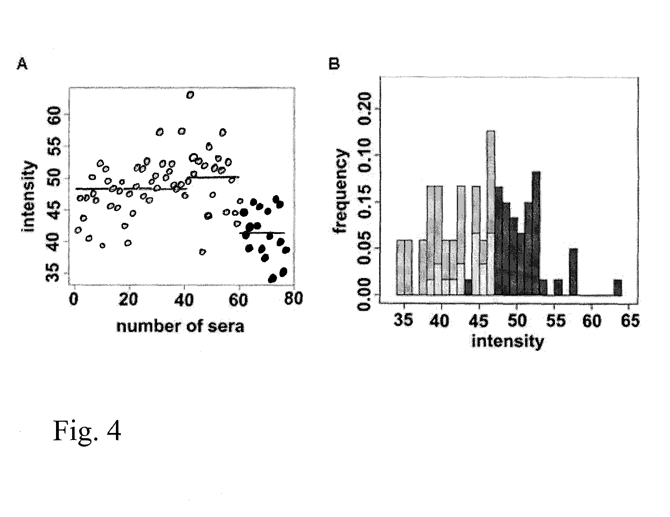

**Figure 4** shows the intensity values of FAM36A shown for all COPD and control sera. A: FAM36A intensity values shown for each serum. Filled circles indicate sera of COPD patients and open circles indicate control sera. B: Distribution of seroreactivity signals according to their intensities. Seroreactivity signals that stem from controls are indicated in dark grey bars, and seroreactivity signals that stem from COPD sera are indicated in grey bars. The overlaps are indicated in light grey bars. For this clone, signals with low intensities are mostly found with COPD sera and signals with higher intensities are mostly found with control sera.

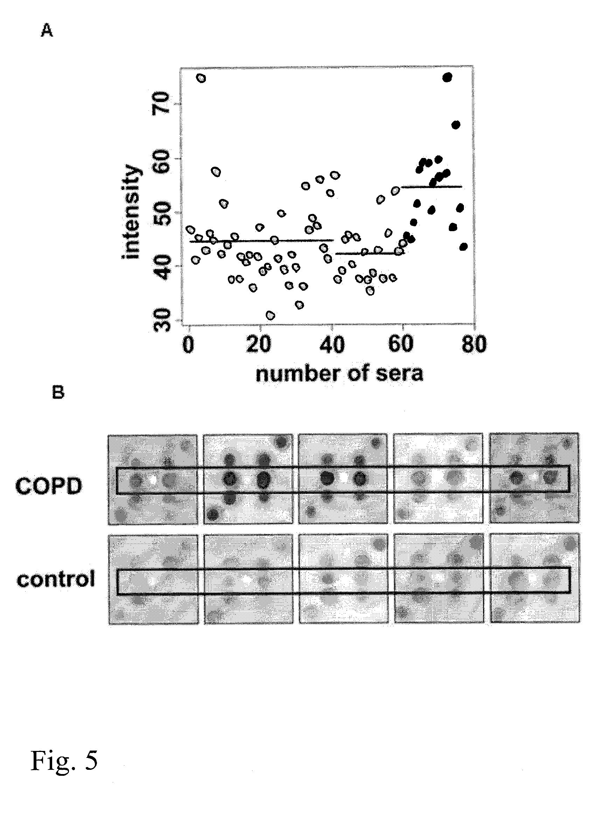

**Figure 5** shows the intensity values of MCM3 shown for all COPD and control sera. A: MCM3 intensity values shown for each serum. Sera of COPD patients are indicated by filled circles and control sera are indicated by open circles. For this clone, signals with low intensities are mostly found with control sera and signals with higher intensities are mostly found with COPD sera. B: Example of seroreactivity signals. The position of the analyzed clone is indicated by the frame. Each clone of the array is spotted in duplicate. By visual analysis, this clone was found positive with all shown COPD sera and negative with all shown normal sera.

### Examples

### Example 1 : Samples

Patient's sera: Blood samples were obtained from the Departments of Pneumology and Hemostaseology of the Saarland University with patients' informed consent. Serum was isolated from the blood samples and stored as aliquots at -70°C. The patient sera stem from 17 patients with COPD. Control sera were obtained from 60 healthy blood donors. Spirometrical data and detailed information on smoking behaviour were only available for COPD patients. Healthy blood donors underwent medical examination and had to complete a detailed sanitary questionnaire. Pre-existing diseases and addictive drug abuse were criterions for exclusion. Detailed information on COPD patients is given in Table 1 and more information of normal blood donors is given in Table 2.

### Example 2 : Protein macroarray screening:

An array of 1827 gridded immunogenic peptide clones was established and screened with 17 sera of COPD patients and 60 healthy controls. Protein arrays were evaluated both by visual inspection and a recently developed computer aided image analysis technique. By this computer aided image analysis technique the intensity values for each peptide clone and each serum were computed and the area under the receiver operator characteristics curve (AUC) was calculated for each clone and the separation COPD sera versus control sera.

1827 different immunogenic clones were assembled from high-density protein macroarrays consisting of 38,016 *E. coli* expressed proteins from the hex1 library (Bussow K, et al., Nucleic Acids Res 1998, 26(21):5007-5008) by carrying out a pre-screening with sera of patients with different human diseases. These 1827 *E. coli* expressed proteins were screened in duplicates with 17 COPD sera and 60 normal control sera. In brief, macroarrays were washed twice with TBSTT (TBS, 0.05% Tween 20, 0.5% Triton X-100) and 4 times with TBS. After blocking with 3% non-fat dry milk powder in TBST (TBS, 0.05% Tween 20), macroarrays were incubated over night with sera 1:1000 diluted in blocking solution (3% non-fat dry milk powder in TBST). After the incubation, sera were stored for the second incubation round. Three washing steps with TBST were followed by incubation with stripping solution at 70°C. Macroarrays were washed twice with TBST and 4 times with TBS. Incubation with blocking solution was followed by a second round of serum incubation over night. Macroarrays were washed three times with TBST, and incubated with secondary antibody (rabbit anti-human IgG, IgA, IgM-Cy5 (H+L)) 1:1000 diluted in blocking solution. Macroarrays were washed four times with TBST, twice with TBS and scanned by the GE Healthcare Typhoon 9410 scanner. The evaluation of the scanned protein arrays was carried out by the image analyzing software tool AIDA version 4.15 and by a newly developed computer-aided analysis procedure.

### Example 3: Computational analysis of seroreactivity patterns

Since manual inspection offers only a subjective and binary analysis of reacting clones, an automated image analysis procedure was developed. After hybridizing the arrays with the different sera, this approach computes the intensity value for each clone on the arrays. Since all clones were spotted in duplicates, the mean value of the two replicates was assigned to each clone. The evaluated antibody profiles were normalized using quantile normalization to minimize between-array-effects. To access the "value" of an antigen with respect to its ability to separate COPD sera from control sera, the area under the Receiver Operator Characteristics curve (AUC) was calculated for each antigen A as follows: the normalized intensities of all control and COPD sera were used as threshold values. For all thresholds t, COPD sera with intensity value above *t* were considered as true positives (TP), COPD sera with intensity value below *t* as false negatives (FN), control sera with intensity value below *t* as true negatives (TN), and control sera with intensity value above *t* as false positives (FP). Likewise for all thresholds, specificity (TN/(TN+FP)) and sensitivity (TP/(TP+FN)) were computed. Please note that in some cases the classification has to be inverted. In these cases, diseased sera with intensity value below *t* are considered as 'true positives' (TP). The Receiver Operator Characteristics (ROC) curve shows the specificity as function of one minus the sensitivity. AUC values can range from 0 to 1. An AUC of 0.5 for a spot means that the distribution of intensity values of COPD sera and control sera can not be distinguished. The more the AUC value of an antigen differs from 0.5, the better this antigen is suited to separate between the two serum groups COPD and control. AUCs of 1 or 0 correspond to a perfect separation of spots generated by COPD and control sera. Antigens with AUC values > 0.5 show higher intensity values in COPD sera than in control sera. Antigens with AUC values < 0.5 show higher intensity values in control sera than in COPD sera. A graphical representation of the AUC value computation is provided in Figure 1.

### Example 4: Visual analysis of protein macroarrays

Out of a fetal brain cDNA expression library encompassing more than 38,000 different peptide clones, a set of 1827 immunogenic clones were compiled by screening serum pools of various human diseases. To identify antigens reactive with COPD sera, these peptide clones were screened with 17 sera of patients with COPD and a control group of 60 sera of healthy blood donors. An example of a protein macroarray analyzed with serum is shown in Figure 2. The reactivity of each peptide clone was analyzed by visual evaluation utilizing the image analyzing software AIDA. Each COPD serum detected on average 63 peptide clones. The six most reactive sera identified more than 70 peptide clones per serum and the three least reactive sera identified less than 40 peptide clones per serum. In total, all 17 sera together detected 381 different peptide clones that reacted with autoantibodies of COPD patients.

In the next step the question of how frequent does any of the peptide clones react with sera of COPD patients was investigated. 17 clones that reacted with more than 60% of all tested COPD sera were detected and seven clones that reacted with more than 90% of all tested COPD sera.

### Example 5: Computational comparison between COPD sera and controls

Autoantibodies are not only found in diseased persons but are also common in healthy individuals. Therefore the seroreactivity of antigens was analyzed not only for COPD patients, but also for healthy blood donors as controls. To perform a low biased evaluation of the arrayed antigens, a newly developed computer-aided image analysis procedure was utilized. This automated evaluation approach allows to compare the seroreactivity of COPD and controls for each antigen. The area under the Receiver Operator Characteristics curve (AUC) was computed by comparing seroreactivity (intensity values) of COPD and control sera for each clone. The overall distribution of AUC values for all 1827 clones is shown in Figure 3. In the following only clones with AUC values < 0.3 or > 0.7 were examined, because these clones are best suited to separate COPD from control sera. 212 peptide clones were found, including 67 clones that represent in frame sequences and 145 peptide clones that represent out of frame sequences. The 67 in frame clones represent 58 different genes. The best clone (AUC=0.10) showed sequence homology to FAM36A but represents an out of frame sequence. The spot intensities of clone FAM36A for all sera and the distribution (frequency) of seroreactivity signals according to the spot intensities are shown in Figure 4. The best in frame clone (AUC=0.87) showed sequence homology to MCM3 that has been identified as immunogenic antigen in colon and prostate cancer. The above mentioned clone FAM36A showed higher immunogenicity in control sera than in COPD sera. Clone MCM3, the best in frame clone, showed higher immunogenicity in COPD sera than in control sera (see Figure 5). In total, 40 of the 67 in frame clones showed higher immunogenicity in COPD sera than in control sera, whereas only 27 of the 67 in frame clones showed higher immunogenicity in control sera than in COPD sera. In addition, 17 clones showed homology to proteins that were identified as immunogenic antigens in various human cancers. These proteins included NME2, CDC42BPB, RPS2, PTBP1, SON, MCM3, CD320, VIM, CENPB, PDE4DIP, CCNL2, HMG-14/HMGN1, HSPD1, MAZ, RPL6, STUB1, and MBTPS1. Antigens YBX1, HMG-14/HMGN1, and CENPB which showed also informative AUC values are involved in the autoimmune disease systemic sclerosis. CENPB was additionally associated with the autoimmune diseases lupus erythematosus and rheumatoid arthritis. Clones with homology to TRAF4 and NME2 have previously been associated with non-cancer lung diseases. More information on all 67 clones is summarized in Table 2.

### Example 6: In silico analysis of the in frame peptide clones

Sequence motives like coiled coil, ELR, RGD, and granzyme B cleavage sites can be characteristic for autoantigens. The 58 different genes were analyzed with the statistical gene analysis tool "GeneTrail". The analysis revealed that 20.7% of the 58 sequences are containing coiled coil motives, whereas only 11.3% of all human gene sequences show such motives. Approximately 10% more sequences were found with ELR motives in the present test set, i.e. the 58 genes, as compared to the training set. Predicted granzyme B cleavage sites were found in 43.1% of the sequences in the present test set and in 40.4% of the sequences in the reference set. In contrast, RGD motives were found in only 5.2% of the sequences in our test set but in 7.5% of the sequences in the reference set.

Out of the 58 genes, eight genes namely RPL37, RPL35, RPS27A, *RPL23A, RPL6, RPS2, RPS6,* and *LOC388720* are involved in the KEGG (Kyoto Encyclopaedia of Genes and Genomes) ribosome pathway. RPS6 is also involved in the insulin signalling and the mTOR signalling pathway. *MCM3* and *VIM* are involved in the pathways cell cycle and cell communication, respectively. *GTF2B,* also known as *TFIIB,* is a member of the pathway basal transcription factors. *HSPD1* is involved in the two pathways Prion disease and Type I diabetes mellitus.

### Discussion and Conclusion

The results of the studies carried out during the present invention support that COPD is an autoimmune disease. In the present study, 381 antigens were identified that are immunogenic in COPD patients by screening protein macroarrays with patients' sera followed by a visual evaluation. Seven clones were identified that were reactive in more than 90% of all COPD sera. In order to overcome a bias possibly caused by visual evaluation of the protein macroarrays, the macroarrays were additionally evaluated with a computer aided image analysis procedure that ensures a standardized evaluation of the arrays. Here, 212 peptide clones with informative AUC values < 0.3 and > 0.7 were identified by comparing seroreactivities of COPD patients and healthy controls. Clones with informative AUC values offer themselves as future biomarkers for COPD. The clones with AUC < 0.3 and > 0.7 included 67 in frame clones, representing 58 different genes and 145 out of frame clones that are termed mimotopes. Mimotopes are defined as peptides capable of binding to the antibody but unrelated in sequence to the natural protein that the antibody recognizes. Since some of the mimotopes yielded rather informative AUC values as for example clone FAM36A, it will be worthwhile to reveal the nature of the natural protein that react *in vivo* with autoantibodies of COPD patients.

As indicated above, a larger number of clones identified in this study were previously associated with an immune response in cancer or with autoimmune diseases. In addition, the two genes TRAF4 and NME2 have been associated with non-cancer lung diseases. TRAF4 deficiency leads to tracheal malformation resulting in airflow limitations in TRAF4-deficient mice. NME2 negatively regulates Rho activity through interactions with other proteins involved in the Rho pathway. In embryonic mouse lungs, the inhibition of the Rho pathway leads *in vitro* to reduced lung bud formation after 48 hours. It should be noted that none of the clones identified in the present study has previously been associated with the development or progression of COPD.

The studies carried out according to the present invention provides clear evidence that COPD shows strong autoimmune features. In contrast to previous studies, the present study did not only demonstrate the prevalence of IgG autoantibodies but determined the nature of antigens reacting with autoantibodies in COPD patients. The identification of novel immunogenic antigens during the studies of the present invention allow the differentiation of COPD patients from healthy controls and provides improved diagnostics for COPD.

**Table 1: Detailed information on COPD patients**

| **patient** | **diagnosis** | **sex** | **age** | **GOLD** | **FeV1 %** | **BMI** | **smoking behaviour** |
|---|---|---|---|---|---|---|---|
| 1 | COPD, Emphysema | m | 63 | GOLD IV | 26,5 | 18,6 | former smoker, 100 py |
| 2 | COPD | w | 72 | GOLD II | 75,7 | 27,5 | former smoker, py ? |
| 3 | COPD, Emphysema | w | 77 | GOLD IV | 33,7 | 18,6 | former smoker, 30 py |
| 4 | COPD | w | 54 | COPD IV | | 23,4 | ? |
| 5 | COPD | w | 55 | GOLD III | 45,6 | 21,4 | 40 py |
| 6 | COPD, Emphysema | m | 67 | GOLD IV | 28,7 | 29,3 | ? |
| 7 | COPD, Emphysema | w | 71 | GOLD IV | 30,3 | 27,4 | former smoker, 50 py |
| 8 | COPD, Emphysema | w | 55 | GOLD IV | 17,3 | 22,9 | ? |
| 9 | COPD | w | 71 | GOLD II | 64,7 | 24,2 5 | ormer smoker, 100 py |
| 10 | COPD | w | 56 | GOLD IV | 15,3 | 20,7 | former smoker, 20 py |
| 11 | COPD | w | 57 | GOLD IV | 21,2 | | former smoker, 20-25 py |
| 12 | COPD | m | 80 | GOLD III | 39,9 | 24,3 | 120 py |
| 13 | COPD | w | 69 | GOLD IV | 21,3 | 30,4 | former smoker, 120 py |
| 14 | COPD | m | 55 | GOLD IV | 28,7 | | former smoker, 30 py |
| 15 | COPD | w | 73 | GOLD II | 55,6 | | ? |
| 16 | COPD | m | 59 | GOLD IV | 27,5 | | former smoker, 30 py |
| 17 | COPD, Emphysema | w | 52 | GOLD IV | 20,1 | 12,5 | former smoker, 30 py |

| | | | | | | | |
|---|---|---|---|---|---|---|---|
| GOLD = Global Initiative for Chronic Obstructive Lung Disease; FeV1 = forced expiratory volume in one second. py = pack years (number of packs per day x duration of smoking [in years]) | | | | | | | |

**Table 2: Blood donor details**

| | **COPD sera** | **normal sera** |
|---|---|---|
| **number** | 17 | 60 |
| **male (%)** | 29.4 | 68.3 |
| **female (%)** | 70.6 | 31.7 |
| **age, mean** | 63.9 | 38.7 |

**Table 3: Antigens in frame with AUC values < 0.3 and > 0.7**

| **gene** | **chromosomal localization** | **Gene ID** | **transcript number** | **AUC values** | **SEQ ID NO:** |
|---|---|---|---|---|---|
| MCM3 | 6p12 | 4172 | NM_002388.3 | 0.87 0.84 | 1 |
| ATP9A | 20q13.11-13.2 | 10079 | NM_006045 | 0.84 | 2 |
| LOC388720 | 1q31.2 | 388720 | XM_371330.6 | 0.83 | 3 |
| CDC42BPB | 14q32.3 | 9578 | NM_006035 | 0.82 | 4 |
| RNF187 | 1q42.13 | 149603 | XM_001129309 | 0.81 | 5 |
| RPS27A | 2p16 | 6233 | NM_002954.3 | 0.81 | 6 |
| SORBS2 | 4q35.1 | 8470 | NM_021069.3 | 0.79 | 7 |
| ZNF346 | 5q35.3 | 23567 | NM_012279 | 0.78 | 8 |
| PDE4DIP | 1q21.1 | 9659 | NM_022359 | 0.78 | 9 |
| CD320 | 19p13.3-p13.2 | 51293 | NM_016579.2 | 0.78 | 10 |
| TSPAN7 | Xq11 | 7102 | NM_004615 | 0.77 | 11 |
| FAM59A | 18q12.1 | 64762 | NM_022751.1 | 0.77 | 12 |
| VIM | 10p13 | 7431 | NM_003380 | 0.77 | 13 |
| SNRPC | 6p21 | 6631 | NM_003093 | 0.77 | 14 |
| TRAF4 | 17q11-q12 | 9618 | NM_004295.3 | 0.77 | 15 |
| ZNF835 | 19q13.43 | 90485 | NM_001005850 | 0.77 | 16 |
| ERP29 | 12q24.13 | 10961 | NM_006817.3 | 0.76 | 17 |
| CENPB | 20p13 | 1059 | NM_001810 | 0.75 | 18 |
| MORF4L1 | 15q24 | 10933 | NM_006791.2 | 0.75 | 19 |
| KIAA0652 | 11p11.2 | 9776 | NM_014741.2 | 0.75 | 20 |
| CAMK1D | 10p13 | 57118 | NM_020397 | 0.75 | 21 |
| RPL35 | 9q34.1 | 11224 | NM_007209.3 | 0.74 | 22 |
| PTBP1 | 19p13.3 | 5725 | NM_031990 | 0.74 | 23 |
| KIAA1279 | 10q22.1 | 26128 | NM_015634 | 0.73 | 24 |
| ANP32A | 15q22.3-15q23 | 8125 | NM_006305 | 0.73 | 25 |
| BTBD7 | 14q32.13 | 55727 | NM_018167 | 0.72 | 26 |
| CCNL2 | 1p36.33 | 81669 | NM_030937 | 0.72 | 27 |
| SON | 21q22.1-q22.2 | 6651 | NM_138927 | 0.72 | 28 |
| CIAPIN1 | 16q13-q21 | 57019 | NM_020313 | 0.72 | 29 |
| GTF2B | 1p22-p21 | 2959 | NM_001514 | 0.72 | 30 |
| LPHN1 | 19p13.2 | 22859 | NM_014921 | 0.72 | 31 |
| HSPD1 | 2q33.1 | 3329 | NM_002156.4 | 0.72 | 32 |
| RPA3 | 7 | 6119 | NM_002947 | 0.71 | 33 |
| KLC1 | 14q32.3 | 3831 | NM_005552 | 0.71 | 34 |
| FAM59B | 2p23.3 | 150946 | XM_097977 | 0.71 | 35 |
| TBRG4 | 7p13 | 9238 | NM_004749.2 | 0.71 | 36 |
| HMG-14 / HMGN1 | 21q22.3 | 3150 | NM_004965 | 0.3 | 37 |
| STUB1 | 16p13.3 | 10273 | NM_005861.2 | 0.3 | 38 |
| MAZ | 16p11.2 | 4150 | NM_001042539.1 | 0.3 | 39 |
| PRG2 | 19p13.3 | 79948 | NM_024888.1 | 0.29 | 40 |
| C20orf20 | 20q13.33 | 55257 | NM_018270.3 | 0.29 | 41 |
| RRP1 | 21q22.3 | 8568 | NM_003683.5 | 0.29 | 42 |
| RPL37 | 5p13 | 6167 | NM_000997.4 | 0.28 | 43 |
| STMN4 | 8p21.2 | 81551 | NM_030795.2 | 0.28 | 44 |
| RPL23A | 17q11.2 | 6147 | NM_000984 | 0.27 | 45 |
| RPS6 | 9p21 | 6194 | NM_001010 | 0.27 | 46 |
| RPS2 | 16p13.3 | 6187 | NM_002952 | 0.27 | 47 |
| EIF1 | 17q21.2 | 10209 | NM_005801.3 | 0.27 | 48 |
| HNRPDL | 4q13-q21 | 9987 | NM_031372.2 | 0.25 | 49 |
| HYAL3 | 3p21.3 | 8372 | NM_003549 | 0.25 | 50 |
| NME2 | 17q21.3 | 4831 | NM_002512.2 | 0.24 | 51 |
| SLU7 | 5q33.3 | 10569 | NM_006425 | 0.23 | 52 |
| SLC35F1 | 6q22.2-q22.31 | 222553 | NM_001029858.2 | 0.23 | 53 |
| YBX1 | 1p34 | 4904 | NM_004559.3 | 0.21 | 54 |
| TUBB2A | 6p25.2 | 7280 | NM_001069 | 0.2 | 55 |
| C3orf19 | 3p25.1 | 51244 | NM_016474.4 | 0.2 | 56 |
| ZNF238 | 1q44-ter | 10472 | NM_205768 | 0.18 | 57 |
| RPL6 | 12q23-24.1 | 6128 | NM_000970 | 0.13 | 58 |

## Claims

1. An in-vitro diagnostic assay or method for diagnosing chronic obstructive pulmonary disease (COPD), comprising screening for the presence of antibodies in a blood or serum sample, wherein the antibodies are directed to MCM3 (SEQ ID NO: 1).

2. The diagnostic assay or method according to claim 1, wherein in addition for the presence of antibodies directed to ATP9A (SEQ ID NO: 2) is screened.

3. The diagnostic assay or method according to claim 1 or 2, wherein in addition for the presence of antibodies directed to LOC388720 (SEQ ID NO: 3) is screened.

4. The diagnostic assay or method according to any one of claims 1 to 3, wherein in addition for the presence of antibodies directed to CDC42BPB (SEQ ID NO: 4) is screened.

5. The diagnostic assay or method according to any one of claims 1 to 4, wherein in addition for the presence of antibodies directed to RNF187 (SEQ ID NO: 5) is screened.

6. The diagnostic assay or method according to any one of claims 1 to 5, wherein in addition for the presence of antibodies directed to RPS27A (SEQ ID NO: 6) is screened.

7. The diagnostic assay or method according to any one of claims 1 to 6, wherein in addition for the presence of antibodies directed to antigens selected from the group SEQ ID NOs: 7 to 36 is screened.

8. The diagnostic assay or method according to any one of claims 1 to 7, wherein in addition for the presence of antibodies directed to RPL6 (SEQ ID NO: 58) is screened.

9. The diagnostic assay or method according to any one of claims 1 to 8, wherein in addition for the presence of antibodies directed to ZNF238 (SEQ ID NO: 57) is screened.

10. The diagnostic assay or method according to any one of claims 1 to 9, wherein in addition for the presence of antibodies directed to C3orf19 (SEQ ID NO: 56) is screened.

11. The diagnostic assay or method according to any one of claims 1 to 10, wherein in addition for the presence of antibodies directed to TUBB2A (SEQ ID NO: 55) is screened.

12. The diagnostic assay or method according to any one of claims 1 to 11, wherein in addition for the presence of antibodies directed to antigens selected from the group SEQ ID NOs: 54 to 37 is screened.

13. A diagnostic assay or method for diagnosing chronic obstructive pulmonary disease (COPD), comprising screening for the presence of antibodies in a blood or serum sample, wherein the antibodies are directed to sequences selected from the group consisting of SEQ ID NOs 1 to 58.

14. A kit for the diagnosis of chronic obstructive pulmonary disease (COPD), comprising one or more antigens selected from the group consisting of SEQ ID NOs 1 to 58.

15. The kit according to claim 14, comprising as antigen the polypeptide MCM3 (SEQ ID NO: 1).
